# EUROPEAN PATENT SPECIFICATION

(11) **EP 2 016 102 B1**
(45) Date of publication and mention of the grant of the patent: **21.03.2012**
(21) Application number: 07797329.5
(22) Date of filing: 03.05.2007
(51) Int. Cl.: C07K 19/00, C07K 14/725

(54) **CHIMERIC T CELL RECEPTORS AND RELATED MATERIALS AND METHODS OF USE**
CHIMÄRE T-ZELLEN-REZEPTOREN SOWIE ENTSPRECHENDE MATERIALIEN UND VERWENDUNGSVERFAHREN
RÉCEPTEURS DE LYMPHOCYTES T CHIMÉRIQUES, MATIÈRES ASSOCIÉES ET MÉTHODES D'UTILISATION

(30) Priority: 03.05.2006 US 796853 P
(43) Date of publication of application: 21.01.2009
(73) Proprietor: Government of the United States of America, Represented by the Secretary, Department of Health and Human Services, Rockville, Maryland 20852 (US)
(72) Inventor: MORGAN, Richard A., Columbia, Maryland 21044 (US); COHEN, Cyrille J., Rockville, Maryland 20852 (US); ROSENBERG, Steven A., Potomac, Maryland 20854 (US)
(74) Representative: Grünecker, Kinkeldey, Stockmair & Schwanhäusser
(86) International application number: PCT/US2007/068113
(87) International publication number: WO 2007/131092

(56) References cited:
- WO-A-2004/074322
- COHEN CYRILLE J ET AL: "Enhanced antitumor activity of murine-human hybrid T-cell receptor (TCR) in human lymphocytes is associated with improved pairing and TCR/CD3 stability." CANCER RESEARCH 1 SEP 2006, vol. 66, no. 17, 1 September 2006 (2006-09-01), pages 8878-8886, XP002466475 ISSN: 1538-7445
- BOULTER JONATHAN M ET AL: "Stable, soluble T-cell receptor molecules for crystallization and therapeutics" PROTEIN ENGINEERING, OXFORD UNIVERSITY PRESS, SURREY, GB, vol. 16, no. 9, September 2003 (2003-09), pages 707-711, XP002275339 ISSN: 0269-2139
- WILLEMSEN R A ET AL: "GRAFTING PRIMARY HUMAN T LYMPHOCYTES WITH CANCER-SPECIFIC CHIMERIC SINGLE CHAIN AND TWO CHAIN TCR" GENE THERAPY, MACMILLAN PRESS LTD., BASINGSTOKE, GB, vol. 7, no. 16, August 2000 (2000-08), pages 1369-1377, XP001181298 ISSN: 0969-7128
- ZHAO ET AL: "High-Efficiency Transfection of Primary Human and Mouse T Lymphocytes Using RNA Electroporation" MOLECULAR THERAPY, ACADEMIC PRESS, SAN DIEGO, CA,, US, vol. 13, no. 1, January 2006 (2006-01), pages 151-159, XP005197704 ISSN: 1525-0016
- COHEN CYRILLE J ET AL: "Recognition of fresh human tumor by human peripheral blood lymphocytes transduced with a bicistronic retroviral vector encoding a murine anti-p53 TCR" JOURNAL OF IMMUNOLOGY, vol. 175, no. 9, November 2005 (2005-11), pages 5799-5808, XP002466476 ISSN: 0022-1767
- KUBALL J ET AL: "Cooperation of human tumor-reactive CD4+ and CD8+ T cells after redirection of their specificity by a high-affinity p53A2.1-specific TCR" IMMUNITY, CELL PRESS, US, vol. 22, no. 1, January 2005 (2005-01), pages 117-129, XP002351551 ISSN: 1074-7613
- CHANG H-C ET AL: "A GENERAL METHOD FOR FACILITATING HETERODIMERIC PAIRING BETWEEN TWO PROTEINS: APPLICATION TO EXPRESSION OF ALPHA AND BETA T-CELL RECEPTOR EXTRACELLULAR SEGMENTS" PROCEEDINGS OF THE NATIONAL ACADEMY OF SCIENCES OF USA, NATIONAL ACADEMY OF SCIENCE, WASHINGTON, DC, US, vol. 91, no. 24, November 1994 (1994-11), pages 11408-11412, XP002008362 ISSN: 0027-8424

## Description

### CROSS-REFERENCE TO RELATED APPLICATIONS

This patent application claims the benefit of U.S. Provisional Patent Application No. 60/796,853, filed May 3, 2006.

### MATERIAL SUBMITTED ON COMPACT DISC

Computer-readable nucleotide/amino acid sequence listing submitted concurrently herewith and identified as follows: One 211,000 Byte ASCII (Text) file named "701228ST25.TX-T," created on May 1, 2007.

### BACKGROUND OF THE INVENTION

Several studies have shown that it is feasible to transduce T cell receptor (TCR) genes into human lymphocytes to redirect the antigenic specificity of transduced populations to antigens of interest (reviewed in Dembic et al., Nature, 320, 232-238 (1986), Schumacher, Nat. Rev. Immunol., 2, 512-519 (2002), Kershaw et al., Nat. Rev. Immunol., 5, 928-940 (2005), Xue et al., Clin. Exp. Immunol., 139, 167-172 (2005), Rossig et al., Mol. Ther., 10, 5-18 (2004), and Murphy et al., Immunity, 22, 403-414 (2005)). Of particular interest is the reprogramming of human lymphocytes for cancer treatment, since cellular adoptive immunotherapy has been shown to mediate the regression of large solid tumors in patients with metastatic melanoma (Dudley et al., Science, 298, 850-854 (2002), and (Dudley et al., J Clin. Oncol., 23, 2346-2357 (2005)). However, a limitation of adoptive immunotherapy is the need to isolate and expand tumor-reactive lymphocytes that pre-exist in the patient. Therefore, TCR transfer procedures to human lymphocytes may overcome the requirement for pre-existing tumor-specific immunity and the need to laboriously identify and isolate tumor-reactive T-cells from each patient. In this regard, several groups have shown that it is possible to engineer lymphocytes to express human TCRs that confer novel anti-tumor activity (Morgan et al., J. Immunol., 171, 3287-3295 (2003), Hughes et al., Hum. Gene Ther., 16, 1-16 (2005), Zhao et al., J. Immunol., 174, 4415-4423 (2005), Roszkowski et al., Cancer Res., 65, 1570-1576 (2005), and Engels et al., Hum. Gene Ther., 16, 799-810 (2005)).

A potential hurdle in TCR gene transfer approaches is the mispairing of the introduced TCR subunits with endogenous TCR chains. The immediate effect of the competition between exogenous and endogenous TCR subunits may result in the reduction of the cell-surface density of the exogenous TCR (Roszkowski et al., Cancer Res., 65, 1570-1576 (2005), Rubinstein et al., J. Immunol., 170, 1209-1217 (2003), Munthe et al., Cell Immunol., 170, 283-290 (1996), Blichfeldt et al., Eur. J. Immunol., 26, 2876-2884 (1996)). Additionally, the mispairing of introduced TCR subunits with endogenous TCR chains may lead to the formation of undesirable TCR heterodimers, such as those with potential self-reactivity (Schumacher, Nat. Rev. Immunol. 2: 512-519 (2002) and Xue et al., Clin. Exp. Immunol. 139: 167-172 (2002)).

In view of the foregoing, there is a need in the art for improved TCRs and cells expressing such TCRs.

The invention provides such TCRs and cells, especially for use in methods of treating or preventing diseases, such as cancer.

### BRIEF SUMMARY OF THE INVENTION

The invention provides chimeric TCRs which have enhanced biological properties providing greater T cell responses against the antigen recognized by the chimeric TCR. The inventive chimeric TCRs comprise a variable region of a human TCR and a constant region comprising at least an extracellular domain of a constant region of a non-human TCR, e.g., a murine TCR.

The invention also provides functional variants of the inventive chimeric TCRs, wherein the functional variants have at least about 75% sequence identity to the chimeric TCR and specifically bind to the antigen for which the chimeric TCR has antigenic specificity.

The invention further provides polypeptides and proteins related to the inventive chimeric TCRs and functional variants described herein, as well as nucleic acids comprising nucleotide sequences encoding any of the inventive chimeric TCRs, including functional variants thereof, polypeptides, and proteins. Related recombinant expression vectors, and host cells, especially human host cells, are furthermore provided herein.

Pharmaceutical compositions comprising the inventive chimeric TCRs, polypeptides, proteins, nucleic acids, recombinant expression vectors, host cells, and populations thereof are also provided by the invention.

Also provided by the invention is a method of treating or preventing a disease, e.g., cancer or an infectious disease, in a host. The method comprises administering to the host the inventive pharmaceutical composition in an amount effective to treat or prevent the disease in the host.

The invention further provides a method of detecting a diseased cell in a host. The method comprises (i) contacting a sample comprising cells of the host with the inventive chimeric TCR, protein, recombinant expression vector, host cell, or population of host cells, thereby forming a complex, and (ii) detecting the complex, wherein detection of the complex is indicative of a diseased cell in the host.

A method of improving the biological activity of a human TCR is further provided by the invention. The method comprises replacing the constant region of the human TCR with a constant region comprising at least an extracellular domain of a constant region of a non-human TCR.

### BRIEF DESCRIPTION OF THE SEVERAL VIEWS OF THE DRAWING(S)

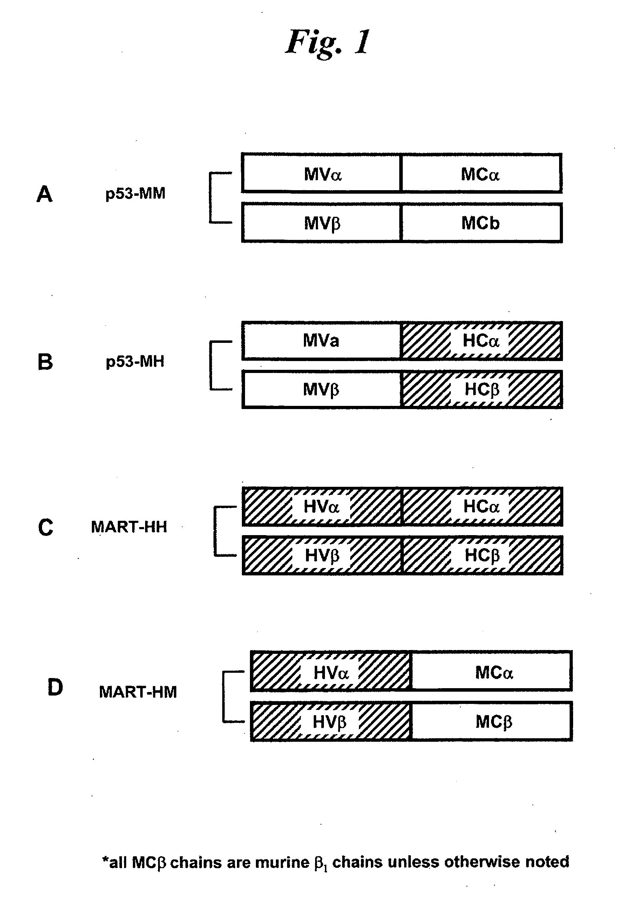

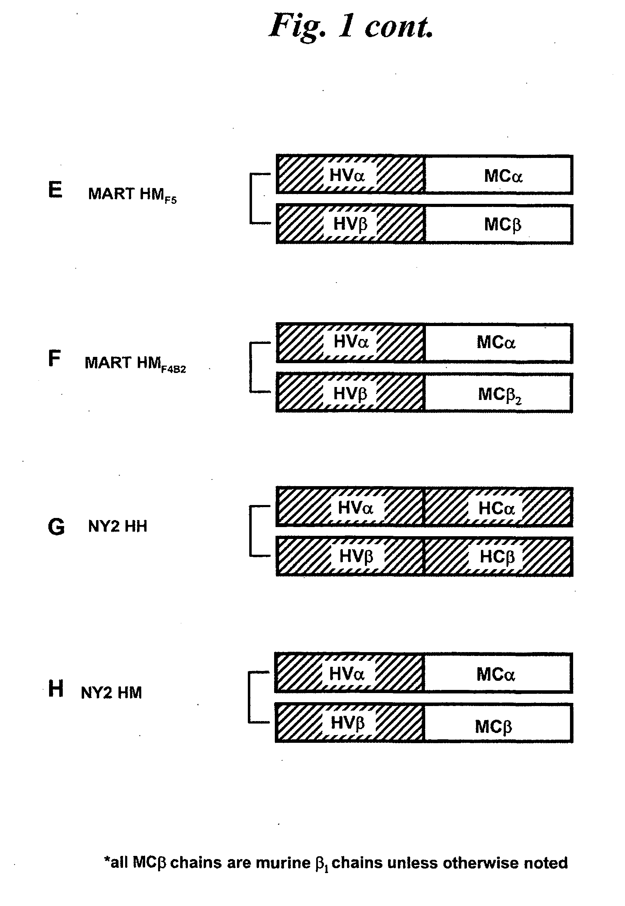

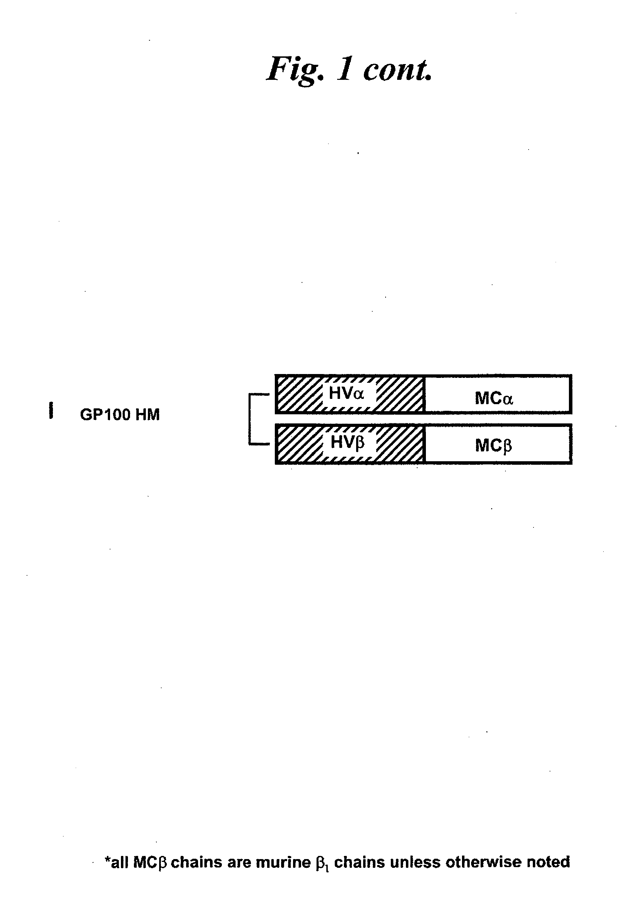

Figures 1A to 1I are schematic representations of the TCRs described herein. Figure 1A is a schematic representation of the p53 MM TCR, which is a fully murine anti-p53 TCR comprising murine α and β chains, each of which comprise a variable region (V) and a constant region (C). Figure 1B is a schematic representation of the p53 MH TCR, which is a humanized version of the p53 MM TCR comprising mouse variable regions on each of α and β chains (MVα and MVβ) and human constant regions on each of the α and β chains (HCα and HCβ). Figure 1C is a schematic representation of the MART HH TCR, which is a fully human anti-MART-1 TCR comprising human α and β chains, each of which comprise a variable region (V) and a constant region (C). Figure 1D is a schematic representation of the MART HM TCR, which is a murinized version of the MART HH TCR comprising human variable regions on each of α and β chains (HVα and HVβ) and mouse constant regions on each of the α and β chains (MCα and MCβ). Figure 1E is a schematic representation of the MART HM_{F5} TCR, which is a murinized human anti-MART-1 TCR comprising human variable regions on each of α and β chains (HVα and HVβ) and mouse constant regions on each of the α and β chains (MCα and MCβ). The MART HM_{F5} TCR comprises variable regions that are different from the variable regions of the MART HM and MART HH TCRs. Figure 1F is a schematic representation of the MART HM_{F4B2} TCR, which is a murinized human anti-MART-1 TCR comprising the human variable regions of MART HM and MART HH TCRs (HVα and HVβ), mouse constant regions from the mouse α chain and mouse β2 chain (MCα and MCβ2). Figure 1G is a schematic representation of the NY2 HH TCR, which is a fully human anti-NY-ESO-1 TCR comprising human α and β chains, each of which comprise a variable region (V) and a constant region (C). Figure 1H is a schematic representation of the NY2 HM, which is a murinized version of NY2 HH comprising the human variable regions of NY2 HH (HVα and HVβ) and mouse constant regions on each of the α and β chains (MCα and MCβ). Figure 1I is a schematic representation of the GP100 HM TCR, which is a murinized human anti-gp100 TCR comprising human variable regions on each of the α and β chains (HVα and HVβ) and mouse β constant regions on each of the α and β chains (MCα and MCβ).

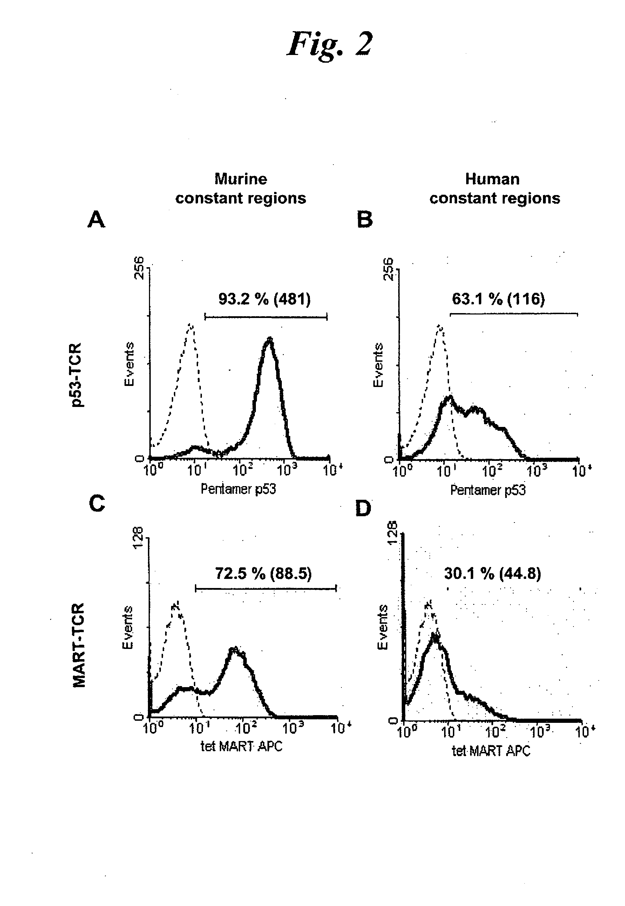

Figures 2A to 2D are flow cytometry graphs of cells electroporated with mRNA encoding the p53 MM TCR (Figure 2A), p53 MH TCR (Figure 2B), MART HM TCR (Figure 2C), or MART HH TCR (Figure 2D), stained with either p53 pentamer (Figures 2A and 2B) or MART-1 tetramer (Figures 2C and 2D), and analyzed by FACS. The percentage of positive cells, as well as the relative mean fluorescence intensity (in brackets) of the gated population, are shown.

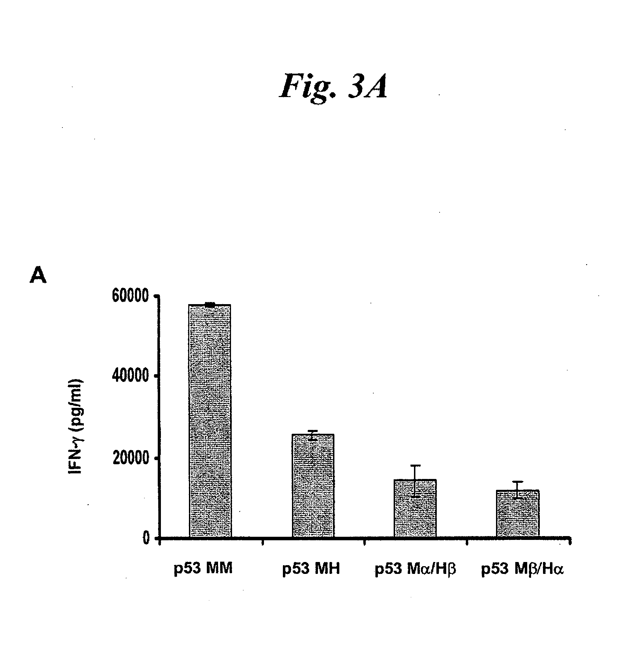

Figure 3A is a graph of the IFNγ secretion as detected by ELISA of human PBLs electroporated with mRNA encoding: the p53-MM TCR, pS3-MH TCR, the α chain of the p53-MR4 and the β chain of the p53-MH (p53 Mα/Hβ), or the β chain of the p53 MM and the α chain of the p53-MH (p53 Mβ/Hα) and co-cultured with T2 cells pulsed with p53₂₆₄₋₂₇₂ peptide or non-specific control peptides (controls not shown).

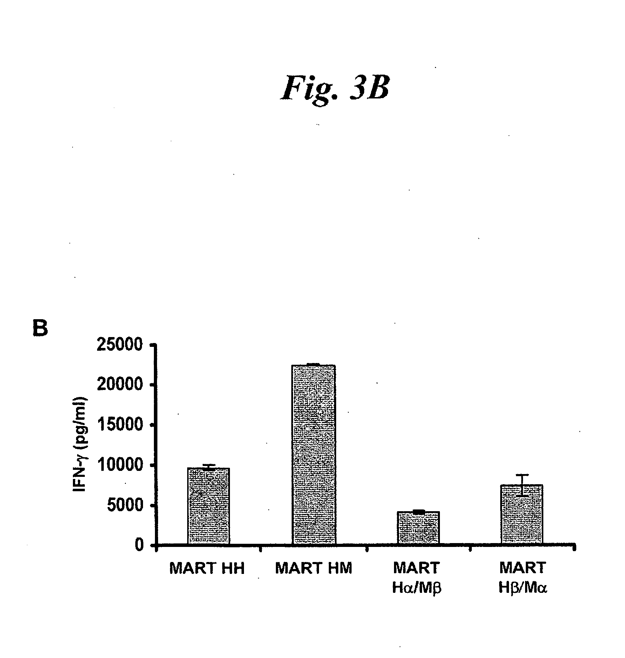

Figure 3B is a graph of the IFNγ secretion as detected by ELISA of human PBLs electroporated with mRNA encoding the MART HH TCR, MART HM TCR, the α chain of the MART HH and the β chain of the MART HM (MART Hα/Mβ), or the β chain of the MART HH and the α chain of the MART HM (MART Hβ/Mα) and co-cultured with T2 cells pulsed with specific peptide (MART-1 27L₂₆₋₃₅) or non-specific control peptides (gp₁₀₀₋₂₀₉, gp₁₀₀₋₂₈₀, p53₁₄₉₋₁₅₇, and HBVc peptide - controls not shown).

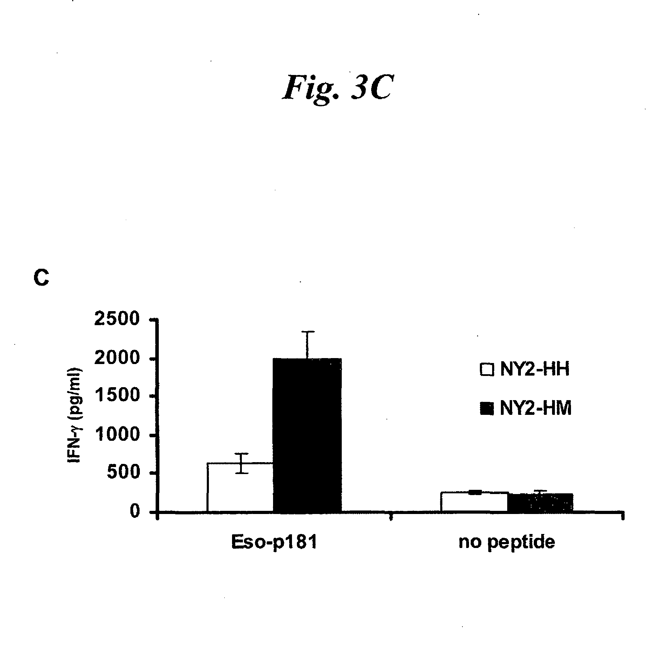

Figure 3C is graph of the IFN-γ secretion as detected by ELISA of CD4-enriched cells electroporated with mRNA encoding the alpha and beta chains of NY2-HH, which is an HLA-DP4-restricted NY-ESO-1 TCR, or its mouse chimeric counterpart (NY2-HM) comprising murine constant regions and co-cultured with HLA-DP4⁺ EBV-B cells (DK-EBV-B) pulsed without (no peptide) or with NY-ESO-1ₚ₁₆₁₋₁₈₀ peptide (NY-ESO-1₁₆₁₋₁₈₀).

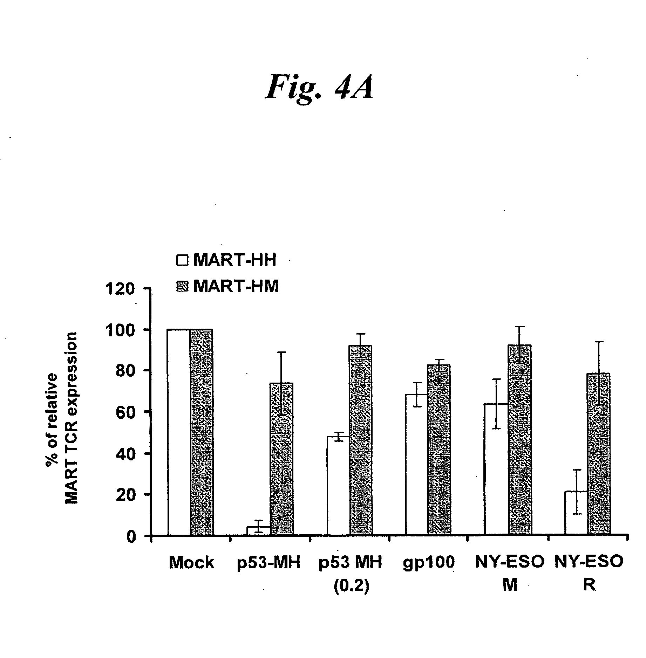

Figure 4A is a graph of the % of relative MART TCR expression of TCR-deficient Jurkat RT3-T3.5 cells electroporated with mRNA encoding each chain of the MART-HH (white) or MART-HM (black), along with mRNA encoding each chain of a competitor TCR (p53 MH, gp100 TCR, NY-ESO M TCR, NY-ESO R TCR) and stained with MART-1 tetramer. The amount of mRNA used in the electroporation was 1 µg of mRNA for each chain of the TCR, except in cells marked p53 MH (0.2), 0.2 µg mRNA encoding each chain of the competitor TCR was used. All the differences were statistically significant based on a *Student's t-test* (p<0.05).

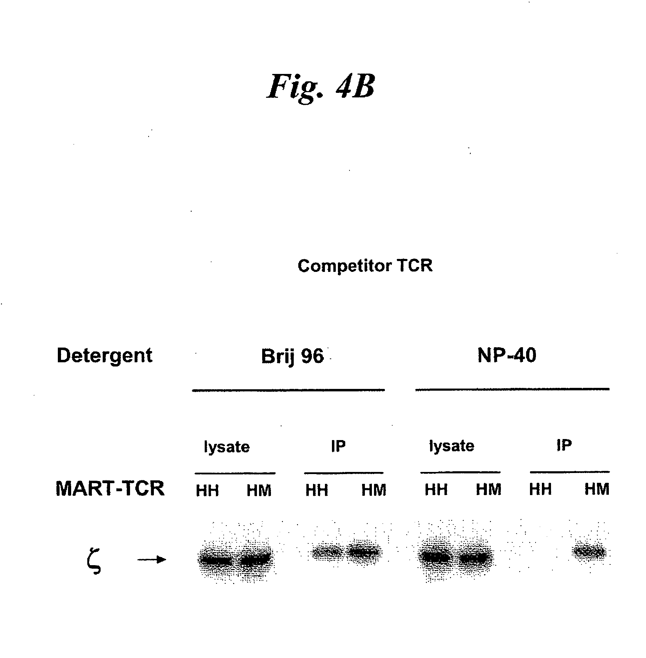

Figure 4B is a collection of Western blots of TCR-deficient Jurkat RT3-T3.5 cells electroporated with the MART-HH or MART-HM, lysed with mild detergent (Brij96) or a strong detergent (NP40), immunoprecipitated with a Vβ 12-specific antibody, and blotted for CD3-ζ. As a control for protein loading, cell lysates not subjected to immunoprecipitation were used and blotted for CD3-ζ. The data represent one of three independent experiments.

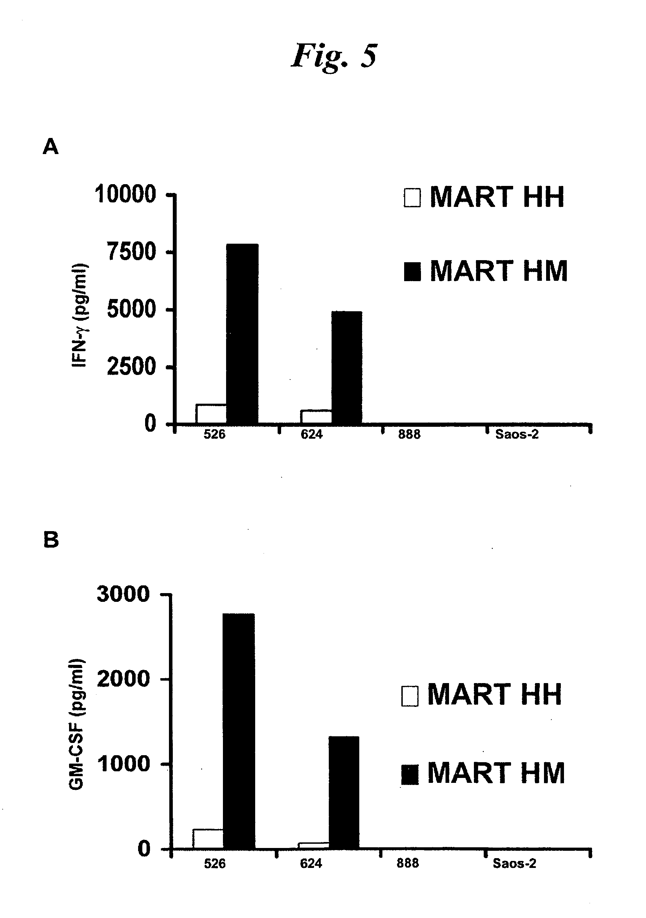

Figures 5A and 5B are graphs of the IFN-γ secretion and GM-CSF secretion, respectively, as determined by ELISA of human PBLs expressing either the MART-HH (white bars) or the MART-HM (black bars) TCR and co-cultured with the HLA-A2⁺ melanoma cell lines (526 and 624), the HLA-A2⁻ melanoma cell line (888), or the HLA-A2⁺ Saos-2 osteosarcoma line.

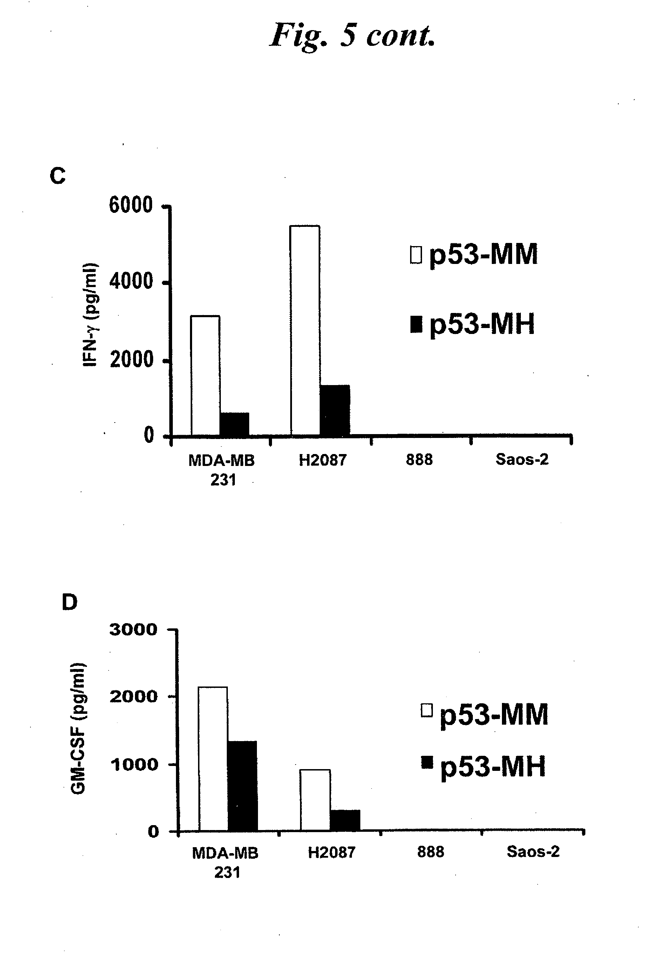

Figures 5C and 5D are graphs of the IFN-γ secretion and GM-CSF secretion, respectively, as determined by ELISA of human PBLs expressing either the p53-MM (white bars) or the p53-MH (black bars) TCR and co-cultured with the p53⁺/HLA-A2⁺ tumor cells (MDA-MB-231 and H2087), the p53-/HLA-A2⁺ Saos-2 cells, and HLA-A2- control cells (888).

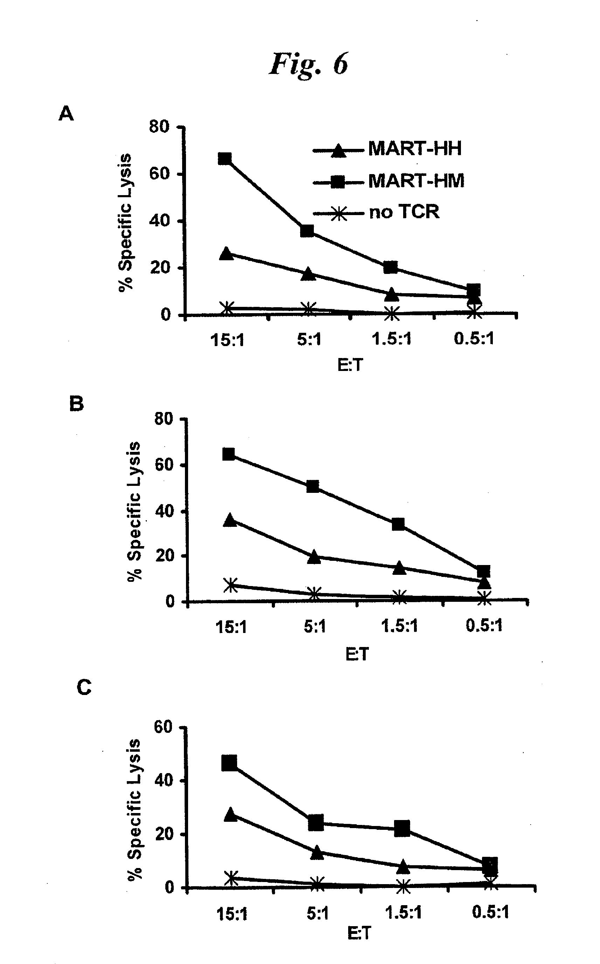

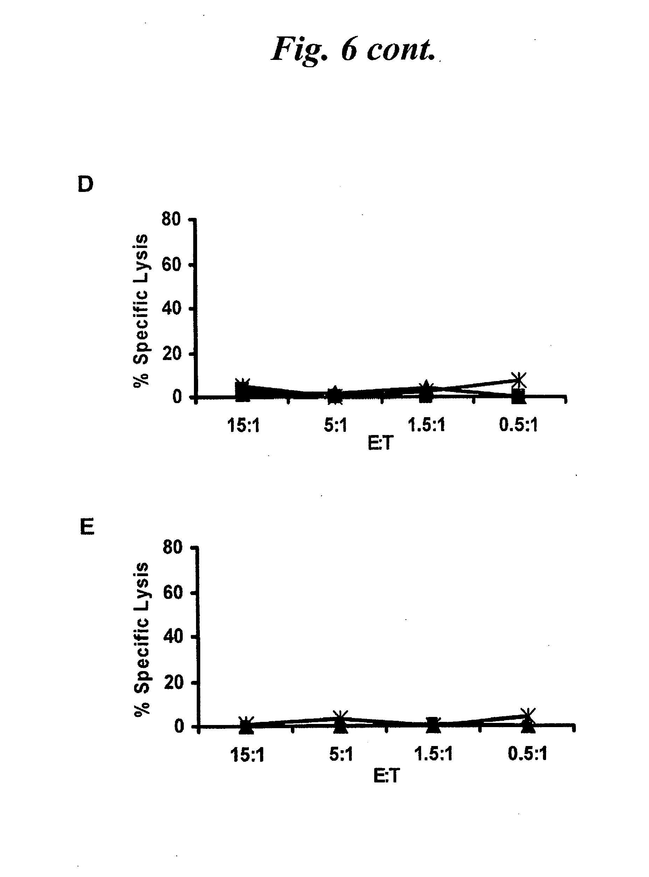

Figures 6A to 6E are a series of graphs of the % specific lysis of effector cells (CD8⁺ human PBLs expressing the MART-HH TCR (triangle) or the MART-HM TCR (square) or PBLs that were mock-electroporated (star)) co-cultured with the target tumor cell lines labeled with ⁵¹Cr prior to co-culture at the specified effector:target (E:T) ratio. In Figure 6A, HLA-A2⁺/melanoma cell line, 526, was the target tumor cell. In Figure 6B, HLA-A2⁺/melanoma cell line, 624.38, was the target tumor cell. In Figure 6C, the HLA-A2⁻/melanoma cell, 938, was used as target tumor cells. In Figure 6D, the HLA-A2⁺/osteosarcoma cell line was used as target tumor cells. In Figure 6E, the target tumor cells were Saos-2 cells.

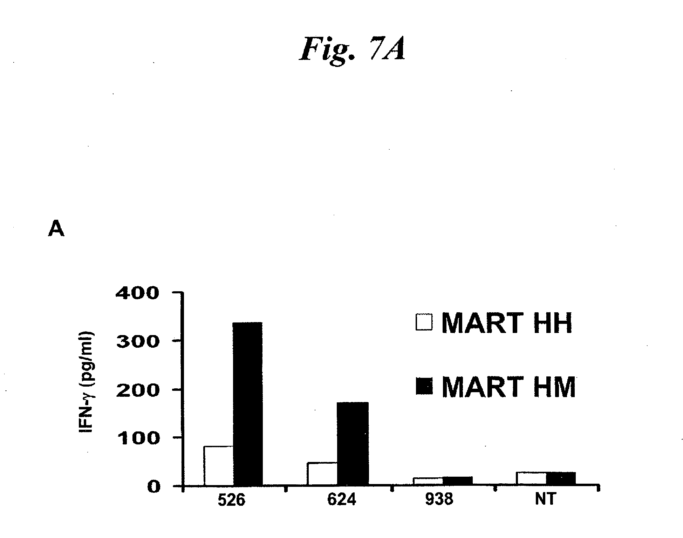

Figures 7A to 7C are graphs of the IFNγ, GM-CSF, and IL-2 secretion, respectively, as determined by ELISA, of purified CD4⁺ human PBLs expressing either the MART-HH (white bars) or the MART-HM (black bars) TCR and co-cultured with HLA-A2⁺/melanoma cell lines (526 and 624), HLA-A2⁻/melanoma cells (938), or with no tumor cells (NT).

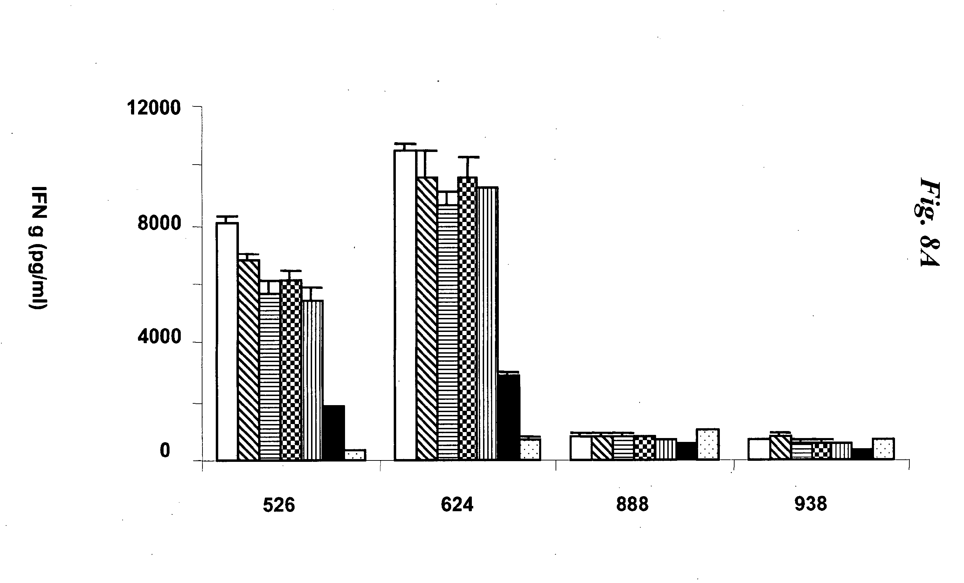

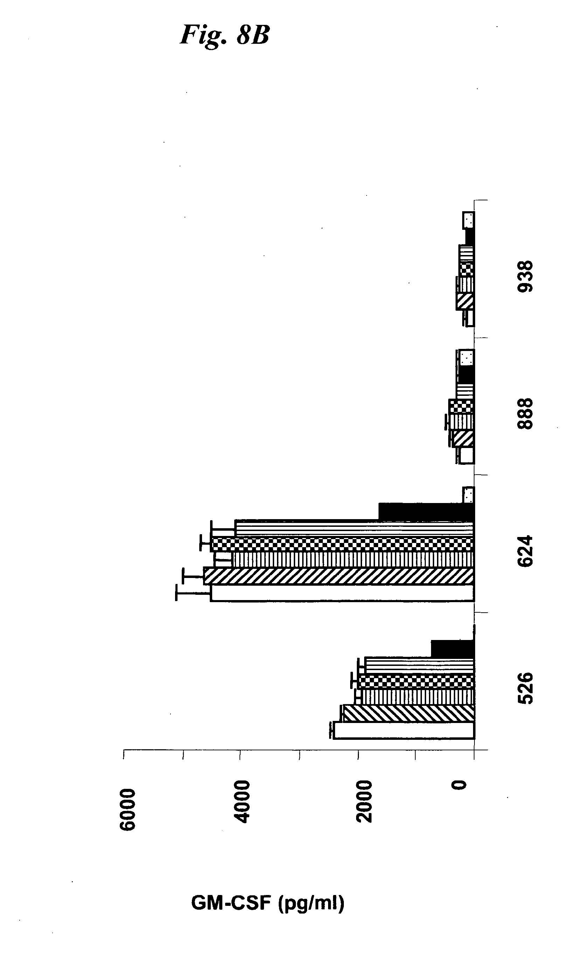

Figures 8A and 8B are graphs of the IFNγ and GM-CSF secretion, respectively, of human PBLs expressing MART-HH (black bars), MART-HM (white bars), F4-Mut31-alpha/MART-HM beta (diagonal lined bars), F4-Mut74-alpha/MART-HM beta (horizontal lined bars), F4-Cpa/MART-HM beta (checkered bars), MART-HM alpha/F4-Cpb (vertical lined bars), or no exogenous TCR (dotted bars) and co-cultured with HLA-A2⁺/melanoma cell lines (526 and 624) or HLA-A2⁻/melanoma cells (938 or 888).

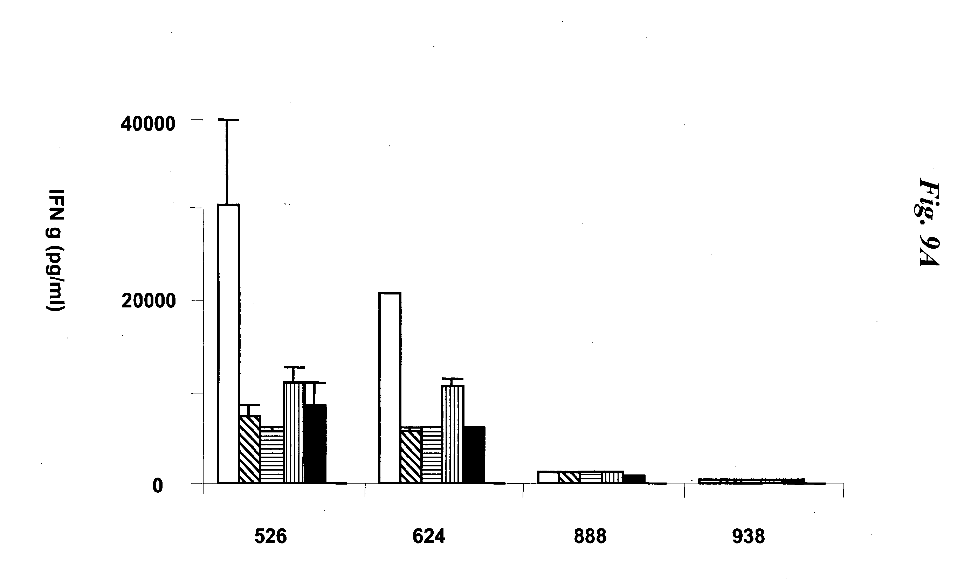

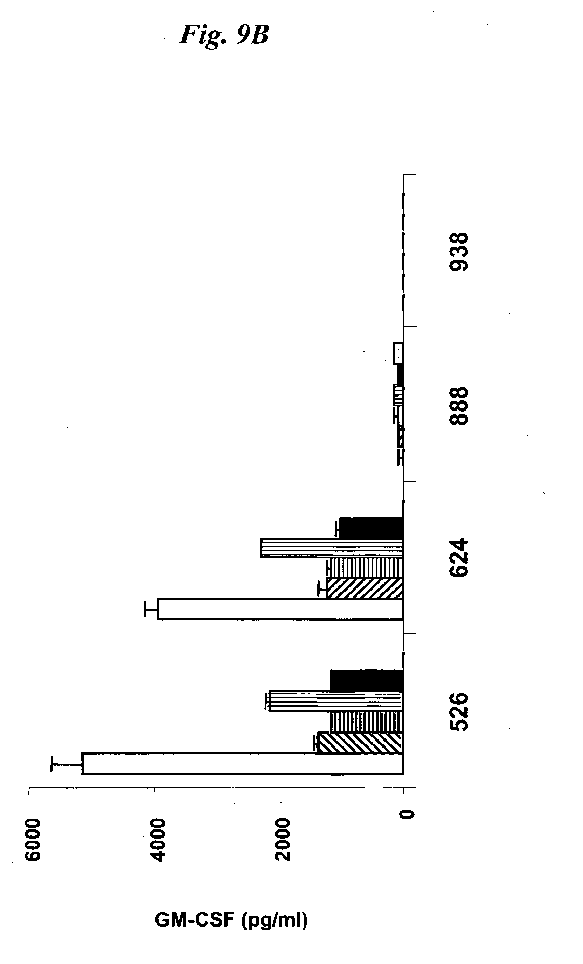

Figures 9A and 9B are graphs of the IFNγ and GM-CSF secretion, respectively, of human PBLs expressing MART-HH (black bars), MART-HM (white bars), MART-HM alpha/F4-Mut62-beta (diagonal lined bars), MART-HM alpha/F4-Mut97-beta (horizontal lined bars), F4-Cpa/F4Cpb (vertical lined bars), or no exogenous TCR (dotted bars) and co-cultured with HLA-A2⁺/melanoma cell lines (526 and 624) or HLA-A2⁻/melanoma cells (938 or 888).

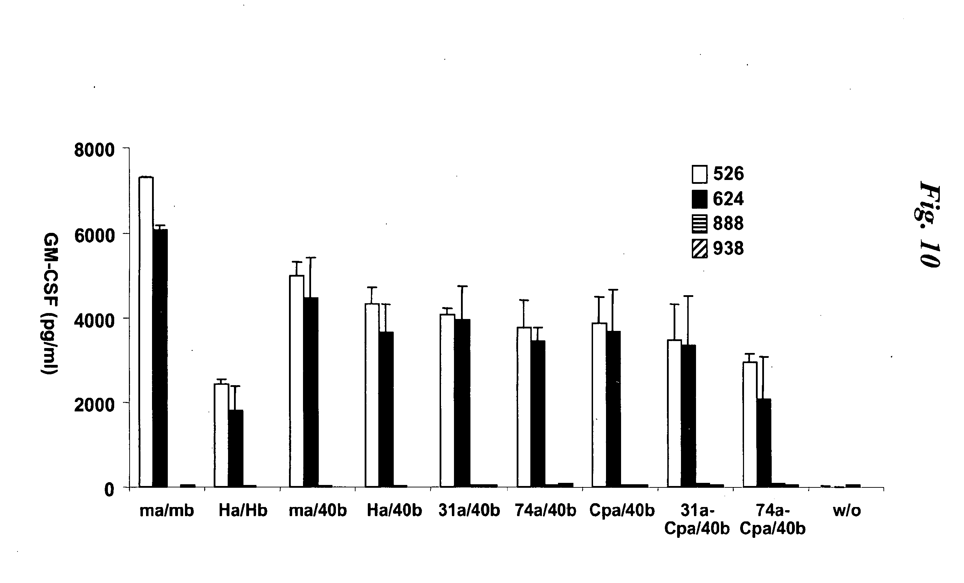

Figure 10 is a graph of the GM-CSF secretion of human PBLs expressing the indicated TCR and co-cultured with 526 cells (white bars), 624 cells (black bars), 888 cells (horizontal lined bars), or 938 (diagonal lined bars).

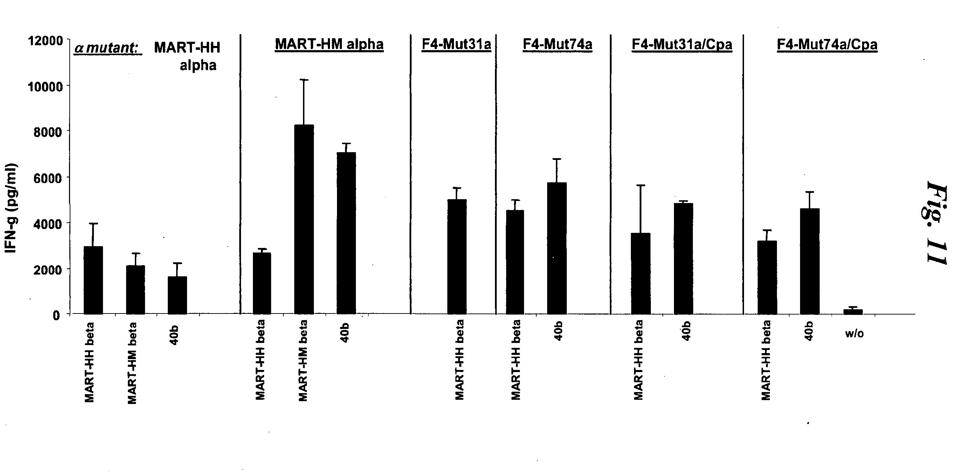

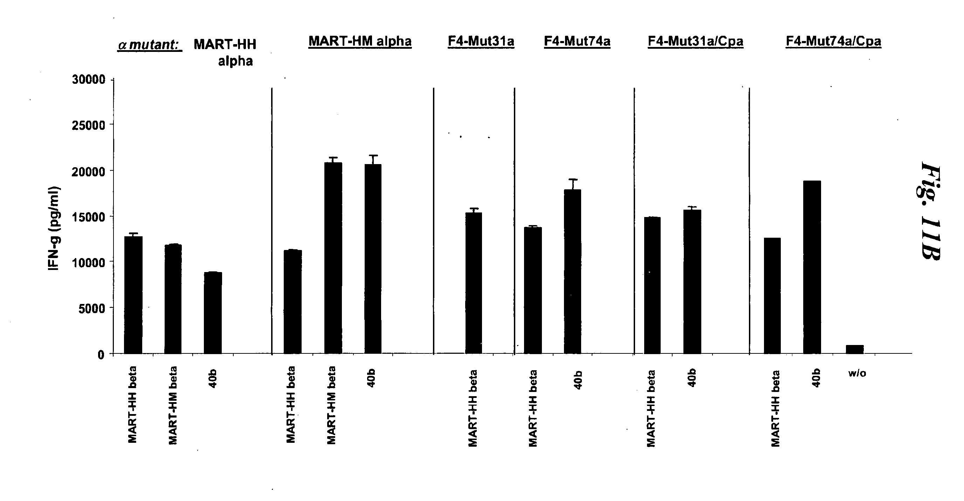

Figures 11A and 11B are graphs of the IFNγ secretion of human PBLs expressing the indicated alpha and beta chains and co-cultured with 526 cells (Figure 11A) or 624 cells (Figure 11B).

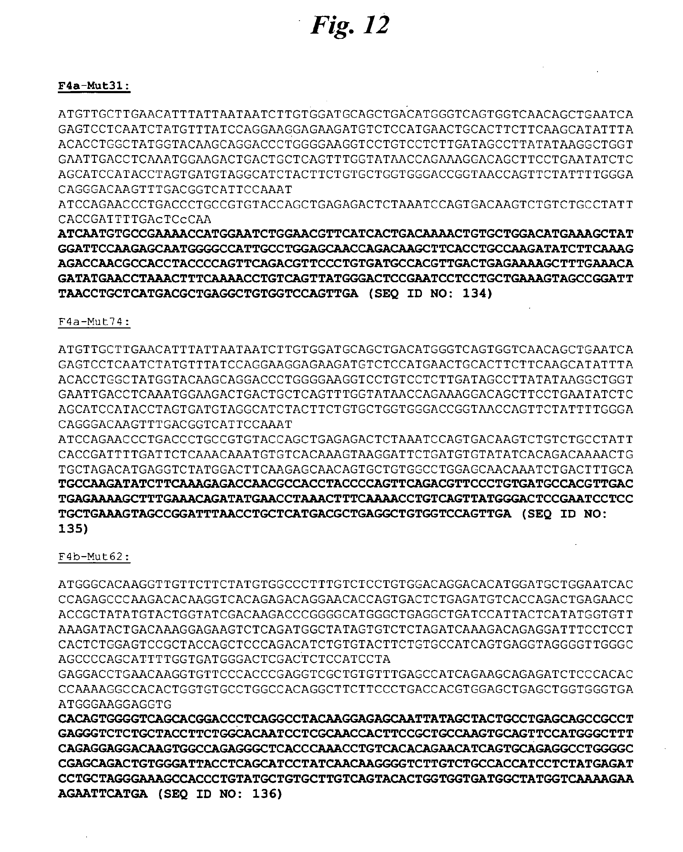

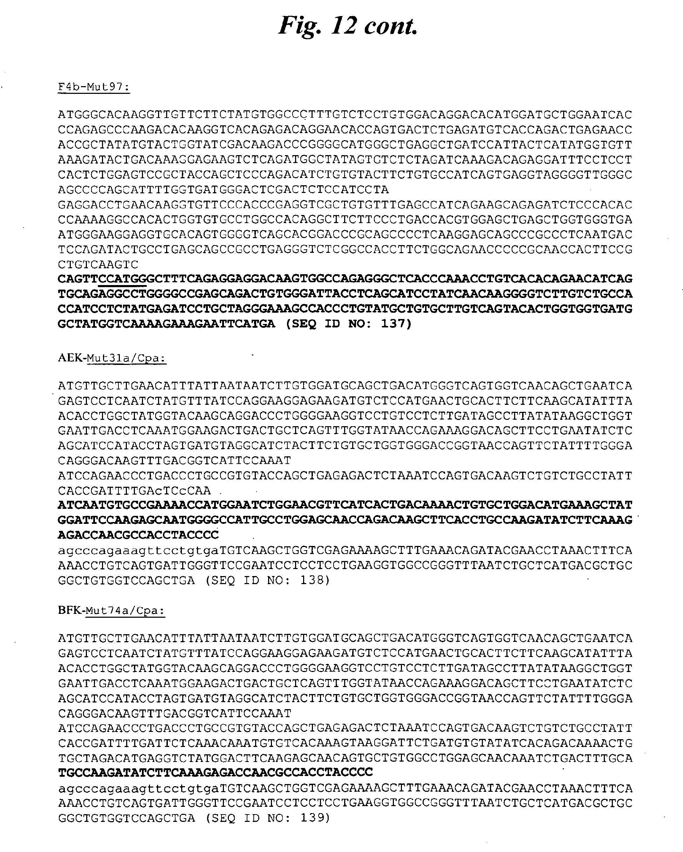

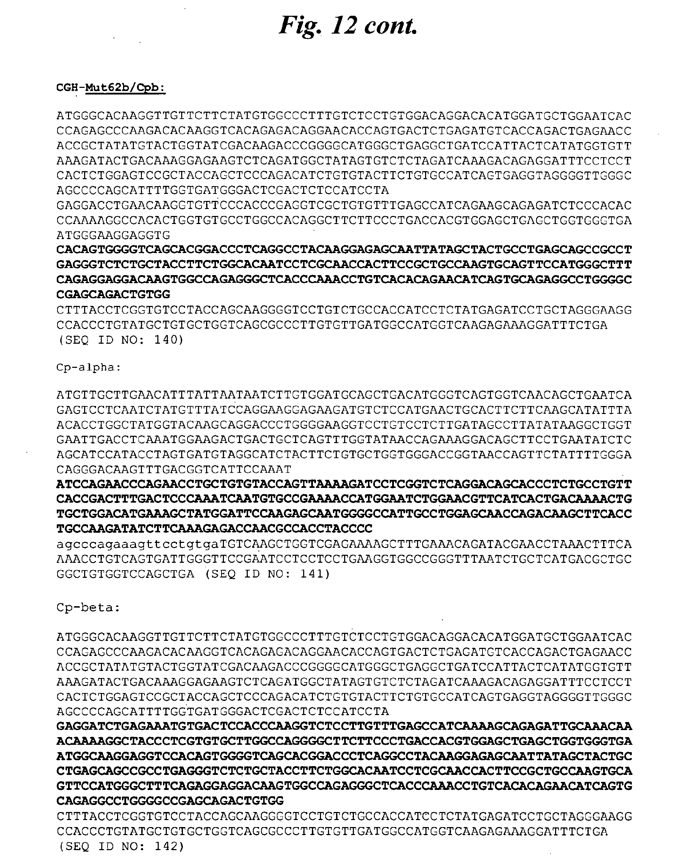

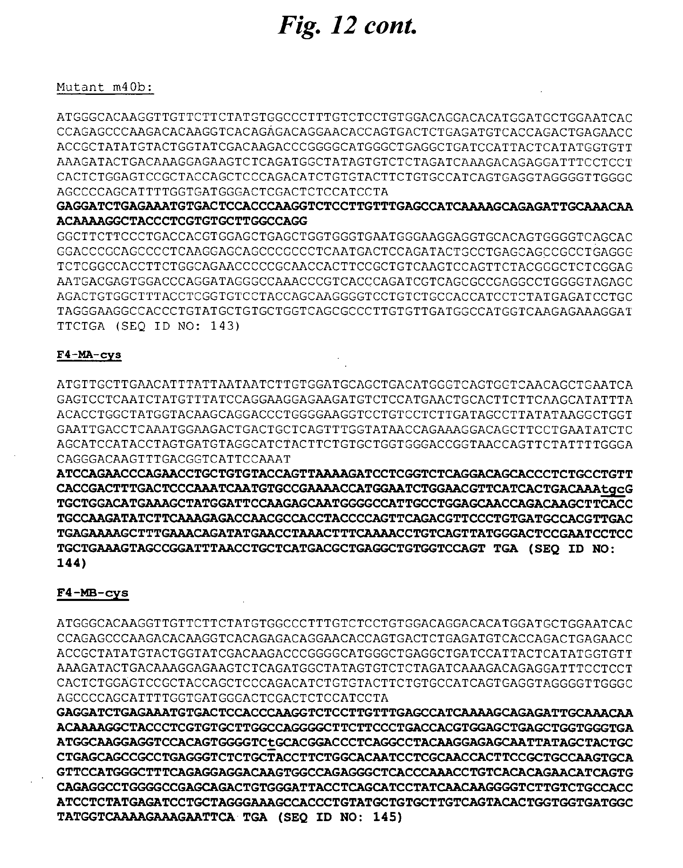

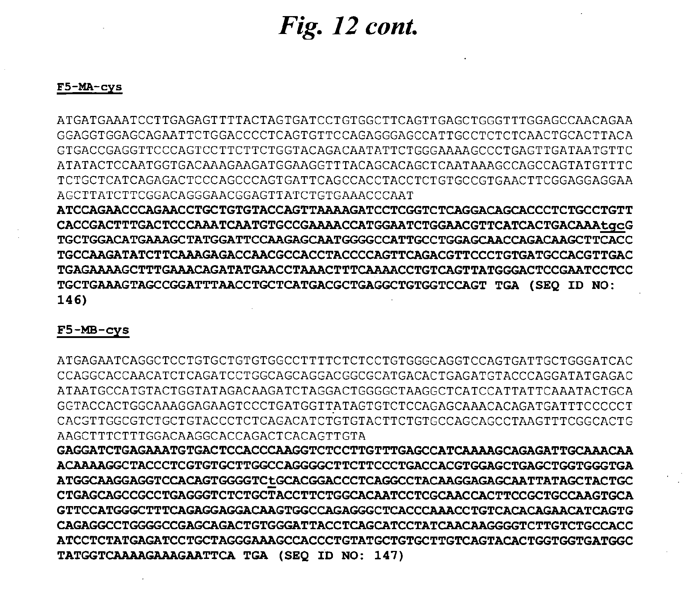

Figure 12 is a listing of nucleotide sequences encoding functional variant TCR chains described herein. The bold nucleotides represent sequence derived from mouse and the unbolded nucleotides represent sequence derived from human.

### DETAILED DESCRIPTION OF THE INVENTION

The invention provides chimeric T cell receptors (TCRs) which exhibit enhanced biological activity. For example, the inventive chimeric TCRs are marked by their higher capacity for cell surface expression, stronger association with CD3, increased ability to secrete cytokines (e.g., IFNγ, GM-CSF, and IL-2) upon antigen stimulation, and enhanced ability to recognize and kill tumor cells. The inventive chimeric TCRs comprise a variable region of a human TCR and a constant region comprising at least an extracellular domain of a constant region of a non-human TCR.

As used herein, the term "chimeric" refers to a molecule, e.g., a TCR, composed of parts of different origins. A chimeric molecule, as a whole, is non-naturally occurring, e.g., synthetic or recombinant, although the parts which comprise the chimeric molecule can be naturally occurring.

For purposes herein, the term "human" or "non-human," when used in reference to a TCR, or a part thereof (e.g., a variable region, a constant region, an α chain, a β chain), is meant that the TCR, or part thereof, is a TCR, or part thereof, that is endogenously expressed by a cell of the "human" or "non-human". For example, the phrase "human TCR" means that the TCR is endogenous to (naturally occurs in) a human. Also, for purposes herein, "non-human" refers to any host, such as any host mentioned herein, which is not a human..

The chimeric TCRs of the invention can have antigenic specificity for any antigen. The phrase "have antigenic specificity" as used herein means that the TCR can specifically bind to and immunologically recognize the antigen, such that binding of the TCR to the antigen elicits an immune response. In a preferred embodiment of the invention, the antigen is an antigen which is characteristic of a disease, e.g., an infectious disease, an autoimmune disease, cancer, etc. In a more preferred embodiment, the antigen is a cancer antigen.

The term "cancer antigen" as used herein refers to any molecule (e.g., protein, peptide, lipid, carbohydrate, etc.) solely or predominantly expressed or over-expressed by a tumor cell or cancer cell, such that the antigen is associated with the tumor or cancer. The cancer antigen can additionally be expressed by normal, non-tumor, or non-cancerous cells. However, in such cases, the expression of the cancer antigen by normal, non-tumor, or non-cancerous cells is not as robust as the expression by tumor or cancer cells. In this regard, the tumor or cancer cells can over-express the antigen or express the antigen at a significantly higher level, as compared to the expression of the antigen by normal, non-tumor, or non-cancerous cells. Also, the cancer antigen can additionally be expressed by cells of a different state of development or maturation. For instance, the cancer antigen can be additionally expressed by cells of the embryonic or fetal stage, which cells are not normally found in an adult host. Alternatively, the cancer antigen can be additionally expressed by stem cells or precursor cells, which cells are not normally found in an adult host.

The cancer antigen can be an antigen expressed by any cell of any cancer or tumor, including the cancers and tumors described herein. The cancer antigen may be a cancer antigen of only one type of cancer or tumor, such that the cancer antigen is associated with or characteristic of only one type of cancer or tumor. Alternatively, the cancer antigen may be a cancer antigen (e.g., may be characteristic) of more than one type of cancer or tumor. For example, the cancer antigen may be expressed by both breast and prostate cancer cells and not expressed at all by normal, non-tumor, or non-cancer cells. In a preferred embodiment of the invention, the cancer antigen is a melanoma antigen. In a more preferred embodiment, the cancer antigen is MART-1, gp-100, p53, orNY-ESO-1.

The inventive chimeric TCR can comprise two polypeptides (i.e., polypeptide chains), such as an α chain of a TCR, a β chain of a TCR, a γ chain of a TCR, a δ chain of a TCR, or a combination thereof, each of which comprises a variable region and a constant region. Such polypeptide chains of TCRs are known in the art. The polypeptides of the inventive chimeric TCR can comprise any variable region and any constant region, provided that the variable region is a variable region of a human TCR and the constant region comprises at least an extracellular domain of a constant region of a non-human TCR. The non-human can independently be any host, such as any of the hosts described herein, which is not a human. In this respect, the chimeric TCR can, for instance, comprise two polypeptides, each of which comprises a variable region of a human TCR and a constant region of a murine TCR.

The constant region of the inventive chimeric TCRs can comprise any amino acid sequence, provided that the constant region comprises at least an extracellular domain of a constant region of a TCR of a non-human host. The constant region of the inventive chimeric TCR can, for example, comprise an extracellular domain of a constant region of a murine TCR, a sheep TCR, a goat TCR, etc. Without being bound to any particular theory, it is contemplated that the extracellular domain of the constant region of the chimeric TCRs, in part, confers the enhanced biological activities of the chimeric TCRs.

In a preferred embodiment of the invention, the chimeric TCR comprises a constant region comprising an amino acid sequence of at least the extracellular domain of a constant region of a murine TCR. In this regard, the chimeric TCR can comprise a constant region comprising any of SEQ ID NOs: 1 to 3, or a combination thereof, e.g., SEQ ID NOs: 1 and 2 or SEQ ID NOs: 1 and 3. In the instance that the extracellular domain is of a murine constant region, the constant region of the chimeric TCR can comprise the extracellular domain of a murine constant region in combination with other parts, e.g., a transmembrane domain and/or an intracellular domain, of a constant region of a murine TCR or of a non-murine TCR. For example, the constant region of the chimeric TCR can comprise an extracellular domain of a constant region of a murine TCR in combination with a transmembrane domain and an intracellular domain of a constant region of a human TCR. Alternatively, the constant region of the chimeric TCR can comprise an extracellular domain, a transmembrane domain, and an intracellular domain of a constant region of a murine TCR. In this respect, the chimeric TCR can comprise a constant region comprising any of SEQ ID NOs: 4 to 6, or a combination thereof, e.g., SEQ ID NOs: 4 and 5 or SEQ ID NOs: 4 and 6.

Alternatively or additionally, the chimeric TCR can comprise any variable region of a human TCR. In this regard, the chimeric TCR can comprise a variable region comprising the amino acid sequence of any of SEQ ID NOs: 7 to 14. In a preferred embodiment of the invention, the chimeric TCR comprises a variable region of an α chain and a variable region of a β chain. In a more preferred embodiment, the variable region of the α chain comprises an amino acid sequence selected from the group consisting of SEQ ID NOs: 7, 9, 11, and 13, and the variable region of the β chain comprises an amino acid sequence selected from the group consisting of SEQ ID NOs: 8, 10, 12, or 14. In a most preferred embodiment, the chimeric TCR comprises a combination of variable regions, one on each polypeptide chain, comprising a combination of amino acid sequences selected from the group consisting of SEQ ID NOs: 7 and 8, 9 and 10, 11 and 12, and 13 and 14.

Alternatively or additionally, the chimeric TCR can comprise an α chain of a TCR and a β chain of a TCR. Each of the α chain and β chain of the inventive chimeric TCR can independently comprise any amino acid sequence. Preferably, the α chain comprises the variable region of an α chain as set forth above. In this regard, the inventive TCR can comprise the amino acid sequence of any of SEQ ID NOs: 15,18,21, and 24 (amino acid sequences of α chain). An inventive TCR of this type can be paired with any β chain of a TCR. Preferably, the β chain of the inventive TCR comprises the variable region of a β chain as set forth above. In this regard, the inventive chimeric TCR can comprise the amino acid sequence of any of SEQ ID NOs: 16, 17, 19, 20, 22, 23, 25, and 26. The inventive TCR, therefore, can comprise the amino acid sequence of any of SEQ ID NOs: 15 to 26, or a combination thereof, e.g., SEQ ID NOs: 15 and 16, 15 and 17, 18 and 19, 18 and 20, 21 and 22, 21 and 23, 24 and 25, or 24 and 26.

Also provided by the invention is an isolated or purified polypeptide comprising the amino acid sequence of any of SEQ ID NOs: 15 to 26 and 86 to 133. The term "polypeptide" as used herein includes oligopeptides and refers to a single chain of amino acids connected by one or more peptide bonds.

The invention further provides an isolated or purified protein comprising at least one of the polypeptides described herein. By "protein" is meant a molecule comprising one or more polypeptide chains. The protein of the invention can comprise, for example, 1, 2, 3, 4, 5, or more polypeptide chains. In a preferred embodiment, the inventive protein comprises two polypeptide chains. In a more preferred embodiment, the inventive protein comprises two polypeptide chains, each of which independently comprises an amino acid sequence selected from the group consisting of SEQ ID NOs: 15-26 and 86 to 133. In a most preferred embodiment of the invention, the protein comprises a first polypeptide chain and a second polypeptide chain, wherein the first polypeptide chain and second polypeptide chain respectively comprise: SEQ ID NOs: 15 and 16, SEQ ID NOs: 15 and 17, SEQ ID NOs: 18 and 19, SEQ ID NOs: 18 and 20, SEQ ID NOs: 21 and 22, SEQ ID NOs: 21 and 23, SEQ ID NOs: 24 and 25, SEQ ID NOs: 24 and 26, SEQ ID NOs: 112 and 16, SEQ ID NOs: 113 and 16, SEQ ID NOs: 115 and 16, SEQ ID NOs: 116 and 16, SEQ ID NOs: 112 and 33, SEQ ID NOs: 113 and 33, SEQ ID NOs: 114 and 33, SEQ ID NOs: 115 and 33, SEQ ID NOs: 15 and 120, SEQ ID NOs: 116 and 120, SEQ ID NOs: 15 and 121, SEQ ID NOs: 32 and 121, SEQ ID NOs: 112 and 121, SEQ ID NOs: 113 and 121, SEQ ID NOs: 116 and 121, SEQ ID NOs: 114 and 121, or SEQ ID NOs: 115 and 121.

Alternatively, the protein can comprise a single polypeptide chain comprising two or more polypeptides fused together. In this instance, the protein can be a fusion protein. In a preferred embodiment of the invention, the fusion protein comprises a single polypeptide comprising two of the inventive polypeptides described herein which are fused together, e.g., a single polypeptide comprising SEQ ID NOs: 15 and 16, SEQ ID NOs: 15 and 17, SEQ ID NOs: 18 and 19, SEQ ID NOs: 18 and 20, SEQ ID NOs: 21 and 22, SEQ ID NOs: 21 and 23, SEQ ID NOs: 24 and 25, SEQ ID NOs: 24 and 26, SEQ ID NOs: 112 and 16, SEQ ID NOs: 113 and 16, SEQ ID NOs: 115 and 16, SEQ ID NOs: 116 and 16, SEQ ID NOs: 112 and 33, SEQ ID NOs: 113 and 33, SEQ ID NOs: 114 and 33, SEQ ID NOs: 115 and 33, SEQ ID NOs: 15 and 120, SEQ ID NOs: 116 and 120, SEQ ID NOs: 15 and 121, SEQ ID NOs: 32 and 121, SEQ ID NOs: 112 and 121, SEQ ID NOs: 113 and 121, SEQ ID NOs: 116 and 121, SEQ ID NOs: 114 and 121, or SEQ ID NOs: 115 and 121.

Alternatively, the fusion protein can comprise a polypeptide of a variable region of a first TCR chain (e.g., an α chain) and a polypeptide of an entire (full-length) second TCR chain (e.g., a β chain). For instance, the fusion protein can comprise a first polypeptide comprising an amino acid sequence of any of SEQ ID NOs: 7-14 and a second polypeptide comprising an amino acid sequence of any of SEQ ID NOs: 15-26. For instance, the fusion protein can comprise SEQ ID NO: 7 and SEQ ID NO: 16 or 17; SEQ ID NOs: 8 and 15; SEQ ID NO: 9 and SEQ ID NO: 19 or 20; SEQ ID NOs: 10 and 18; SEQ ID NO: 11 and SEQ ID NO: 22 or 23; SEQ ID NOs: 12 and 21; SEQ ID NO: 13 and SEQ ID NOs: 25 or 26; SEQ ID NOs: 14 and 24.

The fusion protein can optionally comprise one or more linkers which join the two or more polypeptides together. The linker can be, for instance, a peptide (e.g., a FMDV 2A peptide (see Felipe, Genetic Vaccines and Therapy 2: 13 - e-publication September 13, 2004)) which joins together two polypeptides, as described herein.

Alternatively or additionally, the first and/or second polypeptide chain(s) of the fusion protein further can comprise(s) other amino acid sequences, e.g., an amino acid sequence encoding an immunoglobulin or a portion thereof. In this regard, the invention also provides a fusion protein comprising at least one of the inventive polypeptides described herein along with at least one other polypeptide. The other polypeptide can exist as a separate polypeptide of the fusion protein, or can exist as a polypeptide, which is expressed in frame (in tandem) with one of the inventive polypeptides described herein. The other polypeptide can encode any peptidic or proteinaceous molecule, or a portion thereof, including, but not limited to an immunoglobulin, CD3, CD4, CD8, an MHC molecule, etc.

The fusion protein can comprise one or more copies of the inventive polypeptide and/or one or more copies of the other polypeptide. For instance, the fusion protein can comprise 1, 2, 3, 4, 5, or more, copies of the inventive polypeptide and/or of the other polypeptide. Suitable methods of making fusion proteins are known in the art, and include, for example, recombinant methods. See, for instance, Choi et al., Mol. Biotechnol., 31, 193-202(2005).

The protein of the invention can be a recombinant antibody comprising at least one of the inventive polypeptides described herein. As used herein, "recombinant antibody" refers to a recombinant (e.g., genetically engineered) protein comprising at least one of the polypeptides of the invention and a polypeptide chain of an antibody, or a portion thereof. The polypeptide of an antibody, or portion thereof, can be a heavy chain, a light chain, a variable or constant region of a heavy or light chain, a single chain variable fragment (scFv), or an Fc, Fab, or F(ab)₂' fragment of an antibody, etc. The polypeptide chain of an antibody, or portion thereof, can exist as a separate polypeptide of the recombinant antibody. Alternatively, the polypeptide chain of an antibody, or portion thereof, can exist as a polypeptide, which is expressed in frame (in tandem) with the polypeptide of the invention. The polypeptide of an antibody, or portion thereof, can be a polypeptide of any antibody or any antibody fragment, including any of the antibodies and antibody fragments described herein.

Included in the scope of the invention are functional variants of the inventive chimeric TCRs, polypeptides, and proteins described herein. The term "functional variant" as used herein refers to a TCR, polypeptide, or protein having substantial or significant sequence identity or similarity to a parent TCR, polypeptide, or protein, which functional variant retains the biological activity of the TCR, polypeptide, or protein of which it is a variant. Functional variants encompass, for example, those variants of the TCR, polypeptide, or protein described herein (the parent TCR, polypeptide, or protein) that retain the ability to specifically bind to the cancer antigen for which the parent TCR has antigenic specificity or to which the parent polypeptide or protein specifically binds, to a similar extent, the same extent, or to a higher extent, as the parent TCR, polypeptide, or protein. In reference to the parent TCR, polypeptide, or protein, the functional variant can, for instance, be at least about 30%, 50%, 75%, 80%, 90%, 98% or more identical in amino acid sequence to the parent TCR, polypeptide, or protein.

The functional variant can comprise any amino acid sequence provided that the amino acid sequence has significant sequence identity to the amino acid sequence of the parent TCR. In a preferred embodiment of the invention, the functional variant has an amino acid sequence that is at least about 75% identical to the amino acid sequence of the parent TCR. In a more preferred embodiment of the invention, the functional variant has an amino acid sequence that is at least about 80% identical to the amino acid sequence of the parent TCR. In a most preferred embodiment of the invention, the functional variant has an amino acid sequence that is at least about 90% identical to the amino acid sequence of the parent TCR.

J The amino acid sequence of the functional variant can comprise, for example, the amino acid sequence of the parent TCR, polypeptide, or protein with at least one conservative amino acid substitution. Conservative amino acid substitutions are known in the art, and include amino acid substitutions in which one amino acid having certain physical and/or chemical properties is exchanged for another amino acid that has the same chemical or physical properties. For instance, the conservative amino acid substitution can be an acidic amino acid substituted for another acidic amino acid (e.g., Asp or Glu), an amino acid with a nonpolar side chain substituted for another amino acid with a nonpolar side chain (e.g., Ala, Gly, Val, Ile, Leu, Met, Phe, Pro, Trp, Val, etc.), a basic amino acid substituted for another basic amino acid (Lys, Arg, etc.), an amino acid with a polar side chain substituted for another amino acid with a polar side chain (Asn, Cys, Gln, Ser, Thr, Tyr, etc.), etc.

Alternatively or additionally, the functional variants can comprise the amino acid sequence of the parent TCR, polypeptide, or protein with at least one non-conservative amino acid substitution. In this case, it is preferable for the non-conservative amino acid substitution to not interfere with or inhibit the biological activity of the functional variant. Preferably, the non-conservative amino acid substitution enhances the biological activity of the functional variant, such that the biological activity of the functional variant is increased as compared to the parent TCR, polypeptide, or protein.

The amino acid substitution(s) of the amino acid sequence of the functional variant can be within any region of the amino acid sequence. For example, the amino acid substitution(s) can be located within the region of the amino acid sequence which encodes the variable region or the constant region of the functional variant. In the instance that the amino acid substitution(s) is/are located within the region of the amino acid sequence which encodes the variable region, it is understood that the amino acid substitution(s) do not significantly decrease the ability of the functional variant to bind to the antigen for which the parent TCR has antigenic specificity.

Preferably, the amino acid substitution(s) of the amino acid sequence of the functional variant is/are located within the region of the amino acid sequence which encodes the constant region of the functional variant. In this regard, the functional variant can, for example, comprise a chimeric constant region in which one or more portions of the constant region of the functional variant is derived from a non-human constant region, e.g., a murine constant region, and the other portion(s) of the constant region is/are derived from a human constant region. For purposes herein, the term "portion" is meant any suitable portion of a constant region, such as a portion comprising the extracelluar domain, the connecting peptide, the transmembrane domain, or the intracellular domain of the constant region. The portion can, for instance, comprise at least 20, 21, 30, 40, 50, or 60 contiguous amino acids of the referenced constant region.

The functional variant can, for instance, comprise a chimeric constant region in which the first 31 amino acids are derived from a human constant region and the remaining portion is derived from the murine constant region. Also, for instance, the functional variant can comprise a chimeric constant region in which the first 74 amino acids are derived from a human constant region and the remaining portion is derived from the murine constant region. Other exemplary functional variants comprising chimeric constant regions are described herein at Table 7.

In this regard, the invention provides a functional variant comprising the amino acid sequence of any of SEQ ID NOs: 86-95, which sequences comprise human portions and murine portions of the extracellular domain of the constant region. In a preferred embodiment of the invention, the functional variant comprises a chimeric constant region comprising an amino acid sequence of any of SEQ ID NOs: 99-108. In a more preferred embodiment of the invention, the functional variant comprises an amino acid sequence selected from the group consisting of SEQ ID NOs: 112 to 121, which sequences are encoded by the nucleotide sequences of SEQ ID NOs: 134-147. In a most preferred embodiment of the invention, the functional variant comprises a first polypeptide chain and a second polypeptide chain, wherein the first polypeptide chain and the second polypeptide chain respectively comprise an amino acid sequence selected from the group consisting of SEQ ID NOs: 112 and 16, SEQ ID NOs: 113 and 16, SEQ ID NOs: 116 and 16, SEQ ID NOs: 112 and 33, SEQ ID NOs: 113 and 33, SEQ ID NOs: 114 and 33, SEQ ID NOs:115 and 33, SEQ ID NOs: 15 and 120, SEQ ID NOs: 116 and 120, SEQ ID NOs: 15 and 121, SEQ ID NOs: 32 and 121, SEQ ID NOs: 112 and 121, SEQ ID NOs: 113 and 121, SEQ ID NOs: 114 and 121, SEQ ID NOs: 115 and 121, and SEQ ID NOs: 116 and 121.

Alternatively, the functional variant can comprise a constant region of a non-human, e.g., murine, TCR with an amino acid substitution in which an amino acid has been substituted with a cysteine residue (Cys). The functional variant can, for instance, comprise an extracellular domain comprising an amino acid sequence selected from the group consisting of SEQ ID NOs: 96-98, which sequences comprise an extracellular domain of a murine constant region in which an amino acid has been replaced with Cys. Preferably, the functional variant comprises an amino acid sequence selected from the group consisting of SEQ ID NOs: 109-111, which sequences comprise a murine constant region in which an amino acid has been replaced with Cys. More preferably, the functional variant comprises an amino acid sequence selected from the group consisting of SEQ ID NOs: 122 to 133. Most preferably, the functional variant comprises a first polypeptide chain and a second polypeptide chain, wherein the first polypeptide chain and the second polypeptide chain respectively comprise an amino acid sequence selected from SEQ ID NOs: 122 and 123, SEQ ID NOs: 122 and 124, SEQ ID NOs: 125 and 126, SEQ ID NOs: 125 and 127, SEQ ID NOs: 128 and 129, SEQ ID NOs: 128 and 130, SEQ ID NOs: 131 and 132, and SEQ ID NOs: 131 and 133.

The TCR, polypeptide, or protein can consist essentially of the specified amino acid sequence or sequences described herein, such that other components of the functional variant, e.g., other amino acids, do not materially change the biological activity of the functional variant. In this regard, the inventive TCR, polypeptide, or protein can, for example, consist essentially of the amino acid sequence of SEQ ID NO: 15 or 16, or both SEQ ID NOs: 15 and 16, the amino acid sequence of SEQ ID NO: 15 or 17, or both SEQ ID NOs: 15 and 17, the amino acid sequence of SEQ ID NO: 18 or 19, or both SEQ ID NOs: 18 and 19, the amino acid sequence of SEQ ID NO: 18 or 20, or both SEQ ID NOs: 18 and 20, the amino acid sequence of SEQ ID NO: 21 or 22, or both SEQ ID NOs: 21 and 22, the amino acid sequence of SEQ ID NO: 21 or 23, or both SEQ ID NOs: 21 and 23, the amino acid sequence of SEQ ID NO: 24 or 25, or both SEQ ID NOs: 24 and 25, or the amino acid sequence of SEQ ID NO: 24 or 26, or both SEQ ID NOs: 24 and 26.

Likewise, the functional variant can, for example, consist essentially of SEQ ID NO: 112 or 16, SEQ ID NO: 113 or 16, SEQ ID NO:116 or 16, SEQ ID NO: 112 or 33, SEQ ID NO: 113 or 33, SEQ ID NO: 114 or 33, SEQ ID NO:115 or 33, SEQ ID NO: 15 or 120, SEQ ID NO: 116 or 120, SEQ ID NO: 15 or 121, SEQ ID NO: 32 or 121, SEQ ID NO: 112 or 121, SEQ ID NO: 113 or 121, SEQ ID NO:114 or 121, SEQ ID NO: 115 or 121, SEQ ID NO: 116 or 121, SEQ ID NO: 122 or 123, SEQ ID NO: 122 or 124, SEQ ID NO: 125 or 126, SEQ ID NO: 125 or 127, SEQ ID NO: 128 or 129, SEQ ID NO: 128 or 130, SEQ ID NO: 131 or 132, or SEQ ID NO: 131 or 133, or the functional variant can consist essentially of both of the specified sequences.

The TCRs, polypeptides, and proteins of the invention (including functional portions and functional variants) can be of any length, i.e., can comprise any number of amino acids, provided that the TCRs, polypeptides, or proteins (or functional portions or functional variants thereof) retain their biological activity. For example, the polypeptide can be about 50 to about 5000 amino acids long, such as 50, 70, 75, 100, 125, 150, 175, 200, 300, 400, 500, 600, 700, 800, 900, 1000 or more amino acids in length. In this regard, the polypeptides of the invention also include oligopeptides.

Included in the scope of the invention are functional portions of the inventive chimeric TCRs, polypeptides, and proteins described herein. The functional portions can comprise any portion comprising contiguous amino acids of the inventive TCR, polypeptide, or protein of which it is a part, provided that the functional portion comprises a variable region of a first host and a constant region comprising at least an extracellular domain of a constant region of a second host. The term "functional portion" when used in reference to a TCR refers to any part or fragment of the TCR of the invention, which part or fragment retains the biological activity of the TCR of which it is a part (the parent TCR). Functional portions encompass, for example, those parts of a TCR that retain the ability to, e.g., specifically bind to a cancer antigen or detect a diseased cell, treat or prevent a disease, e.g., cancer, to a similar extent, the same extent, or to a higher extent, as the parent TCR. In reference to the parent TCR, the functional portion can comprise, for instance, about 10%, 25%, 30%, 50%, 68%, 80%, 90%, 95%, or more, of the parent TCR.

The functional portion can comprise additional amino acids at the amino or carboxy terminus of the portion, or at both termini, which additional amino acids are not found in the amino acid sequence of the parent TCR. Desirably, the additional amino acids do not interfere with the biological function of the functional portion, e.g., specifically bind to a cancer antigen or detect a diseased cell, treat or prevent a disease, e.g., cancer, etc. More desirably, the additional amino acids enhance the biological activity, as compared to the biological activity of the parent TCR.

The TCRs, polypeptides, and proteins of the invention (including functional portions and functional variants) of the invention can comprise synthetic amino acids in place of one or more naturally-occurring amino acids. Such synthetic amino acids are known in the art, and include, for example, aminocyclohexane carboxylic acid, norleucine, α-amino n-decanoic acid, homoserine, S-acetylaminomethyl-cysteine, trans-3- and trans-4-hydroxyproline, 4-aminophenylalanine, 4- nitrophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, indoline-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, α-(2-amino-2-norbomane)-carboxylic acid, α,γ-diaminobutyric acid, α,β-diaminopropionic acid, homophenylalanine, and α-tert-butylglycine.

The TCRs, polypeptides, and proteins of the invention (including functional portions and functional variants) can be glycosylated, amidated, carboxylated, phosphorylated, esterified, N-acylated, cyclized via, e.g., a disulfide bridge, or converted into an acid addition salt and/or optionally dimerized or polymerized, or conjugated.

When the TCRs, polypeptides, and proteins of the invention (including functional portions and functional variants) are in the form of a salt, preferably, the polypeptides are in the form of a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, *p*-toluenesulphonic acid.

The TCR, polypeptide, and/or protein of the invention (including functional portions and functional variants thereof) can be obtained by methods known in the art. Suitable methods of *de novo* synthesizing polypeptides and proteins are described in, for example, Chan et al., Fmoc Solid Phase Peptide Synthesis, Oxford University Press, Oxford, United Kingdom, 2005; Peptide and Protein Drug Analysis, ed. Reid, R., Marcel Dekker, Inc., 2000; Epitope Mapping, ed. Westwood et al., Oxford University Press, Oxford, United Kingdom, 2000; and U.S. Patent No. 5,449,752. Also, polypeptides and proteins can be recombinantly produced using the nucleic acids described herein using standard recombinant methods. See, for instance, Sambrook et al., Molecular Cloning: A Laboratory Manual, 3rd ed., Cold Spring Harbor Press, Cold Spring Harbor, NY 2001; and Ausubel et al., Current Protocols in Molecular Biology, Greene Publishing Associates and John Wiley & Sons, NY, 1994. Further, some of the TCRs, polypeptides, and proteins of the invention (including functional portions and functional variants thereof) can be isolated and/or purified, in part, from a source, such as a plant, a bacterium, an insect, a mammal, e.g., a rat, a human, etc. Methods of isolation and purification are well-known in the art. Alternatively, the TCRs, polypeptides, and/or proteins described herein (including functional portions and functional variants thereof) can be commercially synthesized by companies, such as Synpep (Dublin, CA), Peptide Technologies Corp. (Gaithersburg, MD), and Multiple Peptide Systems (San Diego, CA). In this respect, the inventive TCRs, polypeptides, and proteins can be synthetic, recombinant, isolated, and/or purified.

Included in the scope of the invention are conjugates, e.g., bioconjugates, comprising any of the inventive TCRs, polypeptides, or proteins (including any of the functional portions or variants thereof), nucleic acids, recombinant expression vectors, host cells, populations of host cells, or antibodies, or antigen binding portions thereof. Conjugates, as well as methods of synthesizing conjugates in general, are known in the art (See, for instance, Hudecz, F., Methods Mol. Biol., 298: 209-223 (2005) and Kirin et al., Inorg Chem., 44(15): 5405-5415 (2005)).

Further provided by the invention is a nucleic acid comprising a nucleotide sequence encoding any of the TCRs, polypeptides, or proteins described herein (including functional portions and functional variants thereof). The nucleic acid can comprise any nucleotide sequence which encodes any of the TCRs, polypeptides, or proteins, or functional portions or functional variants thereof. For example, the nucleic acid can comprise a nucleotide sequence comprising any of SEQ ID NOs: 27-35 and 134-147. The nucleotide sequence alternatively can comprise a nucleotide sequence which is degenerate to any of SEQ ID NOs: 27-35 and 134-147.

Also provided is a primer nucleic acid comprising a nucleotide sequence which is complcmcntary to a portion of the nucleotide sequence encoding any of the TCRs, polypeptides, or proteins described herein (including functional portions and functional variants thereof). The inventive primer nucleic acid can be modified to comprise a detectable label, such as, for instance, a radioisotope, a fluorophore, and an element particle. The inventive primer nucleic acid is useful in detecting the nucleic acid which encodes the TCR, polypeptide, or protein. Both qualitative and quantitative analyses can be performed on cells comprising the inventive nucleic acid which encodes the TCR, polypeptide, or protein. Such analyses include, for example, any type of PCR based assay or hybridization assay, e.g., Southern blot, Northern blot.

By "nucleic acid" as used herein includes "polynucleotide," "oligonucleotide," and "nucleic acid molecule," and generally means a polymer of DNA or RNA, which can be single-stranded or double-stranded, synthesized or obtained (e.g., isolated and/or purified) from natural sources, which can contain natural, non-natural or altered nucleotides, and which can contain a natural, non-natural or altered internucleotide linkage, such as a phosphoroamidate linkage or a phosphorothioate linkage, instead of the phosphodiester found between the nucleotides of an unmodified oligonucleotide. It is generally preferred that the nucleic acid does not comprise any insertions, deletions, inversions, and/or substitutions. However, it may be suitable in some instances, as discussed herein, for the nucleic acid to comprise one or more insertions, deletions, inversions, and/or substitutions.

Preferably, the nucleic acids of the invention are recombinant. As used herein, the term "recombinant" refers to (i) molecules that are constructed outside living cells by joining natural or synthetic nucleic acid segments to nucleic acid molecules that can replicate in a living cell, or (ii) molecules that result from the replication of those described in (i) above. For purposes herein, the replication can be *in vitro* replication or *in vivo* replication.

The nucleic acids can be constructed based on chemical synthesis and/or enzymatic ligation reactions using procedures known in the art. See, for example, Sambrook et al., *supra,* and Ausubel et al., *supra.* For example, a nucleic acid can be chemically synthesized using naturally occurring nucleotides or variously modified nucleotides designed to increase the biological stability of the molecules or to increase the physical stability of the duplex formed upon hybridization (e.g., phosphorothioate derivatives and acridine substituted nucleotides). Examples of modified nucleotides that can be used to generate the nucleic acids include, but are not limited to, 5-fluorouracil, 5-bromouracil, 5-chlorouracil, 5-iodouracil, hypoxanthine, xanthine, 4-acetylcytosine, 5-(carboxyhydroxymethyl) uracil, 5-carboxymethylaminomethyl-2-thiouridine, 5-carboxymethylaminomethyluracil, dihydrouracil, beta-D-galactosylqueosine, inosine, N⁶-isopentenyladenine, 1-methylguanine, 1-methylinosine, 2,2-dimethylguanine, 2-methyladenine, 2-methylguanine, 3-methylcytosine, 5-methylcytosine, N⁶-substituted adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N⁶-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, 3-(3-amino-3-N-2-carboxypropyl) uracil, and 2,6-diaminopurine. Alternatively, one or more of the nucleic acids of the invention can be purchased from companies, such as Macromolecular Resources (Fort Collins, CO) and Synthegen (Houston, TX).

The nucleic acids of the invention can be incorporated into a recombinant expression vector. In this regard, the invention provides recombinant expression vectors comprising any of the nucleic acids of the invention. For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors of the invention are not naturally-occurring as a whole. However, parts of the vectors can be naturally-occurring. The inventive recombinant expression vectors can comprise any type of nucleotides, including, but not limited to DNA and RNA, which can be single-stranded or double-stranded, synthesized or obtained in part from natural sources, and which can contain natural, non-natural or altered nucleotides. The recombinant expression vectors can comprise naturally-occurring or non-naturally-occuring internucleotide linkages, or both types of linkages. Preferably, the altered nucleotides or non-naturally occurring internucleotide linkages do not hinder the transcription or replication of the vector.

The recombinant expression vector of the invention can be any suitable recombinant expression vector, and can be used to transform or transfect any suitable host. Suitable vectors include those designed for propagation and expansion or for expression or both, such as plasmids and viruses. The vector can be selected from the group consisting of the pUC series (Fermentas Life Sciences), the pBluescript series (Stratagene, LaJolla, CA), the pET series (Novagen, Madison, WI), the pGEX series (Pharmacia Biotech, Uppsala, Sweden), and the pEX series (Clontech, Palo Alto, CA). Bacteriophage vectors, such as λGT10, λGT11, λZapII (Stratagene), λEMBL4, and λNM1149, also can be used. Examples of plant expression vectors include pBI01, pBI101.2, pBI101.3, pBI121 and pBIN19 (Clontech). Examples of animal expression vectors include pEUK-Cl, pMAM and pMAMneo (Clontech). Preferably, the recombinant expression vector is a viral vector, e.g., a retroviral vector.

The recombinant expression vectors of the invention can be prepared using standard recombinant DNA techniques described in, for example, Sambrook et al., *supra,* and Ausubel et al., *supra.* Constructs of expression vectors, which are circular or linear, can be prepared to contain a replication system functional in a prokaryotic or eukaryotic host cell. Replication systems can be derived, e.g., from ColEl, 2µ plasmid, λ, SV40, bovine papilloma virus, and the like.

Desirably, the recombinant expression vector comprises regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based.

The recombinant expression vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G418 resistance genes, hygromycin resistance genes, histidinol resistance genes, tetracycline resistance genes, and ampicillin resistance genes.

The recombinant expression vector can comprise a native or nonnative promoter operably linked to the nucleotide sequence encoding the TCR, polypeptide, or protein (including functional portions and functional variants thereof), or to the nucleotide sequence which is complementary to or which hybridizes to the nucleotide sequence encoding the TCR, polypeptide, or protein. The selection of promoters, e.g., strong, weak, inducible, tissue-specific and developmental-specific, is within the ordinary skill of the artisan. Similarly, the combining of a nucleotide sequence with a promoter is also within the skill of the artisan. The promoter can be a non-viral promoter or a viral promoter, e.g., a cytomegalovirus (CMV) promoter, an SV40 promoter, an RSV promoter, and a promoter found in the long-terminal repeat of the murine stem cell virus.

The inventive recombinant expression vectors can be designed for either transient expression, for stable expression, or for both. Also, the recombinant expression vectors can be made for constitutive expression or for inducible expression. Further, the recombinant expression vectors can be made to include a suicide gene.

As used herein, the term "suicide gene" refers to a gene that causes the cell expressing the suicide gene to die. The suicide gene can be a gene that confers sensitivity to an agent, e.g., a drug, upon the cell in which the gene is expressed, and causes the cell to die when the cell is contacted with or exposed to the agent. Suicide genes are known in the art (see, for example, Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and include, for example, the Herpes Simplex Virus (HSV) thymidine kinase (TK) gene, cytosine daminase, purine nucleoside phosphorylase, and nitroreductase.

The invention further provides a host cell comprising any of the recombinant expression vectors described herein. As used herein, the term "host cell" refers to any type of cell that can contain the inventive recombinant expression vector. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. Suitable host cells are known in the art and include, for instance, DH5α *E. coli* cells, Chinese hamster ovarian cells, monkey VERO cells, COS cells, HEK293 cells, and the like. For purposes of amplifying or replicating the recombinant expression vector, the host cell is preferably a prokaryotic cell, e.g., a DH5α cell. For purposes of producing a recombinant TCR, polypeptide, or protein, the host cell is preferably a mammalian cell. Most preferably, the host cell is a human cell. While the host cell can be of any cell type, can originate from any type of tissue, and can be of any developmental stage, the host cell preferably is a peripheral blood lymphocyte (PBL). More preferably, the host cell is a T cell.

For purposes herein, the T cell can be any T cell, such as a cultured T cell, e.g., a primary T cell, or a T cell from a cultured T cell line, e.g., Jurkat, SupT1, etc., or a T cell obtained from a mammal. If obtained from a mammal, the T cell can be obtained from numerous sources, including but not limited to blood, bone marrow, lymph node, the thymus, or other tissues or fluids. T cells can also be enriched for or purified. The T cell can be obtained by maturing hematopoietic stem cells, either in vitro or in vivo, into T cells. Preferably, the T cell is a human T cell. More preferably, the T cell is a T cell isolated from a human. The T cell can be any type of T cell and can be of any developmental stage, including but not limited to, CD4⁺/CD8⁺ double positive T cells, CD4⁺ helper T cells, e.g., Th₁ and Th₂ cells, CD8⁺ T cells (e.g., cytotoxic T cells), peripheral blood mononuclear cells (PBMCs), peripheral blood leukocytes (PBLs), tumor infiltrating cells (TILs), memory T cells, naïve T cells, and the like. Preferably, the T cell is a CD8⁺ T cell or a CD4⁺ T cell.

Also provided by the invention is a population of cells comprising at least one host cell described herein. The population of cells can be a heterogeneous population comprising the host cell comprising any of the recombinant expression vectors described, in addition to at least one other cell, e.g., a host cell (e.g., a T cell), which does not comprise any of the recombinant expression vectors, or a cell other than a T cell, e.g., a B cell, a macrophage, a neutrophil, an erythrocyte, a hepatocyte, an endothelial cell, an epithelial cells, a muscle cell, a brain cell, etc. Alternatively, the population of cells can be a substantially homogeneous population, in which the population comprises mainly of host cells (e.g., consisting essentially of) comprising the recombinant expression vector. The population also can be a clonal population of cells, in which all cells of the population are clones of a single host cell comprising a recombinant expression vector, such that all cells of the population comprise the recombinant expression vector. In one embodiment of the invention, the population of cells is a clonal population comprising host cells comprising a recombinant expression vector as described herein.

The invention further provides an antibody, or antigen binding portion thereof, which specifically binds to an epitope comprising the junction between the variable region and constant region of any of the TCRs described herein. The antibody can be any type of immunoglobulin that is known in the art. For instance, the antibody can be of any isotype, e.g., IgA, IgD, IgE, IgG, IgM, etc. The antibody can be monoclonal or polyclonal. The antibody can be a naturally-occurring antibody, e.g., an antibody isolated and/or purified from a mammal, e.g., mouse, rabbit, goat, horse, chicken, hamster, human, etc. Alternatively, the antibody can be a genetically-engineered antibody, e.g., a humanized antibody or a chimeric antibody. The antibody can be in monomeric or polymeric form. Also, the antibody can have any level of affinity or avidity for the epitope of the inventive TCR. Desirably, the antibody is specific for the epitope comprising the junction between the variable region and constant region of any of the TCRs described herein, such that there is minimal cross-reaction with other peptides or proteins (epitopes).

Methods of testing antibodies for the ability to bind to the epitope of the inventive TCR are known in the art and include any antibody-antigen binding assay, such as, for example, radioimmunoassay (RIA), ELISA, Western blot, immunoprecipitation, and competitive inhibition assays (see, e.g., Janeway et al., *infra*, and U.S. Patent Application Publication No. 2002/0197266 A1).

Suitable methods of making antibodies are known in the art. For instance, standard hybridoma methods are described in, e.g., Harlow and Lane (eds.), Antibodies: A Laboratory Manual, CSH Press (1988), and C.A. Janeway et al. (eds.), Immunobiology, 5th Ed., Garland Publishing, New York, NY (2001)). Alternatively, other methods, such as EBV-hybridoma methods (Haskard and Archer, J. Immunol. Methods, 74(2), 361-67 (1984), and Roder et al., Methods Enzymol., 121, 140-67 (1986)), and bacteriophage vector expression systems (see, e.g., Huse et al., Science, 246, 1275-81 (1989)) are known in the art. Further, methods of producing antibodies in non-human animals are described in, e.g., U.S. Patents 5,545,806, 5,569,825, and 5,714,352, and U.S. Patent Application Publication No. 2002/0197266 A1).

Phage display furthermore can be used to generate the antibody of the invention. In this regard, phage libraries encoding antigen-binding variable (V) domains of antibodies can be generated using standard molecular biology and recombinant DNA techniques (see, e.g., Sambrook et al. (eds.), Molecular Cloning. A Laboratory Manual, 3rd Edition, Cold Spring Harbor Laboratory Press, New York (2001)). Phage encoding a variable region with the desired specificity are selected for specific binding to the desired antigen, and a complete or partial antibody is reconstituted comprising the selected variable domain. Nucleic acid sequences encoding the reconstituted antibody are introduced into a suitable cell line, such as a myeloma cell used for hybridoma production, such that antibodies having the characteristics of monoclonal antibodies are secreted by the cell (see, e.g., Janeway et al., *supra,* Huse et al., *supra,* and U.S. Patent 6,265,150).

Antibodies can be produced by transgenic mice that are transgenic for specific heavy and light chain immunoglobulin genes. Such methods are known in the art and described in, for example U.S. Patents 5,545,806 and 5,569,825, and Janeway et al., *supra.*

Methods for generating humanized antibodies are well known in the art and are described in detail in, for example, Janeway et al., *supra,* U.S. Patents 5,225,539, 5,585,089 and 5,693,761, European Patent No. 0239400 B1, and United Kingdom Patent No. 2188638. Humanized antibodies can also be generated using the antibody resurfacing technology described in U.S. Patent 5,639,641 and Pedersen et al., J. Mol. Biol., 235, 959-973 (1994).

The invention also provides antigen binding portions of any of the antibodies described herein. The antigen binding portion can be any portion that has at least one antigen binding site, such as Fab, F(ab')₂, dsFv, sFv, diabodies, and triabodies.

A single-chain variable region fragment (sFv) antibody fragment, which consists of a truncated Fab fragment comprising the variable (V) domain of an antibody heavy chain linked to a V domain of a light antibody chain via a synthetic peptide, can be generated using routine recombinant DNA technology techniques (see, e.g., Janeway et al., *supra*). Similarly, disulfide-stabilized variable region fragments (dsFv) can be prepared by recombinant DNA technology (see, e.g., Reiter et al., Protein Engineering, 7, 697-704 (1994)). Antibody fragments of the invention, however, are not limited to these exemplary types of antibody fragments.

Also, the antibody, or antigen binding portion thereof, can be modified to comprise a detectable label, such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles).

The inventive antibodies and antigen binding portions thereof are useful in detecting expression of the inventive TCR in which the epitope that is specifically bound by the antibody or antigen binding portion thereof is found. Both qualitative and quantitative analyses can be performed on cells expressing the inventive TCR using the inventive antibodies or antigen binding portions thereof. Such analyses include any type of immunoassay, including, for example, Western blots, immunofluorescence, immunostaining, immunoprecipitation, ELISA, radioimmunoassay, etc.

The inventive TCRs, polypeptides, proteins, (including functional portions and functional variants thereof), nucleic acids, recombinant expression vectors, host cells (including populations thereof), and antibodies (including antigen binding portions thereof), can be isolated and/or purified. The term "isolated" as used herein means having been removed from its natural environment. The term "purified" as used herein means having been increased in purity, wherein "purity" is a relative term, and not to be necessarily construed as absolute purity. For example, the purity can be at least about 50%, can be greater than 60%, 70% or 80%, or can be 100%.

The inventive TCRs, polypeptides, proteins (including functional portions and variants thereof), nucleic acids, recombinant expression vectors, host cells (including populations thereof), and antibodies (including antigen binding portions thereof), all of which are collectively referred to as "inventive TCR materials" hereinafter, can be formulated into a composition, such as a pharmaceutical composition. In this regard, the invention provides a pharmaceutical composition comprising any of the TCRs, polypeptides, proteins, functional portions, functional variants, nucleic acids, expression vectors, host cells (including populations thereof), and antibodies (including antigen binding portions thereof), and a pharmaceutically acceptable carrier. The inventive pharmaceutical compositions containing any of the inventive TCR materials can comprise more than one inventive TCR material, e.g., a polypeptide and a nucleic acid, or two or more different TCRs. Alternatively, the pharmaceutical composition can comprise an inventive TCR material in combination with another pharmaceutically active agents or drugs, such as a chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

Preferably, the carrier is a pharmaceutically acceptable carrier. With respect to pharmaceutical compositions, the carrier can be any of those conventionally used and is limited only by chemico-physical considerations, such as solubility and lack of reactivity with the active compound(s), and by the route of administration. The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular inventive TCR material, as well as by the particular method used to administer the inventive TCR material. Accordingly, there are a variety of suitable formulations of the pharmaceutical composition of the invention. The following formulations for oral, aerosol, parenteral, subcutaneous, intravenous, intramuscular, intraarterial, intrathecal, interperitoneal, rectal, and vaginal administration are exemplary and are in no way limiting. More than one route can be used to administer the inventive TCR materials, and in certain instances, a particular route can provide a more immediate and more effective response than another route.

Topical formulations are well-known to those of skill in the art. Such formulations are particularly suitable in the context of the invention for application to the skin.

Formulations suitable for oral administration can consist of (a) liquid solutions, such as an effective amount of the inventive TCR material dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and other pharmacologically compatible excipients. Lozenge forms can comprise the inventive TCR material in a flavor, usually sucrose and acacia or tragacanth, as well as pastilles comprising the inventive TCR material in an inert base, such as gelatin and glycerin, or sucrose and acacia, emulsions, gels, and the like containing, in addition to, such excipients as are known in the art.

The inventive TCR material, alone or in combination with other suitable components, can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer. Such spray formulations also may be used to spray mucosa.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The inventive TCR material can be administered in a physiologically acceptable diluent in a pharmaceutical carrier, such as a sterile liquid or mixture of liquids, including water, saline, aqueous dextrose and related sugar solutions, an alcohol, such as ethanol or hexadecyl alcohol, a glycol, such as propylene glycol or polyethylene glycol, dimethylsulfoxide, glycerol, ketals such as 2,2-dimethyl-1,3-dioxolane-4-methanol, ethers, poly(ethyleneglycol) 400, oils, fatty acids, fatty acid esters or glycerides, or acetylated fatty acid glycerides with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-β-aminopropionates, and 2-alkyl-imidazoline quaternary ammonium salts, and (e) mixtures thereof.

The parenteral formulations will typically contain from about 0.5% to about 25% by weight of the inventive TCR material in solution. Preservatives and buffers may be used. In order to minimize or eliminate irritation at the site of injection, such compositions may contain one or more nonionic surfactants having a hydrophile-lipophile balance (HLB) of from about 12 to about 17. The quantity of surfactant in such formulations will typically range from about 5% to about 15% by weight. Suitable surfactants include polyethylene glycol sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multi-dose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Injectable formulations are in accordance with the invention. The requirements for effective pharmaceutical carriers for injectable compositions are well-known to those of ordinary skill in the art (see, e.g., Pharmaceutics and Pharmacy Practice, J.B. Lippincott Company, Philadelphia, PA, Banker and Chalmers, eds., pages 238-250 (1982), and ASHP Handbook on Injectable Drugs, Toissel, 4th ed., pages 622-630 (1986)). Preferably, when administering cells, e.g., dendritic cells, the cells are administered via injection.

Additionally, the inventive TCR materials, or compositions comprising such inventive TCR materials, can be made into suppositories by mixing with a variety of bases, such as emulsifying bases or water-soluble bases. Formulations suitable for vaginal administration can be presented as pessaries, tampons, creams, gels, pastes, foams, or spray formulas containing, in addition to the active ingredient, such carriers as are known in the art to be appropriate.

It will be appreciated by one of skill in the art that, in addition to the above-described pharmaceutical compositions, the inventive TCR materials of the invention can be formulated as inclusion complexes, such as cyclodextrin inclusion complexes, or liposomes.

For purposes of the invention, the amount or dose of the inventive TCR material administered should be sufficient to effect, e.g., a therapeutic or prophylactic response, in the subject or animal over a reasonable time frame. For example, the dose of the inventive TCR material should be sufficient to bind to a cancer antigen, or detect, treat or prevent cancer in a period of from about 1 to 4 weeks or longer, e.g., 5 to 20 or more weeks, from the time of administration. In certain embodiments, the time period could be even longer. The dose will be determined by the efficacy of the particular inventive TCR material and the condition of the animal (e.g., human), as well as the body weight of the animal (e.g., human) to be treated.

Many assays for determining an administered dose are known in the art. For purposes of the invention, an assay, which comprises comparing the extent to which target cells are lysed or IFN-γ is secreted by T cells expressing the inventive TCR, polypeptide, or protein upon administration of a given dose of such T cells to a mammal among a set of mammals of which is each given a different dose of the T cells, could be used to determine a starting dose to be administered to a mammal. The extent to which target cells are lysed or IFN-γ is secreted upon administration of a certain dose can be assayed by methods known in the art, including, for instance, the methods described herein as Examples 2 and 5.

The dose of the inventive TCR material also will be determined by the existence, nature and extent of any adverse side effects that might accompany the administration of a particular inventive TCR material. Typically, the attending physician will decide the dosage of the inventive TCR material with which to treat each individual patient, taking into consideration a variety of factors, such as age, body weight, general health, diet, sex, inventive TCR material to be administered, route of administration, and the severity of the condition being treated. By way of example and not intending to limit the invention, the dose of the inventive TCR material can be about 0.0001 to about 1 g/kg body weight of the subject being treated/day, from about 0.0001 to about 0.001 g/kg body weight/day, or about 0.01 mg to about 1 g/kg body weight/day.

One of ordinary skill in the art will readily appreciate that the inventive TCR materials of the invention can be modified in any number of ways, such that the therapeutic or prophylactic efficacy of the inventive TCR materials is increased through the modification. For instance, the inventive TCR materials can be conjugated either directly or indirectly through a linker to a targeting moiety. The practice of conjugating compounds, e.g., inventive TCR materials, to targeting moieties is known in the art. See, for instance, Wadhwa et al., J. Drug Targeting, 3, 111-127 (1995) and U.S. Patent No. 5,087,616. The term "targeting moiety" as used herein, refers to any molecule or agent that specifically recognizes and binds to a cell-surface receptor, such that the targeting moiety directs the delivery of the inventive TCR materials to a population of cells on which surface the receptor is expressed. Targeting moieties include, but are not limited to, antibodies, or fragments thereof, peptides, hormones, growth factors, cytokines, and any other natural or non-natural ligands, which bind to cell surface receptors (e.g., Epithelial Growth Factor Receptor (EGFR), T-cell receptor (TCR), B-cell receptor (BCR), CD28, Platelet-derived Growth Factor Receptor (PDGF), nicotinic acetylcholine receptor (nAChR), etc.). The term "linker" as used herein, refers to any agent or molecule that bridges the inventive TCR materials to the targeting moiety. One of ordinary skill in the art recognizes that sites on the inventive TCR materials, which are not necessary for the function of the inventive TCR materials, are ideal sites for attaching a linker and/or a targeting moiety, provided that the linker and/or targeting moiety, once attached to the inventive TCR materials, do(es) not interfere with the function of the inventive TCR materials, i.e., the ability to bind to a cancer antigen, or to detect, treat, or prevent cancer.

Alternatively, the inventive TCR materials can be modified into a depot form, such that the manner in which the inventive TCR materials is released into the body to which it is administered is controlled with respect to time and location within the body (see, for example, U.S. Patent No. 4,450,150). Depot forms of inventive TCR materials can be, for example, an implantable composition comprising the inventive TCR materials and a porous or non-porous material, such as a polymer, wherein the inventive TCR materials is encapsulated by or diffused throughout the material and/or degradation of the non-porous material. The depot is then implanted into the desired location within the body and the inventive TCR materials are released from the implant at a predetermined rate.

It is contemplated that the inventive pharmaceutical compositions, TCRs, polypeptides, proteins, nucleic acids, recombinant expression vectors, host cells, or populations of cells can be used in methods of treating or preventing a disease in a host. Without being bound to a particular theory, the inventive TCRs are believed to have enhanced biological activity, such that the TCR (or related inventive polypeptide or protein) when expressed by a cell is able to mediate a stronger immune response against the cell expressing the antigen for which the TCR is specific. In this regard, the invention provides a method of treating or preventing a disease in a host, comprising administering to the host any of the pharmaceutical compositions in an amount effective to treat or prevent the disease in the host.

The disease can be any disease involving an antigen, e.g., an infectious disease, an autoimmune disease, a cancer.

For purposes herein, "infectious disease" means a disease that can be transmitted from person to person or from organism to organism, and is caused by a microbial agent (e.g., common cold). Infectious diseases are known in the art and include, for example, hepatitis, sexually transmitted diseases (e.g., Chlamydia, gonorrhea), tuberculosis, HIV/AIDS, diphtheria, hepatitis B, hepatitis C, cholera, and influenza.

For purposes herein, "autoimmune disease" refers to a disease in which the body produces an immunogenic (i.e., immune system) response to some constituent of its own tissue. In other words the immune system loses its ability to recognize some tissue or system within the body as "self" and targets and attacks it as if it were foreign. Autoimmune diseases can be classified into those in which predominantly one organ is affected (e.g., hemolytic anemia and anti-immune thyroiditis), and those in which the autoimmune disease process is diffused through many tissues (e.g., systemic lupus erythematosus). For example, multiple sclerosis is thought to be caused by T cells attaching the sheaths that surround the nerve fibers of the brain and spinal cord. This results in loss of coordination, weakness, and blurred vision. Autoimmune diseases are known in the art and include, for instance, Hashimoto's thyroiditis, Grave's disease, lupus, multiple sclerosis, rheumatic arthritis, hemolytic anemia, anti-immune thyroiditis, systemic lupus erythematosus, celiac disease, Crohn's disease, colitis, diabetes, scleroderma, psoriasis, and the like.

With respect to the inventive methods, the cancer can be any cancer, including any of acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vulva, chronic lymphocytic leukemia, chronic myeloid cancer, colon cancer, esophageal cancer, cervical cancer, gastrointestinal carcinoid tumor. Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, ovarian cancer, pancreatic cancer, peritoneum, omentum, and mesentery cancer, pharynx cancer, prostate cancer, rectal cancer, renal cancer (e.g., renal cell carcinoma (RCC)), small intestine cancer, soft tissue cancer, stomach cancer, testicular cancer, thyroid cancer, ureter cancer, and urinary bladder cancer. Preferably, the cancer is melanoma.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount of any level of treatment or prevention of cancer in a mammal. Furthermore, the treatment or prevention provided by the inventive method can include treatment or prevention of one or more conditions or symptoms of the disease, e.g., cancer, being treated or prevented. Also, for purposes herein, "prevention" can encompass delaying the onset of the disease, or a symptom or condition thereof.

Also provided is a method of detecting a diseased cell in a host. The method comprises (i) contacting a sample comprising cells of the host with any of the inventive TCRs, polypeptides, proteins, nucleic acids, recombinant expression vectors, host cells, and populations of host cells described herein, thereby forming a complex, and detecting the complex, wherein detection of the complex is indicative of the presence of the disease in the host.

In the method of treating or preventing a disease or of detecting a diseased cell, the inventive TCR has antigenic specificity for an antigen that is characteristic of the disease to be treated, prevented, or detected. For instance, if the disease to be treated, prevented or detected is melanoma, the inventive TCR has antigenic specificity for a melanoma antigen, e.g., MART-1, NY-ESO-1, gp100, etc. If a host cell or a population comprising at least one host cell is used in the method, the host cell desirably expresses a TCR having antigenic specificity for the antigen of the disease. If an inventive nucleic acid or recombinant expression vector is used in the method, the nucleic acid or recombinant expression vector desirably encodes the TCR which has antigenic specificity for an antigen of the disease to be treated, prevented, or detected, such that expression of the nucleic acid or recombinant expression vector is achieved in a cell and the TCR expressed by the cell is capable of binding to the antigen of the disease.

With respect to the inventive method of detecting a diseased cell in a host, the sample comprising cells of the host can be a sample comprising whole cells, lysates thereof, or a fraction of the whole cell lysates, e.g., a nuclear or cytoplasmic fraction, a whole protein fraction, or a nucleic acid fraction. If the sample comprises whole cells, the cells can be any cells of the host, e.g., the cells of any organ or tissue, including blood cells.

For purposes of the inventive detecting method, the contacting step can take place in vitro or *in vivo* with respect to the host. Preferably, the contacting is an *in vitro* step.

Also, detection of the complex can occur through any number of ways known in the art. For instance, the inventive TCRs, polypeptides, proteins, nucleic acids, recombinant expression vectors, host cells, populations of cells, or antibodies, or antigen binding portions thereof, described herein, can be labeled with a detectable label such as, for instance, a radioisotope, a fluorophore (e.g., fluorescein isothiocyanate (FITC), phycoerythrin (PE)), an enzyme (e.g., alkaline phosphatase, horseradish peroxidase), and element particles (e.g., gold particles).

For purposes of the inventive methods, wherein host cells or populations of cells are administered to the host, the cells can be cells that are allogeneic or autologous to the host. Preferably, the cells are autologous to the host

The host referred to herein can be any host. Preferably, the host is a mammal. As used herein, the term "mammal" refers to any mammal, including, but not limited to, mammals of the order Rodentia, such as mice and hamsters, and mammals of the order Logomorpha, such as rabbits. It is preferred that the mammals are from the order Carnivora, including Felines (cats) and Canines (dogs). It is more preferred that the mammals are from the order Artiodactyla, including Bovines (cows) and Swines (pigs) or of the order Perssodactyla, including Equines (horses). It is most preferred that the mammals are of the order Primates, Ceboids, or Simoids (monkeys) or of the order Anthropoids (humans and apes). An especially preferred mammal is the human.

The invention further provides a method of improving the biological activity of a TCR, wherein the TCR is a TCR of a first host. The method comprises replacing, by way of, e.g., genetic engineering, the constant region of the TCR with a constant region comprising at least an extracellular domain of a constant region of a TCR of a second host.

Without being bound to any particular theory, it is contemplated that the inventive chimeric TCRs exhibit enhanced biological activity due to the preferential pairing of non-human, e.g., murine, constant regions in cells expressing the chimeric TCRs. In particular, it is contemplated that the extracellular domain of the non-human constant region confers the chimeric TCR with enhanced biological activities.

As used herein, the term "biological activity" in the context of a TCR refers to any biological activity of a TCR known in the art. For example, the biological activity of a TCR can be the recognition and binding to antigen, the secretion of a cytokine, e.g., IL-2, GM-CSF, IFN-γ, upon antigen binding, the cytolysis of a cell presenting antigen, the expression on the cell surface, the association with CD3, the activation of downstream molecules, the activation of particular genes, etc.

With respect to the inventive method of improving the biological activity of a TCR, the first host can be any host, including any of the hosts described herein. Preferably, the first host is a human. Also, the second host of the inventive method can be any host, including any of the hosts described herein. It is preferred that the second host is a mouse.

### EXAMPLES

The following examples further illustrate the invention but, of course, should not be construed as in any way limiting its scope.

The following cells are used in the examples described herein and are cultured as described below.

All of the PBMCs of this study are from metastatic melanoma patients treated at the Surgery Branch, National Cancer Institute (NCI), NIH, Bethesda, MD. Jurkat RT3-T3.5 is a radiation-induced Jurkat mutant that is surface TCR-negative (Weiss et al., J. Exp. Med., 160, 1284-1299 (1984)) (ATCC TIB-153). Melanoma cell lines: 526 (HLA-A2+), 624 (HLA-A2+), 624.38 (HLA-A2+), 888 (HLA-A2-), 938 (HLA-A2-) are generated at the Surgery Branch as previously described (Topalian et al., J. Immunol., 142, 3714-3725 (1989)). p53+/HLA-A2+ cell lines are: H2087 (ATCC/CRL-5922), MDA-MB-231 (ATCC/HTB-26) and p53⁻/HLA-A2⁺ Saos 2 (ATCC-HTB-85). T2 cells are a lymphoblastoid cell line deficient in TAP function whose HLA/A2 protein can be easily loaded with exogenous peptides (Salter et al., Immunogenetics, 21, 235-246 (1985)).

All cells are cultured in R10 media consisting of RPMI 1640 supplemented with 10% heat inactivated FBS (Biofluids, Rockville, MD) and are maintained in a 37°C and 5% CO₂ incubator. Lymphocytes are cultured in AIM-V medium (Invitrogen, Carlsbad CA) supplemented with 5% human A.B serum (Valley Biomedical, Winchester, VA) and 300 IU/mL IL-2 at 37° C and 5% CO₂.

### EXAMPLE 1

This example demonstrates the generation of chimeric TCRs comprising a human variable region and a constant region comprising at least an extracellular domain of a non-human constant region.

The α and β chains of a murine TCR (comprising both murine variable and constant regions) specific for p53₂₆₄₋₂₇₂ (Cohen et al., J. Immunol., 175, 5799-5808 (2005)) are sub-cloned into the pGEM-4Z/64A vector as previously described (Zhao et al., Blood, 102, 4137-4142 (2003), Zhao et al., Mol. Ther.,12, 247-253 (2005)). This fully murine anti-p53 TCR is termed herein as the p53 MM TCR. The α and β chains of a human TCR (comprising both human variable and constant regions) specific for MART-1/27L (Hughes et al., Hum. Gene Ther., 16, 1-16 (2005)) are sub-cloned into the pGEM-4Z/64A vector in the same manner as for the α and β chains of the p53 MM TCR.

Chimeric TCRs in which the original constant regions are replaced by either mouse or human constant region sequences are designed to generate: (1) a murine p53-specific TCR with human constant regions (p53-MH), (2) a human MART-1-specific TCR with murine β1 constant regions (MART-HM), (3) a human NY-ESO-1-specific TCR with murine constant regions (NY2 HM), (4) a human gp100-specific TCR with murine constant regions (gp100 HM), (5) a second human MART-1-specific TCR with murine β1 constant regions (MART HM_{F5}; which has variable regions that are different from those of MART HM), and (6) a human MART-1-specific TCR with murine β2 constant regions (MART HM_{F4B2}; which is the same TCR as MART HM, except that the murine constant regions are from the murine β2 chain, as opposed to the murine β1 chain).

Specifically, the chimeric TCRs are constructed by swapping the constant regions of the fully human or fully murine TCRs with either human or murine constant regions using a mega-primer based approach (Sarkar et al., Biotechniques, 8, 404-407 (1990)).

To humanize the p53 MM TCR, the murine constant regions are replaced with the human constant regions of the MART HH TCR. The humanized version of p53 MM is called herein as the p53 MH. The primers used to amplify the p53 variable α domain are p53vA-RNAF and p53vA-RNAR to generate the p53vA megaprimer. The primers used to amplify the p53 variable β domain are p53vB-RNAF and p53vB-RNAR to generate the p53vB megaprimer. The mega-primers are then used to fuse the human constant regions of the MART HH TCR to the p53 MM TCR variable regions. p53vA-RNAF and p53HcA-RNAR are used to generate the p53-MH α chain, and p53vB-RNAF and p53HcB-RNAR are used to generate the p53-MH β chain. Each of the p53-MH α and p53-MH β chains are digested with *XBaI* and *NotI* and cloned into a pGEM-4Z/64A vector. The nucleotide sequences of the primers used to construct the p53 MH TCR are shown in Table 1.

**TABLE 1**

| **Primer name** | **Nucleotide sequence** | **SEQ ID NO.** |
|---|---|---|
| p53vA-RNAF | ATCTAGAGCCGCCATGGCTCCTGGCGCTCCTCCCAG | 36 |
| p53vA-RNAR | | 37 |
| p53vB-RNAF | ATCTAGAGCCGCCATGGCTACAAGGCTCCTCTGTTAC | 38 |
| p53vB-RNAR | | 39 |
| p53HcA-RNAR | CTAGGCGGCCGCTCAGCTGGACCACAGCCGCAG | 40 |
| p53HcB-RNAR | | 41 |

Similarly, to "murinize" the human MART HH TCR, the variable α chain is first amplified with the MARTvA-RNAF and MARTvA-RNAR primers to generate the MARTvA megaprimer. The variable β chain is amplified with the MARTvB-RNAF and MARTvB-RNAR primers to generate the MART-vB megaprimer. The megaprimers are then subjected to a second PCR reaction using either the MARTvA-RNAF and MART-McA-RNAR primers to generate the MART-HMα chain or the MARTvB-RNAF and MART-McB-RNAR primers to generate the MART-HMβ chain. The MART-HMα product is digested with *XbaI* and *NotI*, while the MART-HMβ product is digested with *HindIII* and *NotI.* Each of the digestion products is subsequently cloned into a pGEM-4Z/64A vector. The murinized version of MART HH TCR is called herein as MART HM. The sequences of the primers used to construct MART HM are listed in Table 2.

**TABLE 2**

| **Primer name** | **Nucleotide sequence** | **SEQ ID NO.** |
|---|---|---|
| MARTvA -RNAF | | 42 |
| MARTvA -RNAR | | 43 |
| MARTvB -RNAF | | 44 |
| MARTvB -RNAR | | 45 |
| MART-McA-RNAR | CCGCGGCCGCTCAACTGGACCACAGCCTCAGCG | 46 |
| MART-McB-RNAR | CCGCGGCCGCTCATGAATTCTTTCTTTTGACCATAGC | 47 |

A mouse chimeric ("murinized") version of a human HLA-DP4-restricted NY-ESO-1-specific TCR (NY2 HH) (Zhao et al., J. Immunother., In press) is also constructed. The murinized version is called herein NY2 HM. Briefly, the human variable α chain of NY2 HH is amplified using the T7-ESO-II-a and AV9-2-mca-r primers and then linked to a mouse α constant region via PCR using the T7-ESO-II-a and mCA-r1 primers. The variable β chain of NY2 HH is amplified with the T7-ESO-II-b and BV20-1-mcb-r primers and linked to the mouse β constant region via PCR using the primers T7-ESO-II-b and mCB-r1. The nucleotide sequences of the primers used in the preparation of the NY2 MH TCR are shown in Table 3.

**TABLE 3**

| **Primer name** | **Nucleotide sequence** | **SEQ ID NO.** |
|---|---|---|
| T7-ESO-II-a | | 48 |
| AV9-2-mca-r | | 49 |
| mCA-r1 | AATGCGGCCGCTCAACTGGACCACAGCCTCAG | 50 |
| T7-ESO-II-b | | 51 |
| BV20-1-mcb-r | | 52 |
| mCB-r1 | AATGCGGCCGCTCATGAATTCTTTCTTTTGACCATAG | 53 |

The same approach used for constructing NY2 HM is used to construct a murinized verion of a human gp100-specific TCR (gp100 HM), and the approach used to construct MART HM is used to construct two additional murinized MART-1 specific TCRs, in which, for one of the additional MART-1 specific TCR, the murine constant region of MART HM was replaced with the murine constant regions of the murine β2 chain to generate the MART IIM_{F4B2}, while, for the second additional MART-1 specific TCR, the variable regions of MART HM were replaced with different MART-1 specific variable regions to generate the MART HM_{F5} TCR.

Briefly, the human variable α chain of MART HM_{F5} is amplified using the F5-9f primer and F5-10r primers and then linked to generate a MART HM_{F5} vA megaprimer, while the human variable β chain is amplified using the F5-13F and F5-14R primers and then linked to construct the MART HM_{F5} vB megaprimer. Each megaprimer is subjected to a second PCR reaction using the F5-9f and Mca-r1 primers (for the α chain) and the F5-13F and Mcb-rl primers (for the β chain). Each of the alpha and beta chains of the MART HM_{F5} TCR is cloned into a pGEM vector.

The human variable α chain of GP100 HM is amplified using the T7-gp100a and gp100 mcaR primers and then linked to a mouse α constant region via PCR using the T7-gp100a and 64A-Malpha r primers. The human variable β chain of GP100 HM is amplified using the T7-GP100b and gp100-mcBR primers and then linked to a mouse β constant region via PCR using the T7-GP1 00b and 64A-Mbeta r primers. The PCR products of each chain is then in vitro transcribed. The sequences of the primers used to construct these murinized TCRs are shown in Tables 3 and 4.

**TABLE 4**

| **Primer name** | **Nucleotide sequence** | **SEQ ID NO.** |
|---|---|---|
| F5-9f | | 54 |
| F5-10r | | 55 |
| F5-13F | | 56 |
| F5-14R | | 57 |
| T7-gp100a | | 58 |
| gp100-mcaR | | 59 |
| T7-gp100b | | 60 |
| gp100-mcBR | | 61 |
| 64A-Malpha r | | 62 |
| 64A-Mbeta r | | 63 |

*In-vitro* transcribed mRNA for both α and β TCR chains is generated from the pGEM-4Z/64A vectors containing the chimeric TCR genes using mMESSAGE mMACHINE (Ambion, Austin, Texas) and purified using QIAgen RNAeasy mini kit® (Qiagen, Valencia, California).

PBLs are electroporated as described previously (Zhao et al., 2005, *supra*). Briefly, PBLs are collected by leukopheresis and the lymphocytes are separated from the leukopheresed cells by centrifugation on a Ficoll/Hypaque cushion. The lymphocytes are washed in HBSS and resuspended in AIM-V supplemented with 5% human serum, 50 ng/ml OKT3, 300 IU/ml IL-2 at a concentration of 1 x 10⁶ cells/ml. The lymphocytes are then plated at 1×10⁶ cells/ml in 24-well plates (Costar, Cambridge, MA) and cultured for at least 1 week with the addition of new medium (without OKT3) as need to maintain a cell density of 1×10⁶ cells/ml.

The lymphocytes are washed in OPTI-MEM (Invitrogen, Carlsbad CA) and resuspended at 2.5 x 10⁷ cells/ml. Cells are transferred in 2 mm cuvettes chilled on ice and then electroporated at 500V/500 µs using an ElectroSquare Porator ECM 830 (BTX, San Diego, CA). The amount of *in-vitro* transcribed mRNA for each chain is 2 µg per 10⁶ PBMCs unless indicated otherwise. Wherever needed, the amount of electroporated mRNA is normalized using non-specific mRNA. Following electroporation, cells are transferred to 6-well plates containing fresh medium and cultured at 37°C.

Twenty-four hours after electroporation, the cells electroporated with p53 MM or p53 MH mRNA are stained with APC-labeled p53₂₆₄₋₂₇₂/HLA-A2 Pro5 pentamer (ProImmune, Oxford, UK) and the cells electroporated with MART HH or MART HM mRNA are stained with APC-labeled MART-1/27L tetramer (Beckman Coulter, San Jose, CA). Cells are stained in a FACS buffer made of PBS (Bio Whitaker, Walkersville, MD), 0.5% BSA, and 0.02% sodium azide. Immunofluorescence, analyzed as the relative log fluorescence of live cells (1×10⁵), is measured using a FACSCalibur flow cytometer (Becton Dickinson, Franklin Lakes, New Jersey).

As seen in Figure 2, the p53-MM TCR is expressed at a higher level (Mean Fluorescence Intensity - MFI=481) on the surface of most of the electroporated human lymphocytes (93.2 %), while only 63.1 % are positive for the p53 MH chimeric TCR, and expression is lower (MFI=116) (Figure 2A and 2B respectively).

Similarly, the cells that express the MART-HM TCR stain a higher proportion (72.5 %) and have a greater MFI (88.5) than the lymphocytes that express the MART-HH TCR (30.1 %; MFI = 44.8) (Figure 2C and 2D respectively). The duration of cell surface staining for the MART HM TCR is also longer than the MART HH TCR (Table 5). At two days post-electroporation, MART-HH electroporated cells express lower amounts of TCR (7.1%) than the MART-HM (46.8%), and the MART-HH is almost undetectable (1.3 %) on day 3 while more than 17 % of MART-HM-expressing lymphocytes are tetramer positive.

**TABLE 5**

| | **Day 1 Post electroporation** | **Day 2 post electroporation** | **Day 3 post electroporation** |
|---|---|---|---|
| **MART HH** | 30.1 % (44.8) | 7.1%(20.2) | 1.3% (17.2) |
| **MART HM** | 72.5 % (88.5) | 46.8 % (25.2) | 17.6 % (17.2) |

The percentage of positive cells, as well as the relative mean fluorescence intensity (in brackets) of the gated population, are shown at 1,2 and 3 days post-electroporation.

This example demonstrates that the chimeric TCRs comprising human variable regions and murine constant regions exhibit enhanced expression in human PBLs.

### EXAMPLE 2

This example demonstrates the enhanced biological activity of the chimeric TCRs.

Human PBLs are electroporated with the mRNAs of the TCR α and β chains of the p53 MM TCR, MART HH TCR, p53 MH TCR, or MART HM, with the mRNAs of the α chain of the p53 MM TCR and the β chain of the p53 MH (p53Mα/Hβ), with the α chain of the p53 MH TCR and the mRNAs of the β chain of the p53 MM TCR (p53Mβ/Hα), with the α chain of the MART HH TCR and the β chain of the MART HM (MART Hα/ Mβ), or with the α chain of the MART HM TCR and the β chain of the MART HH TCR (MART Hβ/ Mα), as described in Example 1. Electroporated cells are cultured and then stimulated with OKT-3.

T2 cells are pulsed with 1 µg/ml peptides specific for one of the TCRs (p53₂₆₄₋₂₇₂ or MART-1 27L ₂₆₋₃₅ peptides) or with non-specific peptides (gp100 210M ₂₀₉₋₂₁₇, gp100₂₈₀₋₂₈₈, HBVc 23Y ₁₈₋₂₇, or p53₁₄₉₋₁₅₇) in medium for 2 hrs at 37°C. The pulsed cells were washed three times with medium.

The sequences of the peptides of this assay are shown in Table 6.

**TABLE 6**

| **Peptide Name** | **Sequence** | **SEQ ID NO.** |
|---|---|---|
| p53₂₆₄₋₂₇₂ | LLGRNSFEV | 64 |
| MART-1 27L ₂₆₋₃₅ | ELAGIGILTV | 65 |
| gp100 210M₂₀₉₋₂₁₇ | IMDQVPFSV | 66 |
| gp100₂₈₀₋₂₈₈ | YLEPGPVTA | 67 |
| HBVc 23Y ₁₈₋₂₇ | FLPSDYFPSV | 68 |
| p53₁₄₉₋₁₅₇ | STPPPGTRV | 69 |
| Flu-MP₅₈₋₆₆ | GILGFVFTL | 70 |
| NY-ESO-1₁₆₁₋₁₈₀ | WITQCFLPVFLAQPPSGQRA | 71 |

Responder cells (1×10⁵ electroporated PBLs) and 1×10⁵ stimulator cells (pulsed T2 cells) are incubated in a 0.2-ml culture volume in individual wells of 96-well plates. Stimulator cells and responder cells are co-cultured for 16 to 24 h. Cytokine secretion of culture supernatants diluted to the linear range of the assay is measured using commercially available ELISA kits (IFN-γ, IL-2 and GM-CSF; Endogen, Cambridge, MA).

While all of the p53 MM, p53 MH, MART HH, and MART HM TCRs mediate antigen specific IFN-γ release, the p53-MM TCR mediate secretion of more than twice the amount of IFN-γ compared to the humanized p53-MH TCR (57,800 vs. 25,600 pg/ml). The murinized MART-HM TCR mediate an increased level of IFN-γ secretion compared to the fully human TCR, MART-HH (22,450 vs. 9,600 pg/ml for MART-1 TCRs) (Figure 3A-B). Little or no IFN-γ secretion is detected when each chain was electroporated alone or when the T2 cells are pulsed with non-specific peptides (data not shown). Correspondingly, higher levels of GM-CSF are secreted by PBLs expressing TCRs with murine constant regions (p53-MM TCR or MART HM TCR and co-cultured with peptide pulsed T2 cells (p53-MM-24,274 vs. p53-MH-12,317 pg/ml and 31,376 vs. 15,023 pg/ml for MART HM and HH respectively).

The function of different combinations of TCR chains, e.g. the humanized p53-TCR α chain (Hα) with the original full mouse p53-TCR β chain (Mβ) is also tested. Both combinations Hα/Mβ and Mα/Hβ for anti-p53 and anti-MART-1 TCRs are able to mediate antigen-specific secretion of IFN-γ in co-cultures with peptide-pulsed T2 cells. However, these concentrations are always lower than the fully human TCR combination (Hα/Hβ) or the fully murine TCR (Mα/Mβ) (Figure 3A-B). These data suggest that mouse and human constant regions can pair though it results in less biological activity.

To investigate the generality of these results to other chimeric TCRs, the activity of a human class II/HLA-DP4-restricted NY-ESO-1-specific TCR (NY2-HH) is compared to its murinized form (NY2-HM). Cells, which are enriched for CD4⁺ cells via a magnetic bead-based approach (Dynal Biotech, Brown Deer, WI, and Miltenyi Biotech, Album, CA), are electroporated with the mRNAs encoding either NY2-HH and NY2-HM. The electroporated cells are subsequently co-cultured overnight in the presence of HLA-DP4⁺ EBV-B cells pulsed with or without TCR-specific peptide epitope (NY-ESO-1₁₆₁₋₁₈₀; sequence shown in Table 6).

As shown in Figure 3C, higher levels of IFN-γ secreted by PBLs expressing NY2-HM are observed in comparison to NY2-HH (1996 pg/ml vs. 642 pg/ml respectively), while there is no significant difference in levels of IFN-γ secreted by co-cultures with non-pulsed target cells. Additionally, the TCR constant region replacement strategy also proves to be beneficial for two other class-I MHC-restricted human TCRs directed against the melanoma antigens gp100 and MART-1 (data not shown), in that cells expressing the gp100 HM TCR and the MART HM_{F5} TCRs exhibit higher levels of IFN-γ secretion as compared to the fully human counterpart TCRs.

This example demonstrates that chimeric TCRs comprising human variable regions and murine constant regions exhibit a higher biological activity.

### EXAMPLE 3

This example demonstrates the preferential pairing of mouse TCR chains with their counterparts.

Because an increased proportion of MART-1 tetramer positive cells expresses the murinized form of the anti-MART-1 TCR (MART HM), rather than the fully human version (MART HH), it is hypothesized that the mouse constant regions preferentially pair with themselves.

To test this hypothesis, a competition experiment is performed. Briefly, TCR-deficient Jurkat RT3-T3.5 cells are electroporated with 1 µg of each of the α chain mRNA and β chain mRNA of either the MART-HH TCR or MART-HM TCR, in combination with 1 µg of each of the α chain mRNA and β chain mRNA of a competitor TCR (gp100-specific TCR (Morgan et al., J. Immunol., 171, 3287-3295 (2003)), p53-MH, or one of two NY-ESO-1-specific TCRs (a kind gift from Dr. Paul Robbins, Surgery Branch, NCI). One sample is electroporated with 0.2 µg of competitor TCR mRNA. Twenty four hours after electroporation, the cells are stained with MART-1 tetramer. The percentage of MART-1 TCR expression is calculated by dividing the percentage of tetramer positive cells electroporated with competitor TCR mRNA by the percentage of tetramer positive cells without competitor TCR mRNA and then multiplying by 100.

As demonstrated in Figure 4A, the MART-HM TCR is relatively insensitive to the additional expression of competitor TCRs. In contrast, the expression of the MART-HH is significantly reduced when expressed with competitor TCRs. This competition appears to be dose-dependent, since an increase in fluorescence intensity for both MART-HH and MART-HM is observed when the amount of the p53-MH competitor is lowered by five times to 0.2 µg. Additionally, variable levels of competition is noted among the expression of different competitor TCRs, which may reflect preferential interactions of certain TCRs with the MART-1 TCR variable regions leading to different expression efficiencies (Saito et al., J. Immunol., 143, 3379-3384 (1989), Gouaillard et al., Eur. J. Immunol, 31, 3798-3805 (2001), and Li et al., Immunology, 88, 524-530 (1996)) or a "functional allelic exclusion" at the protein level (Sant'Angelo et al., Proc. Natl. Acad. Sci. USA., 98, 6824-6829 (2001)).

The electroporated cells are stained for Vβ12 surface expression with a PE-conjugated human Vβ12 antibody (Immunotech, Westbrook, ME) in the FACS buffer described in Example 1. The stained cells are subsequently analyzed by FACS as previously described in Example 1. Similar levels for both MART-1 TCRs (MART HH and MART HM) are observed (data not shown), suggesting that the decrease in tetramer staining of MART-HH is not due to a lower TCR chain expression, but more likely due to non-specific pairing with the competitor chains.

This example demonstrates a possible mechanism by which the chimeric TCRs exhibit an increased expression as compared to their counterpart receptors.

### EXAMPLE 4

This example demonstrates the increased stability of the CD3ζ/TCR complex comprising chimeric TCRs comprising human variable regions and mouse constant regions.

Due to its relatively short intracellular tail, the TCR heterodimer cannot signal by itself. Rather, the TCR recognition signal is conveyed by the CD3 complex which is bound non-covalently to the TCR α and β chains (Call et al., Cell, 111, 967-979 (2002)). The nature of the interaction between the TCR human or mouse constant regions of the chimeric TCRs and the human CD3 complex is next examined.

Jurkat RT3-T3.5 cells are electroporated with mRNAs encoding either the fully human MART-HH TCR or its mouse chimeric counterpart, MART-HM. Twenty-four hours after the electroporation, the cells are stained with MART-1 tetramer as described in Example 1. The staining revealed that the electroporated cells express the TCRs (either MART-HM or MART-HH) at similar levels. The electroporated cells are washed once with PBS and placed in lysis buffer containing 1% NP-40 or 1% Brij96, 10mM Tris-HCl (pH 7.2), 140mM NaCl, 2mM EDTA, 5mM iodoacetamide, 1mM Na₃VO₄, and complete protease inhibitor cocktail (Boehringer Mannheim) as described previously (Dittel et al, Immunity, 11, 289-298 (1999)). Brij 96 is a mild detergent that does not dissociate the TCR/CD3 complex, whereas NP-40 is known to disrupt human TCR-CD3 interactions (San Jose et al., Eur. J. Immunol., 28, 12-21 (1998), and Call et al., EMBO J., 23, 2348-2357 (2004)). Nuclear debris is removed by centrifugation and the resultant supernatants are subjected to immunoprecipitation with anti-TCR Vβ12 antibody (Immunotech - Westbrook, ME) and immunoblotting with a CD3-ζ-specific antibody (6B10.2, Santa Cruz, Santa Cruz -CA). Controls for sample loading consist of blotting for total CD3-ζ in cell lysates.

As seen in Figure 4B, both human and mouse hybrid TCRs retain their interaction with the CD3-ζ chain under mild detergent conditions (Brij96). However, when the stronger detergent NP40 is used, CD3-ζ association with TCRs comprising human constant regions is lost. In contrast, a clear CD3-ζ band is detected for the cells that are electroporated with MART-HM, demonstrating that the interaction between CD3 and the TCR comprising murine constant regions is not lost upon lysis with a strong detergent.

Further, preliminary experiments show that both murine α and β chains are needed to achieve enhanced TCR/CD3 stability, since neither combination of human α chain and murine β chain (MART Hα/Mβ) nor murine α chain and human β chain (MART Mα/Hβ) are able to mediate such an effect.

This example demonstrates that chimeric TCRs comprising human variable regions and mouse constant regions exhibit a stronger association with CD3.

### EXAMPLE 5

This example demonstrates that chimeric TCRs comprising human variable regions and mouse constant regions have an increased ability to recognize and kill tumors.

Since chimeric TCRs harboring mouse constant regions display a higher biological activity against peptide-pulsed T2 cells, the possible clinical relevance of these observations is examined by testing tumor cell recognition and killing.

PBLs expressing the MART HH or MART HM TCR are co-cultured for twenty four hours with human tumors: HLA-A2⁺ melanoma tumors (526, 624.38, and 624), HLA-A2⁺/non-melanoma tumor (Saos-2), or HLA-A2⁻ melanoma lines (938). The secretion of IFNγ or GMCSF by the PBLs is measured as described in Example 2.

As shown in Figure 5A and 5B, HLA-A2⁺ melanoma tumors (526 and 624) specifically stimulate MART HH or MART HM TCR-expressing T cells to secrete cytokines IFN-γ and GM-CSF. The cells expressing MART HM TCR are able to secrete up to 10-fold more cytokine as compared to the level of cytokine secretion of cells expressing MART HH. No significant cytokine secretion is observed in co-cultures with HLA-A2⁺/non-melanoma tumor (Saos-2) or HLA-A2⁻ melanoma lines (888).

PBLs expressing the original murine anti-p53 TCR (p53 MM) or its humanized version (p53 MH) are co-cultured for 24 hours with HLA-A2⁺/p53⁺ tumor cells (MDA-MB-231 and H2087), 888, or Saos-2 cells. The secretion of IFNγ or GMCSF by the PBLs is measured as described in Example 2.

As shown in Figure 5 C-D, the level of both IFN-γ and GM-CSF secreted by the humanized-TCR (p53 MH) expressing T-cells is decreased as compared to the full-mouse TCR (p53 MM). Little to no significant cytokine secretion is observed by control co-cultures, which consisted of the p53 TCR expressing cells and either HLA-A2- cells or p53⁻cells.

Additionally, cell-mediated cytotoxicity of human PBLs expressing either the MART-HH or its MART-HM hybrid TCR is compared in a 3-hour ⁵¹Cr release assay (Topalian et al., J. Immunol., 142, 3714-3725 (1989)). Briefly, CD8⁺ cells are isolated from PBL cultures by using a magnetic bead based approach (Dynal Biotech, Brown Deer, WI and Miltenyi Biotech, Auburn, CA). The CD8⁺ cells are electroporated with mRNA encoding either the fully human MART HH or murinized MART HM TCR. The electroporated cells are co-cultured with Cr⁵¹-labeled tumor cells, which are labeled by incubating tumor cells (1×10⁵) for 1 hr at 37°C with 50 µCi of ⁵¹Cr(Amersham, Arlington Heights, IL). Labeled target tumor cells (2x10³) are incubated with effector cells (CD8⁺ cells) at 15:1, 5:1, 1.5:1, or 0.5:1 effector cell:target cell (E:T) ratios for 3 h at 37°C in 0.2 ml of medium. Supernatants of the cells are harvested and counted using a Wallac 1470 Wizard automatic γ-counter (Wallac, Gaithersburg, MD). Total and spontaneous Cr⁵¹ releases are determined by incubating 2x10³ labeled targets in either 2% SDS or medium for 3 hours at 37°C respectively. Each data point is done as an average of quadruplicate wells. The percent of specific lysis is calculated as follows: % specific lysis = (specific release-spontaneous release)/(total release-spontaneous release) x 100.

As seen in Figures 6A-C, both the fully human MART HH and the murinized MART HM TCRs are able to mediate specific lysis of HLA-A2⁺ melanoma tumor lines. However, the lymphocytes expressing the murinized MART-HM TCR demonstrate a higher level of cytolysis as compared to the original human MART-HH TCR (e.g. 65.9 % vs. 26.1 % of specific lysis for the 526 target cell-line, at 15:1 E:T ratio, respectively - Figure 6A). No significant lysis is observed by mock-electroporated PBLs or by PBLs co-cultured with HLA-A2⁻ or non-melanoma tumors (Figures 6D and E).

This example demonstrates that the chimeric TCRs comprising human variable regions and mouse constant regions exhibit a higher ability to recognize and kill tumor cells.

### EXAMPLE 6

This example demonstrates that the chimeric TCR comprising murine constant regions can mediate MHC class I-restricted tumor recognition by CD4⁺ cells.

While a high-affinity TCR may be less dependent on the participation of a co-receptor (Sherman et al., Science, 258, 815-818 (1992)), ordinary class I-MHC restricted receptors require CD8 molecules to stabilize binding (van der Merwe et al., Annu. Rev. Immunol., 21, 659-684 (2003), and Wooldridge et al., J. Biol. Chem., 280, 27491-27501 (2005)). Since the avidity of a T-cell is dictated by a combination of the affinity of its TCR for a defined MHC/peptide complex and the number of TCR molecules expressed on the surface (McKee et al., J. Transl. Med., 3, 35 (2005)), it might be possible to overcome the need for a co-receptor by augmenting the density of the transferred TCR. As the murinized MART-HM TCR is expressed at a higher density on the cell surface (Figures 2C and 2D), it is postulated that this chimeric TCR might be biologically active in CD4⁺ cells.

CD4⁺ cells are purified (over 95 % purity) from PBL cultures as described in Example 2. The CD4⁺ cells are stimulated with OKT3 and electroporated with vectors encoding the original fully human MART-1 TCR (MART-HH) or the murinized MART-1 TCR (MART-1HM), as described in Example 1. The electroporated cells are co-cultured without (NT) or with an HLA-A2⁺ melanoma tumor line (526 and 624) or with an HLA-A2⁻⁼melanoma tumor line (938). Cytokine secretion of the electroporated cells is determined as described in Example 2.

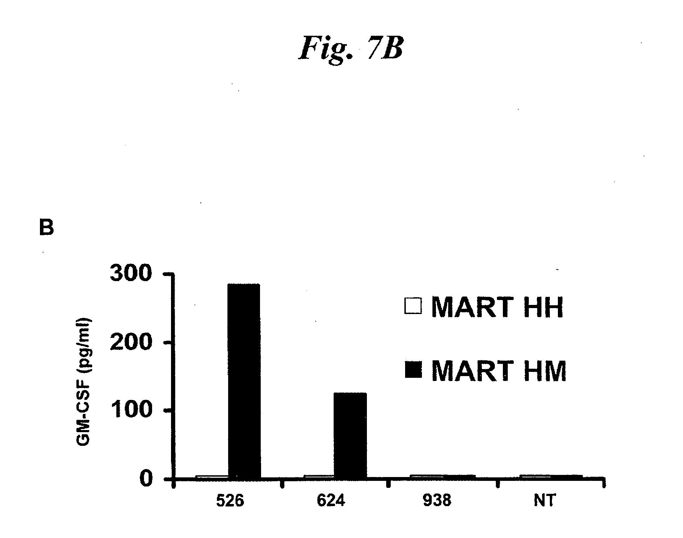

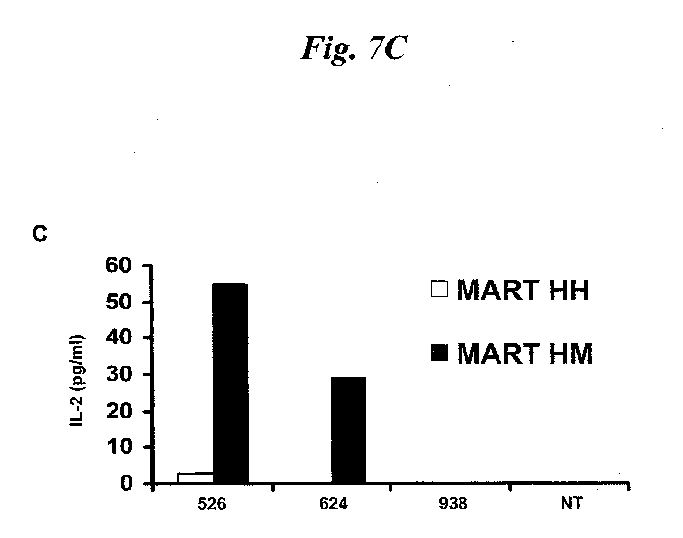

As shown in Figure 7A, CD4⁺ effector cells expressing MART-HM were able to achieve a higher level of IFN-γ secretion as compared to CD4⁺ effector cells expressing MART-HH. Also, GM-CSF and IL-2 secretions are only detected by cells expressing MART-HM-electroporated PBLs (Figures 7B and 7C). Significant cytokine secretion by cells electroporated with control mRNA (GFP) or by cells not exposed to targets is not detected.

### EXAMPLE 7

This example demonstrates the generation and testing of functional variant chimeric TCRs comprising a human variable region and a chimeric mouse/human constant region.

The alpha and beta chains of functional variant chimeric TCRs shown in Table 7 are constructed using over-lapping PCR techniques. Briefly, a forward primer that matches the 5' of the variable region and a reverse primer that contains the mutation in the constant region are used. The amplified product of this reaction is then used in a second PCR reaction as part of the template DNAs with the same forward primer and a reverse primer at the 3' of the constant region. The full length TCR is then cloned and sequenced to verify the mutation.

**TABLE 7**

| **Name** | **SEQ ID NO:** | **TCR chain** | **Description of Variable Region** | **Description of Constant Region** |
|---|---|---|---|---|
| F4-Mut31 alpha | 112 | alpha | same as variable of MART-HM (human MART-1 specific variable) | aa 129-1549 of SEQ ID NO: 112 derived from human alpha constant region + aa 150-264 of SEQ ID NO: 112 derived from mouse alpha constant region |
| F4-Mut74 alpha | 113 | alpha | same as variable of MART-HM | aa 129-199 of SEQ ID NO: 113 derived from human alpha constant region + aa 200-264 of SEQ ID NO: 113 derived from mouse alpha constant region |
| F4-AEK-Mut31a/Cpa | 114 | alpha | same as variable of MART-HM | aa 129-167 and 216-245 of SEQ ID NO: 114 derived from human alpha constant region + aa 168-215 and 246-266 of SEQ ID NO: 114 derived from mouse alpha constant region |
| F4-BFK-74a/Cpa | 115 | alpha | same as variable of MART-HM | aa 129-199 and 214-243 of SEQ ID NO: 115 derived from human alpha constant region + aa 200-213 and 244-264 of SEQ ID NO: 115 derived from mouse alpha constant region |
| F4-Mut Cp alpha | 116 | alpha | same as variable of MART-HM | aa 129-213 and 244-264 of SEQ ID NO: 116 derived from mouse alpha constant region + aa 214-243 of SEQ ID NO: 116 derived from human alpha constant region |
| F4-Mut62 beta | 117 | beta | same as variable of MART-HM | aa 133-166 of SEQ ID NO: 117 derived from human beta constant region + aa 167-305 of SEQ ID NO: 117 derived from mouse beta₁ constant region |
| F4-Mut97 beta | 118 | beta | same as variable of MART-HM | aa 133-220 of SEQ ID NO: 118 derived from human beta constant region + aa 221-309 of SEQ 10 NO: 118 derived from mouse beta₁ constant region |
| F4-CGH-Mut62b/Cpb | 119 | beta | same as variable of MART-HM | aa 133-166 and 261-304 of SEQ 10 NO: 119 derived from human beta constant region + aa 167-260 of SEQ ID NO: 119 derived from mouse beta₁ constant region |
| F4-Mut Cp beta | 120 | beta | same as variable of MART-HM | aa 133-260 of SEQ ID NO: 120 derived from mouse beta₁ constant region + aa 261-304 of SEQ ID NO: 120 derived from human beta constant region |
| 40 beta | 121 | beta | same as variable of MART-HM | aa 133-193 of SEQ ID NO: 121 derived from mouse beta₁ constant region + aa 194-308 of SEQ ID NO: 121 derived from human beta constant region |
| Cys-F4 (H/M) alpha | 122 | alpha | same as variable of MART-HM | mouse alpha constant region with aa 175 mutated to Cys |
| Cys-F4 (H/M) beta₁ | 123 | beta | same as variable of MART-HM | mouse beta₁ constant region with aa 189 mutated to Cys |
| Cys-F4 (H/M) beta₂ | 124 | beta | same as variable of MART-HM | mouse beta₂ constant region with aa 189 mutated to Cys |
| Cys-F5 (H/M) alpha | 125 | alpha | same as variable of MART HM_{F5} | mouse alpha constant region with aa 180 mutated to Cys |
| Cys-F5 (H/M) beta₁ | 126 | beta | same as variable of MART HM_{F5} | mouse beta₁ constant region with aa 188 mutated to Cys |
| Cys-F5 (H/M) beta₂ | 127 | beta | same as variable of MART HM_{F6} | mouse beta₂ constant region with aa 188 mutated to Cys |
| Cys NY-ESO-1 (H/M) alpha | 128 | alpha | same as variable of NY2 HM | mouse alpha constant region with aa 180 mutated to Cys |
| Cys NY-ESO-1 (HIM) beta₁ | 129 | beta | same as variable of NY2 HM | mouse beta₁ constant region with aa 185 mutated to Cys |
| Cys NY-ESO-1 (H/M) beta₂ | 130 | beta | same as variable of NY2 HM | mouse beta₂ constant region with aa 185 mutated to Cys |
| Cys-gp100 (H/M) alpha | 131 | alpha | same as variable of gp100 HM | mouse alpha constant region with aa 180 mutated to Cys |
| Cys-gp100 (H/M) beta₁ | 132 | beta | same as variable of gyp100 HM | mouse beta₁ constant region with aa 188 mutated to Cys |
| Cys-gp100 (H/M) beta₂ | 133 | beta | same as variable of gp100 HM | mouse beta₂ constant region with aa 188 mutated to Cys |
| Cys-IG4 (H/M) alpha | 134 | alpha | variable of human NY-ESO-1-specific TCR (1G4) | mouse alpha constant region with aa 181 mutated to Cys |
| Cys IG4 (H/M) beta₁ | 135 | beta | variable of human NY-ESO-1-specific TCR (1G4) | mouse beta₁ constant region with aa 189 mutated to Cys |
| Cys IG4 (H/M) beta₂ | 136 | beta | variable of human NY-ESO-1-specific TCR (1G4) | mouse beta₂ constant region with aa 189 mutated to Cys |

The functional variant TCR chains shown in Table 7 are paired with another TCR chain as shown in Table 8 to form TCRs. Specifically, RNA encoding either the alpha or beta chains as shown in Table 8 are electroporated into PBLs stimulated with OKT3 5-12 days prior to electroporation. PBLs nock-electroporated or electroporated with vector encoding GFP or with RNA encoding the alpha and beta chains of MART-HM or of MART-HH serve as controls. The PBLs are subsequently tested for expression by flow cytometry using a labeled-MART-1 tetramer and for cytokine secretion upon co-culture with MART-1⁺/HLA-A2⁺ cells (526, 624) or HLA-A2⁻ cells (888 or 938) by ELISA as essentially described in Example 1 and 2.

**TABLE 8**

| **Name of Alpha Chain** | **Alpha Chain SEQ ID NO:** | **Name of Beta Chain** | **Beta Chain SEQ ID NO:** |
|---|---|---|---|
| F4-Mut31 alpha | 112 | beta chain of MART-HM | 16 |
| F4-Mut74 alpha | 113 | beta chain of MART-HM | 16 |
| F4-AEK-Mut31a/Cpa | 114 | beta chain of MART-HM | 16 |
| F4-BFK-Mut74a/Cpa | 115 | beta chain of MART-HM | 16 |
| F4-Mut Cp alpha | 116 | beta chain of MART-HM | 16 |
| F4-Mut31 alpha | 112 | beta chain of MART-HH | 33 |
| F4-Mut74 alpha | 113 | beta chain of MART-HH | 33 |
| F4-AEK-Mut31a/Cpa | 114 | beta chain of MART-HH | 33 |
| F4-BFK-Mut74a/Cpa | 115 | beta chain of MART-HH | 33 |
| F4-Mut Cp alpha | 116 | beta chain of MART-HH | 33 |
| alpha chain of MART-HM | 15 | F4-Mut Cp beta | 120 |
| alpha chain of MART-HM | 15 | F4-Mut62 beta | 117 |
| alpha chain of MART-HM | 15 | F4-Mut97 beta | 118 |
| alpha chain of MART-HM | 15 | F4-CHG-Mut62b/Cpb | 119 |
| alpha chain of MART-HH | 32 | F4-Mut Cp beta | 120 |
| alpha chain of MART-HH | 32 | F4-Mut62 beta | 117 |
| alpha chain of MART-HH | 32 | F4-Mut97 beta | 118 |
| alpha chain of MART-HH | 32 | F4-CHG-Mut62b/Cpb | 119 |
| F4-Mut Cp alpha | 116 | F4-Mut Cp beta | 120 |
| alpha chain of MART-HM | 15 | 40 beta | 121 |
| alpha chain of MART-HH | 32 | 40 beta | 121 |
| F4-Mut31 alpha | 112 | 40 beta | 121 |
| F4-Mut74 alpha | 113 | 40 beta | 121 |
| F4-Mut Cp alpha | 116 | 40 beta | 121 |
| 31a/Cpa | 114 | 40 beta | 121 |
| 74a/Cpa | 115 | 40 beta | 121 |
| Cys-F5 (H/M) alpha | 125 | Cys-F5 (HIM) beta₁ | 126 |
| Cys-F5 (H/M) alpha | 125 | Cys-F5 (H/M) beta₂ | 127 |
| Cys-F4 (H/M) alpha | 122 | Cys-F4 (H/M) beta₁ | 123 |
| Cys-F4 (H/M) alpha | 122 | Cys-F4 (H/M) beta₂ | 124 |
| Cys-NY-ESO-1 (H/M) alpha | 128 | Cys-NY-ESO-1 (H/M) beta₁ | 129 |
| Cys-NY-ESO-1 (H/M) alpha | 128 | Cys-NY-ESO-1 (H/M) beta₂ | 130 |
| Cys-gp100 (H/M) alpha | 131 | Cys-gp100 (H/M) beta₁ | 132 |
| Cys-gp100 (H/M) alpha | 131 | Cys-gp100 (H/M) beta₂ | 133 |

As shown in Table 9, the electroporated cells express the indicated chimeric TCR.

**TABLE 9**

| **Name of Alpha Chain** | **Alpha Chain SEQ ID NO:** | **Name of Beta Chain** | **Beta Chain SEQ ID NO:** | **% MART-1 Tetramer-labeled cells of electroporated cells** |
|---|---|---|---|---|
| F4-Mut31 alpha | 112 | beta chain of MART-HM | 16 | 84.2 |
| F4-Mut74 alpha | 113 | beta chain of MART-HM | 16 | 76.2 |
| F4-Mut Cp alpha | 116 | beta chain of MART-HM | 16 | 79.8 |
| alpha chain of MART-HM | 15 | F4-Mut Cp beta | 120 | 81.9 |
| alpha chain of MART-HM | 15 | F4-Mut62 beta | 117 | 41.4 |
| alpha chain of MART-HM | 15 | F4-Mut97 beta | 118 | 34.7 |
| F4-Mut Cp alpha | 116 | F4-Mut Cp beta | 120 | 72.0 |

As shown in Figures 8-11, cells expressing functional variant chimeric TCRs secrete more cytokine (either IFN-gamma or GM-CSF) in response to antigen than cells expressing a wild-type human TCR or secrete a comparable amount of cytokine in response to antigen as compared to cells expressing the chimeric MART-HM TCR.

As shown in Table 10, cells expressing the Cys-F5 (H/M) alpha and beta chains secrete IFN-gamma to a greater extent than cells expressing the chimeric MART-HM_{F5}, which in turn secretes the cytokine to a greater extent than cells expressing the wild-type human MART-1 F5 TCR.

**TABLE 10**

| | **526** | **624** | **888** | **938** |
|---|---|---|---|---|
| **Mock** | 178 | 97 | 100 | 0 |
| **MART-HH_{F5}** | 3845 | 7751 | 166 | 229 |
| **MART-HM_{F5}** | 8411 | 15463 | 150 | 174 |
| **Cys-F5 (HIM)** | 9211 | 18771 | 49 | 96 |

This example demonstrates that functional variant chimeric TCRs are expressed by PBLs and react to antigen to a better extent than the fully human counterpart TCR.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A human cell comprising a chimeric T cell receptor (TCR) comprising a variable region of a human TCR and a constant region comprising at least the extracellular domain of a constant region of a non-human host TCR, or a functional variant thereof, wherein the functional variant has at least about 80% sequence identity to the chimeric TCR and specifically binds to the antigen for which the chimeric TCR has antigenic specificity.

2. A chimeric TCR comprising a variable region of a human TCR and a constant region comprising at least the extracellular domain of a constant region of a non-human host TCR, or a functional variant thereof, wherein the chimeric TCR has antigenic specificity for a cancer antigen selected from the group consisting of MART-1, gp-100, p53, and NY-ESO-1, wherein the functional variant has at least about 80% sequence identity to the chimeric TCR and specifically binds to the antigen for which the chimeric TCR has antigenic specificity.

3. The human cell of claim 1 or the chimeric TCR of claim 2, wherein the non-human host TCR is a murine TCR.

4. The human cell or chimeric TCR of claim 3, wherein the extracellular domain comprises an amino acid sequence selected from the group consisting of SEQ ID NOs: 1 to 3.

5. The human cell or chimeric TCR of claim 4, wherein the constant region comprises an amino acid sequence selected from the group consisting of SEQ ID NOs: 4 to 6.

6. The human cell or chimeric TCR of claim 3, wherein the functional variant comprises a constant region in which a first portion of the constant region is derived from a murine constant region and a second portion of the constant region is derived from a human constant region, wherein the first and the second portions each comprise at least 20 contiguous amino acids of the constant region.

7. The human cell or chimeric TCR of claim 6, wherein the functional variant comprises an amino acid sequence selected from the group consisting of SEQ ID NOs: 86-95.

8. The human cell or chimeric TCR of claim 7, wherein the functional variant comprises an amino acid sequence selected from the group consisting of SEQ ID NOs: 99-108.

9. The human cell or chimeric TCR of claim 3, wherein the functional variant comprises a constant region of a murine TCR with an amino acid substitution in which an amino acid has been substituted with Cys.

10. The human cell or chimeric TCR of claim 9, wherein the functional variant comprises an amino acid sequence selected from the group consisting of SEQ ID NOs: 96-98.

11. The human cell or chimeric TCR of claim 10, wherein the functional variant comprises an amino acid sequence selected from the group consisting of SEQ ID NOs: 109-111.

12. The human cell of any of claims 1 or 3 to 10, wherein the chimeric TCR, or a functional variant thereof, has antigenic specificity for a cancer antigen, preferably MART-1, gp-100, p53, or NY-ESO-1.

13. The human cell of claim 12 or the chimeric TCR of any one of claims 2 to 11, wherein the chimeric TCR, or functional variant thereof, comprises an amino acid sequence selected from the group consisting of SEQ ID NOs: 7 to 14, or a combination thereof.

14. The human cell or chimeric TCR of claim 13, wherein the chimeric TCR comprises an amino acid sequence selected from the group consisting of SEQ ID NOs: 15 to 26, or a combination thereof, or wherein the functional variant comprises an amino acid sequence selected from the group consisting of SEQ ID NOs: 112-133, or a combination thereof.

15. The human cell of any of claims 1 or 2 to 14, wherein the cell is a PBL, preferably a T lymphocyte.

16. A population of two or more cells, wherein at least one cell is the human cell of any of claims 1 or 2 to 15.

17. A polypeptide comprising the amino acid sequence of any of SEQ ID NOs: 15 to 26 and 86 to 133.

18. A protein comprising at least one of the polypeptides of claim 17.

19. The protein of claim 18, comprising a first polypeptide chain and a second polypeptide chain, wherein the first polypeptide chain and second polypeptide chain respectively comprise: SEQ ID NOs: 15 and 16, SEQ ID NOs: 15 and 17, SEQ ID NOs: 18 and 19, SEQ ID NOs: 18 and 20, SEQ ID NOs: 21 and 22, SEQ ID NOs: 21 and 23, SEQ ID NOs: 24 and 25, SEQ ID NOs: 24 and 26, SEQ ID NOs: 112 and 16, SEQ ID NOs: 113 and 16, SEQ ID NOs: 115 and 16, SEQ ID NOs: 116 and 16, SEQ ID NOs: 112 and 33, SEQ ID NOs: 113 and 33, SEQ ID NOs: 114 and 33, SEQ ID NOs: 115 and 33, SEQ ID NOs: 15 and 120, SEQ ID NOs: 116 and 120, SEQ ID NOs: 15 and 121, SEQ ID NOs: 32 and 121, SEQ ID NOs: 112 and 121, SEQ ID NOs: 113 and 121, SEQ ID NOs: 116 and 121, SEQ ID NOs: 114 and 121, or SEQ ID NOs: 115 and 121.

20. A nucleic acid comprising a nucleotide sequence encoding the chimeric TCR of any of claims 2 to 11 or claim 14, the polypeptide of claim 17, or the protein of claim 18 or 19.

21. The nucleic acid of claim 20, wherein the nucleotide sequence comprises a nucleotide sequence selected from the group consisting of SEQ ID NOs: 27-35 and 134 to 147.

22. A recombinant expression vector comprising the nucleic acid of claim 20 or 21.

23. A host cell comprising the recombinant expression vector of claim 22.

24. A pharmaceutical composition comprising the human cell of any of claims 1 or 2 to 15, the population of cells of claim 16, the chimeric TCR of any of claims 2 to 11 or claim 14, the polypeptide of claim 17, the protein of claim 18 or 19, the nucleic acid of claim 20 or 21, the recombinant expression vector of claim 22, the host cell of claim 23, or a combination thereof.

25. A human cell of any of claims 1 or 2 to 15, a population of cells of claim 16, a chimeric TCR of any of claims 2 to 11 or claim 14, a polypeptide of claim 17, a protein of claim 18 or 19, a nucleic acid of claim 20 or 21, a recombinant expression vector of claim 22, a host cell of claim 23, or a combination thereof for use in treating or preventing a disease in a host.

26. Use of a composition of claim 24 for the preparation of a medicament for treating or preventing a disease in a host.

27. Use of claim 26, wherein the disease is a cancer, preferably melanoma, or an infectious disease.

28. Use of claim 26, wherein the human cell is autologous to the host.

29. Use of a human cell of any of claims 1 or 2 to 15, a population of cells of claim 16, a chimeric TCR of any of claims 2 to 11 or claim 14, a polypeptide of claim 17, a protein of claim 18 or 19, a nucleic acid of claim 20 or 21, a recombinant expression vector of claim 22, a host cell of claim 23, or a combination thereof in the preparation of a composition for the detection of a diseased cell in a host.

30. Use of claim 29, wherein the diseased cell is a cancer cell, preferably melanoma, or an infected cell.

31. Use of any of claims 26 to 30, wherein the host is a human.

32. An *in vitro* method of improving the biological activity of a human TCR, comprising replacing a constant region of the human TCR with a constant region comprising at least the extracellular domain of a constant region of a non-human host TCR.

33. The method of claim 32, wherein the non-human host TCR is a murine TCR.

## Patentansprüche

1. Menschliche Zelle, umfassend einen chimären T-Zell-Rezeptor (TCR), umfassend eine variable Region eines menschlichen TCR und eine konstante Region, umfassend mindestens die extrazelluläre Domäne einer konstanten Region eines nicht menschlichen Wirts-TCR, oder eine funktionsfähige Variante davon, wobei die funktionsfähige Variante mindestens ungefähr 80% Sequenzidentität mit dem chimären TCR aufweist und spezifisch an das Antigen bindet, für welches der chimäre TCR Antigenspezifität aufweist.

2. Chimärer TCR, umfassend eine variable Region eines menschlichen TCR und eine konstante Region, umfassend mindestens die extrazelluläre Domäne einer konstanten Region eines nicht menschlichen Wirts-TCR, oder eine funktionsfähige Variante davon, wobei der chimäre TCR Antigenspezifität für ein Krebsantigen, ausgewählt aus der Gruppe, bestehend aus MART-1, gp-100, p53 und NY-ESO-1, aufweist, wobei die funktionsfähige Variante mindestens ungefähr 80% Sequenzidentität mit dem chimären TCR aufweist und spezifisch an das Antigen bindet, für welches der chimäre TCR Antigenspezifität aufweist.

3. Menschliche Zelle nach Anspruch 1 oder chimärer TCR nach Anspruch 2, wobei es sich bei dem nicht menschlichen Wirts-TCR um einen Maus-TCR handelt.

4. Menschliche Zelle oder chimärer TCR nach Anspruch 3, wobei die extrazelluläre Domäne eine Aminosäuresequenz umfasst, ausgewählt aus der Gruppe, bestehend aus SEQ ID NO: 1 bis 3.

5. Menschliche Zelle oder chimärer TCR nach Anspruch 4, wobei die konstante Region eine Aminosäuresequenz umfasst, ausgewählt aus der Gruppe, bestehend aus SEQ ID NO: 4 bis 6.

6. Menschliche Zelle oder chimärer TCR nach Anspruch 3, wobei die funktionsfähige Variante eine konstante Region umfasst, in der ein erster Teil der konstanten Region von einer konstanten Region der Maus abgeleitet ist und ein zweiter Teil der konstanten Region von einer menschlichen konstanten Region abgeleitet ist, wobei der erste und der zweite Teil jeweils mindestens 20 zusammenhängende Aminosäuren der konstanten Region umfassen.

7. Menschliche Zelle oder chimärer TCR nach Anspruch 6, wobei die funktionsfähige Variante eine Aminosäuresequenz umfasst, ausgewählt aus der Gruppe, bestehend aus SEQ ID NO: 86 - 95.

8. Menschliche Zelle oder chimärer TCR nach Anspruch 7, wobei die funktionsfähige Variante eine Aminosäuresequenz umfasst, ausgewählt aus der Gruppe, bestehend aus SEQ ID NO: 99 - 108.

9. Menschliche Zelle oder chimärer TCR nach Anspruch 3, wobei die funktionsfähige Variante eine konstante Region eines Maus-TCR mit einer Aminosäuresubstitution, bei welcher eine Aminosäure durch Cys substituiert worden ist, umfasst.

10. Menschliche Zelle oder chimärer TCR nach Anspruch 9, wobei die funktionsfähige Variante eine Aminosäuresequenz umfasst, ausgewählt aus der Gruppe, bestehend aus SEQ ID NO: 96 - 98.

11. Menschliche Zelle oder chimärer TCR nach Anspruch 10, wobei die funktionsfähige Variante eine Aminosäuresequenz umfasst, ausgewählt aus der Gruppe, bestehend aus SEQ ID NO: 109 - 111.

12. Menschliche Zelle nach einem der Ansprüche 1 oder 3 bis 10, wobei der chimäre TCR oder eine funktionsfähige Variante davon Antigenspezifität für ein Krebsantigen, vorzugsweise MART-1, gp-100, p53 oder NY-ESO-1, aufweist.

13. Menschliche Zelle nach Anspruch 12 oder chimärer TCR nach einem der Ansprüche 2 bis 11, wobei der chimäre TCR oder die funktionelle Variante davon eine Aminosäuresequenz umfasst, ausgewählt aus der Gruppe, bestehend aus SEQ ID NO: 7 bis 14 oder einer Kombination davon.

14. Menschliche Zelle oder chimärer TCR nach Anspruch 13, wobei der chimäre TCR eine Aminosäuresequenz umfasst, ausgewählt aus der Gruppe, bestehend aus SEQ ID NO: 15 bis 26 oder einer Kombination davon, oder wobei die funktionsfähige Variante eine Aminosäuresequenz umfasst, ausgewählt aus der Gruppe, bestehend aus SEQ ID NO: 112 bis 133 oder einer Kombination davon.

15. Menschliche Zelle nach einem der Ansprüche 1 oder 2 bis 14, wobei es sich bei der Zelle um einen PBL, vorzugsweise einen T-Lymphozyten, handelt.

16. Population von zwei oder mehr Zellen, wobei es sich bei mindestens einer Zelle um die menschliche Zelle nach einem der Ansprüche 1 oder 2 bis 15 handelt.

17. Polypeptid, umfassend die Aminosäuresequenz nach einer der Sequenzen SEQ ID NO: 15 bis 26 und 86 bis 133.

18. Protein, umfassend mindestens eines der Polypeptide nach Anspruch 17.

19. Protein nach Anspruch 18, umfassend eine erste Polypeptidkette und eine zweite Polypeptidkette, wobei die erste Polypeptidkette beziehungsweise die zweite Polypeptidkette umfasst: SEQ ID NO: 15 und 16, SEQ ID NO: 15 und 17, SEQ ID NO: 18 und 19, SEQ ID NO: 18 und 20, SEQ ID NO: 21 und 22, SEQ ID NO: 21 und 23, SEQ ID NO: 24 und 25, SEQ ID NO: 24 und 26, SEQ ID NO: 112 und 16, SEQ ID NO: 113 und 16, SEQ ID NO: 115 und 16, SEQ ID NO: 116 und 16, SEQ ID NO: 112 und 33, SEQ ID NO: 113 und 33, SEQ ID NO: 114 und 33, SEQ ID NO: 115 und 33, SEQ ID NO: 15 und 120, SEQ ID NO: 116 und 120, SEQ ID NO: 15 und 121, SEQ ID NO: 32 und 121, SEQ ID NO: 112 und 121, SEQ ID NO: 113 und 121, SEQ ID NO: 116 und 121, SEQ ID NO: 114 und 121 oder SEQ ID NO: 115 und 121.

20. Nucleinsäure, umfassend eine Nucleotidsequenz, die den chimären TCR nach einem der Ansprüche 2 bis 11 oder Anspruch 14, das Polypeptid nach Anspruch 17 oder das Protein nach Anspruch 18 oder 19 kodiert.

21. Nucleinsäure nach Anspruch 20, wobei die Nucleotidsequenz eine Nucleotidsequenz umfasst, ausgewählt aus der Gruppe, bestehend aus SEQ ID NO: 27 - 35 und 135 bis 147.

22. Rekombinanter Expressionsvektor, umfassend die Nucleinsäure nach Anspruch 20 oder 21.

23. Wirtszelle, umfassend den rekombinanten Expressionsvektor nach Anspruch 22.

24. Pharmazeutische Zusammensetzung, umfassend die menschliche Zelle nach einem der Ansprüche 1 oder 2 bis 15, die Zellpopulation nach Anspruch 16, den chimären TCR nach einem der Ansprüche 2 bis 11 oder Anspruch 14, das Polypeptid nach Anspruch 17, das Protein nach Anspruch 18 oder 19, die Nucleinsäure nach Anspruch 20 oder 21, den rekombinanten Expressionsvektor nach Anspruch 22, die Wirtszelle nach Anspruch 23 oder eine Kombination davon.

25. Menschliche Zelle nach einem der Ansprüche 1 oder 2 bis 15, Zellpopulation nach Anspruch 16, chimärer TCR nach einem der Ansprüche 2 bis 11 oder Anspruch 14, Polypeptid nach Anspruch 17, Protein nach Anspruch 18 oder 19, Nucleinsäure nach Anspruch 20 oder 21, rekombinanter Expressionsvektor nach Anspruch 22, Wirtszelle nach Anspruch 23 oder Kombination davon zur Verwendung beim Behandeln oder Vorbeugen einer Krankheit bei einem Wirt.

26. Verwendung einer Zusammensetzung nach Anspruch 24 zur Herstellung eines Medikaments zum Behandeln oder Vorbeugen einer Krankheit bei einem Wirt.

27. Verwendung nach Anspruch 26, wobei es sich bei der Krankheit um eine Krebserkrankung, vorzugsweise Melanom, oder eine Infektionskrankheit handelt.

28. Verwendung nach Anspruch 26, wobei die menschliche Zelle zu dem Wirt autolog ist.

29. Verwendung einer menschlichen Zelle nach einem der Ansprüche 1 oder 2 bis 15, einer Zellpopulation nach Anspruch 16, eines chimären TCR nach einem der Ansprüche 2 bis 11 oder Anspruch 14, eines Polypeptids nach Anspruch 17, eines Proteins nach Anspruch 18 oder 19, einer Nucleinsäure nach Anspruch 20 oder 21, eines rekombinanten Expressionsvektors nach Anspruch 22, einer Wirtszelle nach Anspruch 23 oder einer Kombination davon bei der Herstellung einer Zusammensetzung zum Nachweisen einer erkrankten Zelle bei einem Wirt.

30. Verwendung nach Anspruch 29, wobei es sich bei der erkrankten Zelle um eine Krebszelle, vorzugsweise Melanom, oder eine infizierte Zelle handelt.

31. Verwendung nach einem der Ansprüche 26 bis 30, wobei es sich bei dem Wirt um einen Menschen handelt.

32. *In-vitro*-Verfahren zum Verbessern der biologischen Aktivität eines menschlichen TCR, umfassend Ersetzen einer konstanten Region des menschlichen TCR mit einer konstanten Region, umfassend mindestens die extrazelluläre Domäne einer konstanten Region eines nicht menschlichen Wirts-TCR.

33. Verfahren nach Anspruch 32, wobei es sich bei dem nicht menschlichen Wirts-TCR um einen Maus-TCR handelt.

## Revendications

1. Cellule humaine comprenant un récepteur chimérique de cellules T (TCR) comprenant une région variable d'un TCR humain et une région constante comprenant au moins le domaine extracellulaire d'une région constante d'un TCR hôte non humain, ou un variant fonctionnel de celui-ci, 1 dans laquelle le variant fonctionnel a une identité de séquence au moins d'environ 80 % au TCR chimérique et se lie spécifiquement à l'antigène pour lequel le TCR chimérique a une spécificité antigénique.

2. TCR chimérique comprenant une région variable d'un TCR humain et une région constante comprenant au moins le domaine extracellulaire d'une région constante d'un TCR hôte non humain, ou un variant fonctionnel de celui-ci, le TCR chimérique ayant une spécificité antigénique pour un antigène de cancer choisi parmi le groupe constitué de MART-1, gp-100, p53 et NY-ESO-1, dans lequel le variant fonctionnel a une idenité de séquence au moins d'environ 80 % au TCR chimérique et se lie spécifiquement à l'antigène pour lequel le TCR chimérique a une spécificité antigénique.

3. Cellule humaine selon la revendication 1 ou TCR chimérique selon la revendication 2, dans lequel le TCR hôte non humain est un TCR murin.

4. Cellule humaine ou TCR chimérique selon la revendication 3, dans lequel le domaine extracellulaire comprend une séquence d'aminoacides choisie parmi le groupe constitué des SEQ ID N° 1 à 3.

5. Cellule humaine ou TCR chimérique selon la revendication 4, dans lequel la région constante comprend une séquence d'aminoacides choisie parmi le groupe constitué des SEQ ID N° 4 à 6.

6. Cellule humaine ou TCR chimérique selon la revendication 3, dans lequel le variant fonctionnel comprend une région constante dans laquelle une première portion de la région constante est dérivée d'une région constante murine et une seconde portion de la région constante est dérivée d'une région constante humaine, où les première et seconde portions comprennent chacune au moins 20 aminoacides contigus de la région constante.

7. Cellule humaine ou TCR chimérique selon la revendication 6, dans lequel le variant fonctionnel comprend une séquence d'aminoacides choisie parmi le groupe constitué des SEQ ID N° 86 à 95.

8. Cellule humaine ou TCR chimérique selon la revendication 7, dans lequel le variant fonctionnel comprend une séquence d'aminoacides choisie parmi le groupe constitué des SEQ ID N° 99 à 108.

9. Cellule humaine ou TCR chimérique selon la revendication 3, dans lequel le variant fonctionnel comprend une région constante d'un TCR murin avec une substitution d'aminoacide dans laquelle un aminoacide a été substitué par Cys.

10. Cellule humaine ou TCR chimérique selon la revendication 9, dans lequel le variant fonctionnel comprend une séquence d'aminoacides choisie parmi le groupe constitué des SEQ ID N° 96 à 98.

11. Cellule humaine ou TCR chimérique selon la revendication 10, dans lequel le variant fonctionnel comprend une séquence d'aminoacides choisie parmi le groupe constitué des SEQ ID N° 109 à 111.

12. Cellule humaine selon l'une quelconque des revendications 1 ou 3 à 10, dans laquelle le TCR chimérique, ou un variant fonctionnel de celui-ci, a une spécificité antigénique pour un antigène de cancer, de préférence MART-1, gp-100, p53 ou NY-ESO-1.

13. Cellule humaine selon la revendication 12 ou TCR chimérique selon l'une quelconque des revendications 2 à 11, le TCR chimérique, ou un variant fonctionnel de celui-ci, comprenant une séquence d'aminoacides choisie parmi le groupe constitué de SEQ ID N° 7 à 14, ou une combinaison de celles-ci.

14. Cellule humaine ou TCR chimérique selon la revendication 13, dans lequel le TCR chimérique comprend une séquence d'aminoacides choisie parmi le groupe constitué des SEQ ID N° 15 à 26, ou une combinaison de celles-ci, ou dans lequel le variant fonctionnel comprend une séquence d'aminoacides choisie parmi le groupe constitué des SEQ ID N° 112 à 133, ou une combinaison de celles-ci.

15. Cellule humaine selon l'une quelconque des revendications 1 ou 2 à 14, la cellule étant un PBL (lymphocyte du sang périphérique), de préférence un lymphocyte T.

16. Population de deux ou plus de deux cellules, dans laquelle au moins une cellule est la cellule humaine selon l'une quelconque des revendications 1 ou 2 à 15.

17. Polypeptide comprenant la séquence d'aminoacides de l'une quelconque des SEQ ID N° 15 à 26 et 86 à 133.

18. Protéine comprenant au moins un des polypeptides selon la revendication 17.

19. Protéine selon la revendication 18, comprenant une première chaîne polypeptidique et une seconde chaîne polypeptidique, dans laquelle la première chaîne polypeptidique et la seconde chaîne polypeptidique comprennent respectivement : SEQ ID N° 15 et 16, SEQ ID N° 15 et 17, SEQ ID N° 18 et 19, SEQ ID N° 18 et 20, SEQ ID N° 21 et 22, SEQ ID N° 21 et 23, SEQ ID N° 24 et 25, SEQ ID N° 24 et 26, SEQ ID N° 112 et 16, SEQ ID N° 113 et 16, SEQ ID N° 115 et 16, SEQ ID N° 116 et 16, SEQ ID N° 112 et 33, SEQ ID N° 113 et 33, SEQ ID N° 114 et 33, SEQ ID N° 115 et 33, SEQ ID N° 15 et 120, SEQ ID N° 116 et 120, SEQ ID N° 15 et 121, SEQ ID N° 32 et 121, SEQ ID N° 112 et 121, SEQ ID N° 113 et 121, SEQ ID N° 116 et 121, SEQ ID N° 114 et 121, ou SEQ ID N° 115 et 121.

20. Acide nucléique comprenant une séquence nucléotidique codant pour le TCR chimérique selon l'une quelconque des revendications 2 à 11 ou la revendication 14, le polypeptide selon la revendication 17, ou la protéine selon la revendication 18 ou 19.

21. Acide nucléique selon la revendication 20, dans lequel la séquence nucléotidique comprend une séquence nucléotidique choisie parmi le groupe constitué de SEQ ID N° 27 à 35 et 134 à 147.

22. Vecteur d'expression recombinant comprenant l'acide nucléique selon la revendication 20 ou 21.

23. Cellule hôte comprenant le vecteur d'expression recombinant selon la revendication 22.

24. Composition pharmaceutique comprenant la cellule humaine selon l'une quelconque des revendications 1 ou 2 à 15, la population de cellules selon la revendication 16, le TCR chimérique selon l'une quelconque des revendications 2 à 11 ou la revendication 14, le polypeptide selon la revendication 17, la protéine selon la revendication 18 ou 19, l'acide nucléique selon la revendication 20 ou 21, le vecteur d'expression recombinant selon la revendication 22, la cellule hôte selon la revendication 23, ou une combinaison de ceux-ci.

25. Cellule humaine selon l'une quelconque des revendications 1 ou 2 à 15, une population de cellules selon la revendication 16, un TCR chimérique selon l'une quelconque des revendications 2 à 11 ou la revendication 14, un polypeptide selon la revendication 17, une protéine selon la revendication 18 ou 19, un acide nucléique selon la revendication 20 ou 21, un vecteur d'expression recombinant selon la revendication 22, une cellule hôte selon la revendication 23, ou une combinaison de ceux-ci pour utilisation dans le traitement ou la prévention d'une maladie chez un hôte.

26. Utilisation d'une composition selon la revendication 24 pour la préparation d'un médicament destiné à traiter ou à prévenir une maladie chez un hôte.

27. Utilisation selon la revendication 26, la maladie étant un cancer, de préférence un mélanome, ou une maladie infectieuse.

28. Utilisation selon la revendication 26, la cellule humaine étant autologue pour l'hôte.

29. Utilisation d'une cellule humaine selon l'une quelconque des revendications 1 ou 2 à 15, d'une population de cellules selon la revendication 16, d'un TCR chimérique selon l'une quelconque des revendications 2 à 11 ou la revendication 14, d'un polypeptide selon la revendication 17, d'une protéine selon la revendication 18 ou 19, d'un acide nucléique selon la revendication 20 ou 21, d'un vecteur d'expression recombinant selon la revendication 22, d'une cellule hôte selon la revendication 23, ou d'une combinaison de ceux-ci pour la préparation d'une composition pour la détection d'une cellule malade chez un hôte.

30. Utilisation selon la revendication 29, la cellule malade étant une cellule cancéreuse, de préférence de mélanome, ou une cellule infectée.

31. Utilisation selon l'une quelconque des revendications 26 à 30, dans laquelle l'hôte est un humain.

32. Méthode *in vitro* pour améliorer l'activité biologique d'un TCR humain, comprenant le remplacement d'une région constante du TCR humain par une région constante comprenant au moins le domaine extracellulaire d'une région constante d'un TCR hôte non humain.

33. Méthode selon la revendication 32, dans laquelle le TCR hôte non humain est un TCR murin.


### SEQUENCE LISTING

<110> GOVERNMENT OF THE UNITED STATES OF AMERICA, REPRESENTED BY THE SECRETARY, DEPARTMENT OF HEALTH AND HUMAN SERVICES
<120> CHIMERIC T CELL RECEPTORS AND RELATED MATERIALS AND METHODS OF USE
<130> 701228
<150> 60/796,853
   <151> 2006-05-03
<160> 147
<170> PatentIn version 3.4
<210> 1
   <211> 116
   <212> PRT
   <213> Mus musculus
<400> 1
   Ile Gln Asn Pro Glu Pro Ala Val Tyr Gln Leu Lys Asp Pro Arg Ser
   1               5                   10                  15
   Gln Asp Ser Thr Leu Cys Leu Phe Thr Asp Phe Asp Ser Gln Ile Asn
               20                  25                  30
   Val Pro Lys Thr Met Glu Ser Gly Thr Phe Ile Thr Asp Lys Thr Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Ser Asp Val Pro Cys Asp Ala Thr Leu Thr Glu
                   85                  90                  95
   Lys Ser Phe Glu Thr Asp Met Asn Leu Asn Phe Gln Asn Leu Ser Val
               100                 105                 110
   Met Gly Leu Arg
           115
<210> 2
   <211> 146
   <212> PRT
   <213> Mus musculus
<400> 2
   Glu Asp Leu Arg Asn Val Thr Pro Pro Lys Val Ser Leu Phe Glu Pro
   1 5 10 15
   Ser Lys Ala Glu Ile Ala Asn Lys Gln Lys Ala Thr Leu Val Cys Leu
   20 25 30
   Ala Arg Gly Phe Phe Pro Asp His Val Glu Leu Ser Trp Trp Val Asn
   35 40 45
   Gly Lys Glu Val His Ser Gly Val Ser Thr Asp Pro Gln Ala Tyr Lys
   50 55 60
   Glu Ser Asn Tyr Ser Tyr Cys Leu Ser Ser Arg Leu Arg Val Ser Ala
   65 70 75 80
   Thr Phe Trp His Asn Pro Arg Asn His Phe Arg Cys Gln Val Gln Phe
   85 90 95
   His Gly Leu Ser Glu Glu Asp Lys Trp Pro Glu Gly Ser Pro Lys Pro
   100 105 110
   Val Thr Gln Asn Ile Ser Ala Glu Ala Trp Gly Arg Ala Asp Cys Gly
   115 120 125
   Ile Thr Ser Ala Ser Tyr Gln Gln Gly Val Leu Ser Ala Thr Ile Leu
   130 135 140
   Tyr Glu
   145
<210> 3
   <211> 146
   <212> PRT
   <213> Mus musculus
<400> 3
   Glu Asp Leu Arg Asn Val Thr Pro Pro Lys Val Ser Leu Phe Glu Pro
   1 5 10 15
   Ser Lys Ala Glu Ile Ala Asn Lys Gln Lys Ala Thr Leu Val Cys Leu
   20 25 30
   Ala Arg Gly Phe Phe Pro Asp His Val Glu Leu Ser Trp Trp Val Asn
   35 40 45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Ile Thr Ser Ala Ser Tyr His Gln Gly Val Leu Ser Ala Thr Ile Leu
       130                 135                 140
   Tyr Glu
   145
<210> 4
   <211> 136
   <212> PRT
   <213> Mus musculus
<400> 4
   Ile Gln Asn Pro Glu Pro Ala Val Tyr Gln Leu Lys Asp Pro Arg Ser
   1               5                   10                  15
   Gln Asp Ser Thr Leu Cys Leu Phe Thr Asp Phe Asp Ser Gln Ile Asn
               20                  25                  30
   Val Pro Lys Thr Met Glu Ser Gly Thr Phe Ile Thr Asp Lys Thr Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Ser Asp Val Pro Cys Asp Ala Thr Leu Thr Glu
                   85                  90                  95
   Lys Ser Phe Glu Thr Asp Met Asn Leu Asn Phe Gln Asn Leu Ser Val
               100                 105                 110
   Met Gly Leu Arg Ile Leu Leu Leu Lys Val Ala Gly Phe Asn Leu Leu
           115                 120                 125
   Met Thr Leu Arg Leu Trp Ser Ser
       130                 135
<210> 5
   <211> 173
   <212> PRT
   <213> Mus musculus
<400> 5
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Ile Thr Ser Ala Ser Tyr Gln Gln Gly Val Leu Ser Ala Thr Ile Leu
       130                 135                 140
   Tyr Glu Ile Leu Leu Gly Lys Ala Thr Leu Tyr Ala Val Leu Val Ser
   145                 150                 155                 160
   Thr Leu Val Val Met Ala Met Val Lys Arg Lys Asn Ser
                   165                 170
<210> 6
   <211> 173
   <212> PRT
   <213> Mus musculus
<400> 6
   Glu Asp Leu Arg Asn Val Thr Pro Pro Lys Val Ser Leu Phe Glu Pro
   1 5 10 15
   Ser Lys Ala Glu Ile Ala Asn Lys Gln Lys Ala Thr Leu Val Cys Leu
   20 25 30
   Ala Arg Gly Phe Phe Pro Asp His Val Glu Leu Ser Trp Trp Val Asn
   35 40 45
   Gly Lys Glu Val His Ser Gly Val Ser Thr Asp Pro Gln Ala Tyr Lys
   50 55 60
   Glu Ser Asn Tyr Ser Tyr Cys Leu Ser Ser Arg Leu Arg Val Ser Ala
   65 70 75 80
   Thr Phe Trp His Asn Pro Arg Asn His Phe Arg Cys Gln Val Gln Phe
   85 90 95
   His Gly Leu Ser Glu Glu Asp Lys Trp Pro Glu Gly Ser Pro Lys Pro
   100 105 110
   Val Thr Gln Asn Ile Ser Ala Glu Ala Trp Gly Arg Ala Asp Cys Gly
   115 120 125
   Ile Thr Ser Ala Ser Tyr His Gln Gly Val Leu Ser Ala Thr Ile Leu
   130 135 140
   Tyr Glu Ile Leu Leu Gly Lys Ala Thr Leu Tyr Ala Val Leu Val Ser
   145 150 155 160
   Gly Leu Val Leu Met Ala Met Val Lys Lys Lys Asn Ser
   165 170
<210> 7
   <211> 128
   <212> PRT
   <213> Homo sapiens
<400> 7
   Met Leu Leu Glu His Leu Leu Ile Ile Leu Trp Met Gln Leu Thr Trp
   1 5 10 15
   Val Ser Gly Gln Gln Leu Asn Gln Ser Pro Gln Ser Met Phe Ile Gln
   20 25 30
   Glu Gly Glu Asp Val Ser Met Asn Cys Thr Ser Ser Ser Ile Phe Asn
   35 40 45
   Thr Trp Leu Trp Tyr Lys Gln Asp Pro Gly Glu Gly Pro Val Leu Leu
   50 55 60
   Ile Ala Leu Tyr Lys Ala Gly Glu Leu Thr Ser Asn Gly Arg Leu Thr
   65 70 75 80
   Ala Gln Phe Gly Ile Thr Arg Lys Asp Ser Phe Leu Asn Ile Ser Ala
   85 90 95
   Ser Ile Pro Ser Asp Val Gly Ile Tyr Phe Cys Ala Gly Gly Thr Gly
   100 105 110
   Asn Gln Phe Tyr Phe Gly Thr Gly Thr Ser Leu Thr Val Ile Pro Asn
   115 120 125
<210> 8
   <211> 132
   <212> PRT
   <213> Homo sapiens
<400> 8
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu
       130
<210> 9
   <211> 133
   <212> PRT
   <213> Homo sapiens
<400> 9
   Met Met Lys Ser Leu Arg Val Leu Leu Val Ile Leu Trp Leu Gln Leu
   1               5                   10                  15
   Ser Trp Val Trp Ser Gln Gln Lys Glu Val Glu Gln Asn Ser Gly Pro
               20                  25                  30
   Leu Ser Val Pro Glu Gly Ala Ile Ala Ser Leu Asn Cys Thr Tyr Ser
           35                  40                  45
   Asp Arg Gly Ser Gln Ser Phe Phe Trp Tyr Arg Gln Tyr Ser Gly Lys
       50                  55                  60
   Ser Pro Glu Leu Ile Met Phe Ile Tyr Ser Asn Gly Asp Lys Glu Asp
   65                  70                  75                  80
   Gly Arg Phe Thr Ala Gln Leu Asn Lys Ala Ser Gln Tyr Val Ser Leu
                   85                  90                  95
   Leu Ile Arg Asp Ser Gln Pro Ser Asp Ser Ala Thr Tyr Leu Cys Ala
               100                 105                 110
   Val Asn Phe Gly Gly Gly Lys Leu Ile Phe Gly Gln Gly Thr Glu Leu
           115                 120                 125
   Ser Val Lys Pro Asn
       130
<210> 10
   <211> 131
   <212> PRT
   <213> Homo sapiens
<400> 10
   Met Arg Ile Arg Leu Leu Cys Cys Val Ala Phe Ser Leu Leu Trp Ala
   1               5                   10                  15
   Gly Pro Val Ile Ala Gly Ile Thr Gln Ala Pro Thr Ser Gln Ile Leu
               20                  25                  30
   Ala Ala Gly Arg Arg Met Thr Leu Arg Cys Thr Gln Asp Met Arg His
           35                  40                  45
   Asn Ala Met Tyr Trp Tyr Arg Gln Asp Leu Gly Leu Gly Leu Arg Leu
       50                  55                  60
   Ile His Tyr Ser Asn Thr Ala Gly Thr Thr Gly Lys Gly Glu Val Pro
   65                  70                  75                  80
   Asp Gly Tyr Ser Val Ser Arg Ala Asn Thr Asp Asp Phe Pro Leu Thr
                   85                  90                  95
   Leu Ala Ser Ala Val Pro Ser Gln Thr Ser Val Tyr Phe Cys Ala Ser
               100                 105                 110
   Ser Leu Ser Phe Gly Thr Glu Ala Phe Phe Gly Gln Gly Thr Arg Leu
           115                 120                 125
   Thr Val Val
       130
<210> 11
   <211> 133
   <212> PRT
   <213> Homo sapiens
<400> 11
   Met Asn Tyr Ser Pro Gly Leu Val Ser Leu Ile Leu Leu Leu Leu Gly
   1               5                   10                  15
   Arg Thr Arg Gly Asn Ser Val Thr Gln Met Glu Gly Pro Val Thr Leu
               20                  25                  30
   Ser Glu Glu Ala Phe Leu Thr Ile Asn Cys Thr Tyr Thr Ala Thr Gly
           35                  40                  45
   Tyr Pro Ser Leu Phe Trp Tyr Val Gln Tyr Pro Gly Glu Gly Leu Gln
       50                  55                  60
   Leu Leu Leu Lys Ala Thr Lys Ala Asp Asp Lys Gly Ser Asn Lys Gly
   65                  70                  75                  80
   Phe Glu Ala Thr Tyr Arg Lys Glu Thr Thr Ser Phe His Leu Glu Lys
                   85                  90                  95
   Gly Ser Val Gln Val Ser Asp Ser Ala Val Tyr Phe Cys Ala Leu Ser
               100                 105                 110
   Ala Asn Gln Ala Gly Thr Ala Leu Ile Phe Gly Lys Gly Thr Thr Leu
           115                 120                 125
   Ser Val Ser Ser Asn
       130
<210> 12
   <211> 128
   <212> PRT
   <213> Homo sapiens
<400> 12
   Met Leu Leu Leu Leu Leu Leu Leu Gly Pro Gly Ser Gly Leu Gly Ala
   1               5                   10                  15
   Val Val Ser Gln His Pro Ser Arg Val Ile Cys Lys Ser Gly Thr Ser
               20                  25                  30
   Val Lys Ile Glu Cys Arg Ser Leu Asp Phe Gln Ala Thr Thr Met Phe
           35                  40                  45
   Trp Tyr Arg Gln Phe Pro Lys Gln Ser Leu Met Leu Met Ala Thr Ser
       50                  55                  60
   Asn Glu Gly Ser Lys Ala Thr Tyr Glu Gln Gly Val Glu Lys Asp Lys
   65                  70                  75                  80
   Phe Leu Ile Asn His Ala Ser Leu Thr Leu Ser Thr Leu Thr Val Thr
                   85                  90                  95
   Ser Ala His Pro Glu Asp Ser Ser Phe Tyr Ile Cys Ser Ala Ser Val
               100                 105                 110
   Ala Thr Glu Thr Gln Tyr Phe Gly Pro Gly Thr Arg Leu Leu Val Leu
           115                 120                 125
<210> 13
   <211> 133
   <212> PRT
   <213> Homo sapiens
<400> 13
   Met Val Lys Ile Arg Gln Phe Leu Leu Ala Ile Leu Trp Leu Gln Leu
   1               5                   10                  15
   Ser Cys Val Ser Ala Ala Lys Asn Glu Val Glu Gln Ser Pro Gln Asn
               20                  25                  30
   Leu Thr Ala Gln Glu Gly Glu Phe Ile Thr Ile Asn Cys Ser Tyr Ser
           35                  40                  45
   Val Gly Ile Ser Ala Leu His Trp Leu Gln Gln His Pro Gly Gly Gly
       50                  55                  60
   Ile Val Ser Leu Phe Met Leu Ser Ser Gly Lys Lys Lys His Gly Arg
   65                  70                  75                  80
   Leu Ile Ala Thr Ile Asn Ile Gln Glu Lys His Ser Ser Leu His Ile
                   85                  90                  95
   Thr Ala Ser His Pro Arg Asp Ser Ala Val Tyr Ile Cys Ala Ala Ser
               100                 105                 110
   Leu Ile Gln Gly Ala Gln Lys Leu Val Phe Gly Gln Gly Thr Arg Leu
           115                 120                 125
   Thr Ile Asn Pro Asn
       130
<210> 14
   <211> 131
   <212> PRT
   <213> Homo sapiens
<400> 14
   Met Asp Ser Trp Thr Phe Cys Cys Val Ser Leu Cys Ile Leu Val Ala
   1               5                   10                  15
   Lys His Thr Asp Ala Gly Val Ile Gln Ser Pro Arg His Glu Val Thr
               20                  25                  30
   Glu Met Gly Gln Glu Val Thr Leu Arg Cys Lys Pro Ile Ser Gly His
           35                  40                  45
   Asn Ser Leu Phe Trp Tyr Arg Gln Thr Met Met Arg Gly Leu Glu Leu
       50                  55                  60
   Leu Ile Tyr Phe Asn Asn Asn Val Pro Ile Asp Asp Ser Gly Met Pro
   65                  70                  75                  80
   Glu Asp Arg Phe Ser Ala Lys Met Pro Asn Ala Ser Phe Ser Thr Leu
                   85                  90                  95
   Lys Ile Gln Pro Ser Glu Pro Arg Asp Ser Ala Val Tyr Phe Cys Ala
               100                 105                 110
   Ser Ser Pro Gly Gly Asn Glu Gln Phe Phe Gly Pro Gly Thr Arg Leu
           115                 120                 125
   Thr Val Leu
       130
<210> 15
   <211> 264
   <212> PRT
   <213> Artificial
<220>
   <223> Synthetic
<400> 15
   Met Leu Leu Glu His Leu Leu Ile Ile Leu Trp Met Gln Leu Thr Trp
   1               5                   10                  15
   Val Ser Gly Gln Gln Leu Asn Gln Ser Pro Gln Ser Met Phe Ile Gln
               20                  25                  30
   Glu Gly Glu Asp Val Ser Met Asn Cys Thr Ser Ser Ser Ile Phe Asn
           35                  40                  45
   Thr Trp Leu Trp Tyr Lys Gln Asp Pro Gly Glu Gly Pro Val Leu Leu
       50                  55                  60
   Ile Ala Leu Tyr Lys Ala Gly Glu Leu Thr Ser Asn Gly Arg Leu Thr
   65                  70                  75                  80
   Ala Gln Phe Gly Ile Thr Arg Lys Asp Ser Phe Leu Asn Ile Ser Ala
                   85                  90                  95
   Ser Ile Pro Ser Asp Val Gly Ile Tyr Phe Cys Ala Gly Gly Thr Gly
               100                 105                 110
   Asn Gln Phe Tyr Phe Gly Thr Gly Thr Ser Leu Thr Val Ile Pro Asn
           115                 120                 125
   Ile Gln Asn Pro Glu Pro Ala Val Tyr Gln Leu Lys Asp Pro Arg Ser
       130                 135                 140
   Gln Asp Ser Thr Leu Cys Leu Phe Thr Asp Phe Asp Ser Gln Ile Asn
   145                 150                 155                 160
   Val Pro Lys Thr Met Glu Ser Gly Thr Phe Ile Thr Asp Lys Thr Val
                   165                 170                 175
   Leu Asp Met Lys Ala Met Asp Ser Lys Ser Asn Gly Ala Ile Ala Trp
               180                 185                 190
   Ser Asn Gln Thr Ser Phe Thr Cys Gln Asp Ile Phe Lys Glu Thr Asn
           195                 200                 205
   Ala Thr Tyr Pro Ser Ser Asp Val Pro Cys Asp Ala Thr Leu Thr Glu
       210                 215                 220
   Lys Ser Phe Glu Thr Asp Met Asn Leu Asn Phe Gln Asn Leu Ser Val
   225                 230                 235                 240
   Met Gly Leu Arg Ile Leu Leu Leu Lys Val Ala Gly Phe Asn Leu Leu
                   245                 250                 255
   Met Thr Leu Arg Leu Trp Ser Ser
               260
<210> 16
   <211> 305
   <212> PRT
   <213> Artificial
<220>
   <223> Synthetic
<400> 16
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
           50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Arg Asn Val Thr Pro Pro Lys Val Ser
       130                 135                 140
   Leu Phe Glu Pro Ser Lys Ala Glu Ile Ala Asn Lys Gln Lys Ala Thr
   145                 150                 155                 160
   Leu Val Cys Leu Ala Arg Gly Phe Phe Pro Asp His Val Glu Leu Ser
                   165                 170                 175
   Trp Trp Val Asn Gly Lys Glu Val His Ser Gly Val Ser Thr Asp Pro
               180                 185                 190
   Gln Ala Tyr Lys Glu Ser Asn Tyr Ser Tyr Cys Leu Ser Ser Arg Leu
           195                 200                 205
   Arg Val Ser Ala Thr Phe Trp His Asn Pro Arg Asn His Phe Arg Cys
       210                 215                 220
   Gln Val Gln Phe His Gly Leu Ser Glu Glu Asp Lys Trp Pro Glu Gly
   225                 230                 235                 240
   Ser Pro Lys Pro Val Thr Gln Asn Ile Ser Ala Glu Ala Trp Gly Arg
                   245                 250                 255
   Ala Asp Cys Gly Ile Thr Ser Ala Ser Tyr Gln Gln Gly Val Leu Ser
               260                 265                 270
   Ala Thr Ile Leu Tyr Glu Ile Leu Leu Gly Lys Ala Thr Leu Tyr Ala
           275                 280                 285
   Val Leu Val Ser Thr Leu Val Val Met Ala Met Val Lys Arg Lys Asn
       290                 295                 300
   Ser
   305
<210> 17
   <211> 305
   <212> PRT
   <213> Artificial
<220>
   <223> Synthetic
<400> 17
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Arg Asn Val Thr Pro Pro Lys Val Ser
       130                 135                 140
   Leu Phe Glu Pro Ser Lys Ala Glu Ile Ala Asn Lys Gln Lys Ala Thr
   145                 150                 155                 160
   Leu Val Cys Leu Ala Arg Gly Phe Phe Pro Asp His Val Glu Leu Ser
                   165                 170                 175
   Trp Trp Val Asn Gly Lys Glu Val His Ser Gly Val Ser Thr Asp Pro
   180 185 190
   Gln Ala Tyr Lys Glu Ser Asn Tyr Ser Tyr Cys Leu Ser Ser Arg Leu
   195 200 205
   Arg Val Ser Ala Thr Phe Trp His Asn Pro Arg Asn His Phe Arg Cys
   210 215 220
   Gln Val Gln Phe His Gly Leu Ser Glu Glu Asp Lys Trp Pro Glu Gly
   225 230 235 240
   Ser Pro Lys Pro Val Thr Gln Asn Ile Ser Ala Glu Ala Trp Gly Arg
   245 250 255
   Ala Asp Cys Gly Ile Thr Ser Ala Ser Tyr His Gln Gly Val Leu Ser
   260 265 270
   Ala Thr Ile Leu Tyr Glu Ile Leu Leu Gly Lys Ala Thr Leu Tyr Ala
   275 280 285
   Val Leu Val Ser Gly Leu Val Leu Met Ala Met Val Lys Lys Lys Asn
   290 295 300
   Ser
   305
<210> 18
   <211> 269
   <212> PRT
   <213> Artificial
<220>
   <223> Synthetic
<400> 18
   Met Met Lys Ser Leu Arg Val Leu Leu Val Ile Leu Trp Leu Gln Leu
   1 5 10 15
   Ser Trp Val Trp Ser Gln Gln Lys Glu Val Glu Gln Asn Ser Gly Pro
   20 25 30
   Leu Ser Val Pro Glu Gly Ala Ile Ala Ser Leu Asn Cys Thr Tyr Ser
   35 40 45
   Asp Arg Gly Ser Gln Ser Phe Phe Trp Tyr Arg Gln Tyr Ser Gly Lys
   50 55 60
   Ser Pro Glu Leu Ile Met Phe Ile Tyr Ser Asn Gly Asp Lys Glu Asp
   65                  70                  75                  80
   Gly Arg Phe Thr Ala Gln Leu Asn Lys Ala Ser Gln Tyr Val Ser Leu
                   85                  90                  95
   Leu Ile Arg Asp Ser Gln Pro Ser Asp Ser Ala Thr Tyr Leu Cys Ala
               100                 105                 110
   Val Asn Phe Gly Gly Gly Lys Leu Ile Phe Gly Gln Gly Thr Glu Leu
           115                 120                 125
   Ser Val Lys Pro Asn Ile Gln Asn Pro Glu Pro Ala Val Tyr Gln Leu
       130                 135                 140
   Lys Asp Pro Arg Ser Gln Asp Ser Thr Leu Cys Leu Phe Thr Asp Phe
   145                 150                 155                 160
   Asp Ser Gln Ile Asn Val Pro Lys Thr Met Glu Ser Gly Thr Phe Ile
                   165                 170                 175
   Thr Asp Lys Thr Val Leu Asp Met Lys Ala Met Asp Ser Lys Ser Asn
               180                 185                 190
   Gly Ala Ile Ala Trp Ser Asn Gln Thr Ser Phe Thr Cys Gln Asp Ile
           195                 200                 205
   Phe Lys Glu Thr Asn Ala Thr Tyr Pro Ser Ser Asp Val Pro Cys Asp
       210                 215                 220
   Ala Thr Leu Thr Glu Lys Ser Phe Glu Thr Asp Met Asn Leu Asn Phe
   225                 230                 235                 240
   Gln Asn Leu Ser Val Met Gly Leu Arg Ile Leu Leu Leu Lys Val Ala
                   245                 250                 255
   Gly Phe Asn Leu Leu Met Thr Leu Arg Leu Trp Ser Ser
               260                 265
<210> 19
   <211> 304
   <212> PRT
   <213> Artificial
<220>
   <223> Synthetic
<400> 19
   Met Arg Ile Arg Leu Leu Cys Cys Val Ala Phe Ser Leu Leu Trp Ala
   1               5                   10                  15
   Gly Pro Val Ile Ala Gly Ile Thr Gln Ala Pro Thr Ser Gln Ile Leu
               20                  25                  30
   Ala Ala Gly Arg Arg Met Thr Leu Arg Cys Thr Gln Asp Met Arg His
           35                  40                  45
   Asn Ala Met Tyr Trp Tyr Arg Gln Asp Leu Gly Leu Gly Leu Arg Leu
       50                  55                  60
   Ile His Tyr Ser Asn Thr Ala Gly Thr Thr Gly Lys Gly Glu Val Pro
   65                  70                  75                  80
   Asp Gly Tyr Ser Val Ser Arg Ala Asn Thr Asp Asp Phe Pro Leu Thr
                   85                  90                  95
   Leu Ala Ser Ala Val Pro Ser Gln Thr Ser Val Tyr Phe Cys Ala Ser
               100                 105                 110
   Ser Leu Ser Phe Gly Thr Glu Ala Phe Phe Gly Gln Gly Thr Arg Leu
           115                 120                 125
   Thr Val Val Glu Asp Leu Arg Asn Val Thr Pro Pro Lys Val Ser Leu
       130                 135                 140
   Phe Glu Pro Ser Lys Ala Glu Ile Ala Asn Lys Gln Lys Ala Thr Leu
   145                 150                 155                 160
   Val Cys Leu Ala Arg Gly Phe Phe Pro Asp His Val Glu Leu Ser Trp
                   165                 170                 175
   Trp Val Asn Gly Lys Glu Val His Ser Gly Val Ser Thr Asp Pro Gln
               180                 185                 190
   Ala Tyr Lys Glu Ser Asn Tyr Ser Tyr Cys Leu Ser Ser Arg Leu Arg
           195                 200                 205
   Val Ser Ala Thr Phe Trp His Asn Pro Arg Asn His Phe Arg Cys Gln
       210                 215                 220
   Val Gln Phe His Gly Leu Ser Glu Glu Asp Lys Trp Pro Glu Gly Ser
   225                 230                 235                 240
   Pro Lys Pro Val Thr Gln Asn Ile Ser Ala Glu Ala Trp Gly Arg Ala
   245 250 255
   Asp Cys Gly Ile Thr Ser Ala Ser Tyr Gln Gln Gly Val Leu Ser Ala
   260 265 270
   Thr Ile Leu Tyr Glu Ile Leu Leu Gly Lys Ala Thr Leu Tyr Ala Val
   275 280 285
   Leu Val Ser Thr Leu Val Val Met Ala Met Val Lys Arg Lys Asn Ser
   290 295 300
<210> 20
   <211> 304
   <212> PRT
   <213> Artificial
<220>
   <223> Synthetic
<400> 20
   Met Arg Ile Arg Leu Leu Cys Cys Val Ala Phe Ser Leu Leu Trp Ala
   1 5 10 15
   Gly Pro Val Ile Ala Gly Ile Thr Gln Ala Pro Thr Ser Gln Ile Leu
   20 25 30
   Ala Ala Gly Arg Arg Met Thr Leu Arg Cys Thr Gln Asp Met Arg His
   35 40 45
   Asn Ala Met Tyr Trp Tyr Arg Gln Asp Leu Gly Leu Gly Leu Arg Leu
   50 55 60
   Ile His Tyr Ser Asn Thr Ala Gly Thr Thr Gly Lys Gly Glu Val Pro
   65 70 75 80
   Asp Gly Tyr Ser Val Ser Arg Ala Asn Thr Asp Asp Phe Pro Leu Thr
   85 90 95
   Leu Ala Ser Ala Val Pro Ser Gln Thr Ser Val Tyr Phe Cys Ala Ser
   100 105 110
   Ser Leu Ser Phe Gly Thr Glu Ala Phe Phe Gly Gln Gly Thr Arg Leu
   115 120 125
   Thr Val Val Glu Asp Leu Arg Asn Val Thr Pro Pro Lys Val Ser Leu
   130 135 140
   Phe Glu Pro Ser Lys Ala Glu Ile Ala Asn Lys Gln Lys Ala Thr Leu
   145 150 155 160
   Val Cys Leu Ala Arg Gly Phe Phe Pro Asp His Val Glu Leu Ser Trp
   165 170 175
   Trp Val Asn Gly Lys Glu Val His Ser Gly Val Ser Thr Asp Pro Gln
   180 185 190
   Ala Tyr Lys Glu Ser Asn Tyr Ser Tyr Cys Leu Ser Ser Arg Leu Arg
   195 200 205
   Val Ser Ala Thr Phe Trp His Asn Pro Arg Asn His Phe Arg Cys Gln
   210 215 220
   Val Gln Phe His Gly Leu Ser Glu Glu Asp Lys Trp Pro Glu Gly Ser
   225 230 235 240
   Pro Lys Pro Val Thr Gln Asn Ile Ser Ala Glu Ala Trp Gly Arg Ala
   245 250 255
   Asp Cys Gly Ile Thr Ser Ala Ser Tyr His Gln Gly Val Leu Ser Ala
   260 265 270
   Thr Ile Leu Tyr Glu Ile Leu Leu Gly Lys Ala Thr Leu Tyr Ala Val
   275 280 285
   Leu Val Ser Gly Leu Val Leu Met Ala Met Val Lys Lys Lys Asn Ser
   290 295 300
<210> 21
   <211> 269
   <212> PRT
   <213> Artificial
<220>
   <223> Synthetic
<400> 21
   Met Asn Tyr Ser Pro Gly Leu Val Ser Leu Ile Leu Leu Leu Leu Gly
   1 5 10 15
   Arg Thr Arg Gly Asn Ser Val Thr Gln Met Glu Gly Pro Val Thr Leu
   20 25 30
   Ser Glu Glu Ala Phe Leu Thr Ile Asn Cys Thr Tyr Thr Ala Thr Gly
   35 40 45
   Tyr Pro Ser Leu Phe Trp Tyr Val Gln Tyr Pro Gly Glu Gly Leu Gln
       50                  55                  60
   Leu Leu Leu Lys Ala Thr Lys Ala Asp Asp Lys Gly Ser Asn Lys Gly
   65                  70                  75                  80
   Phe Glu Ala Thr Tyr Arg Lys Glu Thr Thr Ser Phe His Leu Glu Lys
                   85                  90                  95
   Gly Ser Val Gln Val Ser Asp Ser Ala Val Tyr Phe Cys Ala Leu Ser
               100                 105                 110
   Ala Asn Gln Ala Gly Thr Ala Leu Ile Phe Gly Lys Gly Thr Thr Leu
           115                 120                 125
   Ser Val Ser Ser Asn Ile Gln Asn Pro Glu Pro Ala Val Tyr Gln Leu
       130                 135                 140
   Lys Asp Pro Arg Ser Gln Asp Ser Thr Leu Cys Leu Phe Thr Asp Phe
   145                 150                 155                 160
   Asp Ser Gln Ile Asn Val Pro Lys Thr Met Glu Ser Gly Thr Phe Ile
                   165                 170                 175
   Thr Asp Lys Thr Val Leu Asp Met Lys Ala Met Asp Ser Lys Ser Asn
               180                 185                 190
   Gly Ala Ile Ala Trp Ser Asn Gln Thr Ser Phe Thr Cys Gln Asp Ile
           195                 200                 205
   Phe Lys Glu Thr Asn Ala Thr Tyr Pro Ser Ser Asp Val Pro Cys Asp
       210                 215                 220
   Ala Thr Leu Thr Glu Lys Ser Phe Glu Thr Asp Met Asn Leu Asn Phe
   225                 230                 235                 240
   Gln Asn Leu Ser Val Met Gly Leu Arg Ile Leu Leu Leu Lys Val Ala
                   245                 250                 255
   Gly Phe Asn Leu Leu Met Thr Leu Arg Leu Trp Ser Ser
               260                 265
<210> 22
   <211> 301
   <212> PRT
   <213> Artificial
<220>
   <223> Synthetic
<400> 22
   Met Leu Leu Leu Leu Leu Leu Leu Gly Pro Gly Ser Gly Leu Gly Ala
   1               5                   10                  15
   Val Val Ser Gln His Pro Ser Arg Val Ile Cys Lys Ser Gly Thr Ser
               20                  25                  30
   Val Lys Ile Glu Cys Arg Ser Leu Asp Phe Gln Ala Thr Thr Met Phe
           35                  40                  45
   Trp Tyr Arg Gln Phe Pro Lys Gln Ser Leu Met Leu Met Ala Thr Ser
       50                  55                  60
   Asn Glu Gly Ser Lys Ala Thr Tyr Glu Gln Gly Val Glu Lys Asp Lys
   65                  70                  75                  80
   Phe Leu Ile Asn His Ala Ser Leu Thr Leu Ser Thr Leu Thr Val Thr
                   85                  90                  95
   Ser Ala His Pro Glu Asp Ser Ser Phe Tyr Ile Cys Ser Ala Ser Val
               100                 105                 110
   Ala Thr Glu Thr Gln Tyr Phe Gly Pro Gly Thr Arg Leu Leu Val Leu
           115                 120                 125
   Glu Asp Leu Arg Asn Val Thr Pro Pro Lys Val Ser Leu Phe Glu Pro
       130                 135                 140
   Ser Lys Ala Glu Ile Ala Asn Lys Gln Lys Ala Thr Leu Val Cys Leu
   145                 150                 155                 160
   Ala Arg Gly Phe Phe Pro Asp His Val Glu Leu Ser Trp Trp Val Asn
                   165                 170                 175
   Gly Lys Glu Val His Ser Gly Val Ser Thr Asp Pro Gln Ala Tyr Lys
               180                 185                 190
   Glu Ser Asn Tyr Ser Tyr Cys Leu Ser Ser Arg Leu Arg Val Ser Ala
           195                 200                 205
   Thr Phe Trp His Asn Pro Arg Asn His Phe Arg Cys Gln Val Gln Phe
       210                 215                 220
   His Gly Leu Ser Glu Glu Asp Lys Trp Pro Glu Gly Ser Pro Lys Pro
   225                 230                 235                 240
   Val Thr Gln Asn Ile Ser Ala Glu Ala Trp Gly Arg Ala Asp Cys Gly
                   245                 250                 255
   Ile Thr Ser Ala Ser Tyr Gln Gln Gly Val Leu Ser Ala Thr Ile Leu
               260                 265                 270
   Tyr Glu Ile Leu Leu Gly Lys Ala Thr Leu Tyr Ala Val Leu Val Ser
           275                 280                 285
   Thr Leu Val Val Met Ala Met Val Lys Arg Lys Asn Ser
       290                 295                 300
<210> 23
   <211> 301
   <212> PRT
   <213> Artificial
<220>
   <223> Synthetic
<400> 23
   Met Leu Leu Leu Leu Leu Leu Leu Gly Pro Gly Ser Gly Leu Gly Ala
   1               5                   10                  15
   Val Val Ser Gln His Pro Ser Arg Val Ile Cys Lys Ser Gly Thr Ser
               20                  25                  30
   Val Lys Ile Glu Cys Arg Ser Leu Asp Phe Gln Ala Thr Thr Met Phe
           35                  40                  45
   Trp Tyr Arg Gln Phe Pro Lys Gln Ser Leu Met Leu Met Ala Thr Ser
       50                  55                  60
   Asn Glu Gly Ser Lys Ala Thr Tyr Glu Gln Gly Val Glu Lys Asp Lys
   65                  70                  75                  80
   Phe Leu Ile Asn His Ala Ser Leu Thr Leu Ser Thr Leu Thr Val Thr
                   85                  90                  95
   Ser Ala His Pro Glu Asp Ser Ser Phe Tyr Ile Cys Ser Ala Ser Val
               100                 105                 110
   Ala Thr Glu Thr Gln Tyr Phe Gly Pro Gly Thr Arg Leu Leu Val Leu
   115 120 125
   Glu Asp Leu Arg Asn Val Thr Pro Pro Lys Val Ser Leu Phe Glu Pro
   130 135 140
   Ser Lys Ala Glu Ile Ala Asn Lys Gln Lys Ala Thr Leu Val Cys Leu
   145 150 155 160
   Ala Arg Gly Phe Phe Pro Asp His Val Glu Leu Ser Trp Trp Val Asn
   165 170 175
   Gly Lys Glu Val His Ser Gly Val Ser Thr Asp Pro Gln Ala Tyr Lys
   180 185 190
   Glu Ser Asn Tyr Ser Tyr Cys Leu Ser Ser Arg Leu Arg Val Ser Ala
   195 200 205
   Thr Phe Trp His Asn Pro Arg Asn His Phe Arg Cys Gln Val Gln Phe
   210 215 220
   His Gly Leu Ser Glu Glu Asp Lys Trp Pro Glu Gly Ser Pro Lys Pro
   225 230 235 240
   Val Thr Gln Asn Ile Ser Ala Glu Ala Trp Gly Arg Ala Asp Cys Gly
   245 250 255
   Ile Thr Ser Ala Ser Tyr His Gln Gly Val Leu Ser Ala Thr Ile Leu
   260 265 270
   Tyr Glu Ile Leu Leu Gly Lys Ala Thr Leu Tyr Ala Val Leu Val Ser
   275 280 285
   Gly Leu Val Leu Met Ala Met Val Lys Lys Lys Asn Ser
   290 295 300
<210> 24
   <211> 273
   <212> PRT
   <213> Artificial
<220>
   <223> Synthetic
<400> 24
   Met Val Lys Ile Arg Gln Phe Leu Leu Ala Ile Leu Trp Leu Gln Leu
   1 5 10 15
   Ser Cys Val Ser Ala Ala Lys Asn Glu Val Glu Gln Ser Pro Gln Asn
   20 25 30
   Leu Thr Ala Gln Glu Gly Glu Phe Ile Thr Ile Asn Cys Ser Tyr Ser
   35 40 45
   Val Gly Ile Ser Ala Leu His Trp Leu Gln Gln His Pro Gly Gly Gly
   50 55 60
   Ile Val Ser Leu Phe Met Leu Ser Ser Gly Lys Lys Lys His Gly Arg
   65 70 75 80
   Leu Ile Ala Thr Ile Asn Ile Gln Glu Lys His Ser Ser Leu His Ile
   85 90 95
   Thr Ala Ser His Pro Arg Asp Ser Ala Val Tyr Ile Cys Ala Ala Ser
   100 105 110
   Leu Ile Gln Gly Ala Gln Lys Leu Val Phe Gly Gln Gly Thr Arg Leu
   115 120 125
   Thr Ile Asn Pro Asn Ile Gln Asn Pro Asp Pro Ala Val Tyr Gln Leu
   130 135 140
   Arg Asp Ser Lys Ser Ser Asp Lys Ser Val Cys Leu Phe Thr Asp Phe
   145 150 155 160
   Asp Ser Gln Thr Asn Val Ser Gln Ser Lys Asp Ser Asp Val Tyr Ile
   165 170 175
   Thr Asp Lys Thr Val Leu Asp Met Arg Ser Met Asp Phe Lys Ser Asn
   180 185 190
   Ser Ala Val Ala Trp Ser Asn Lys Ser Asp Phe Ala Cys Ala Asn Ala
   195 200 205
   Phe Asn Asn Ser Ile Ile Pro Glu Asp Thr Phe Phe Pro Ser Pro Glu
   210 215 220
   Ser Ser Cys Asp Val Lys Leu Val Glu Lys Ser Phe Glu Thr Asp Thr
   225 230 235 240
   Asn Leu Asn Phe Gln Asn Leu Ser Val Ile Gly Phe Arg Ile Leu Leu
   245 250 255
   Leu Lys Val Ala Gly Phe Asn Leu Leu Met Thr Leu Arg Leu Trp Ser
   260 265 270
   Ser
<210> 25
   <211> 304
   <212> PRT
   <213> Artificial
<220>
   <223> Synthetic
<400> 25
   Met Asp Ser Trp Thr Phe Cys Cys Val Ser Leu Cys Ile Leu Val Ala
   1               5                   10                  15
   Lys His Thr Asp Ala Gly Val Ile Gln Ser Pro Arg His Glu Val Thr
               20                  25                  30
   Glu Met Gly Gln Glu Val Thr Leu Arg Cys Lys Pro Ile Ser Gly His
           35                  40                  45
   Asn Ser Leu Phe Trp Tyr Arg Gln Thr Met Met Arg Gly Leu Glu Leu
       50                  55                  60
   Leu Ile Tyr Phe Asn Asn Asn Val Pro Ile Asp Asp Ser Gly Met Pro
   65                  70                  75                  80
   Glu Asp Arg Phe Ser Ala Lys Met Pro Asn Ala Ser Phe Ser Thr Leu
                   85                  90                  95
   Lys Ile Gln Pro Ser Glu Pro Arg Asp Ser Ala Val Tyr Phe Cys Ala
               100                 105                 110
   Ser Ser Pro Gly Gly Asn Glu Gln Phe Phe Gly Pro Gly Thr Arg Leu
           115                 120                 125
   Thr Val Leu Glu Asp Leu Arg Asn Val Thr Pro Pro Lys Val Ser Leu
       130                 135                 140
   Phe Glu Pro Ser Lys Ala Glu Ile Ala Asn Lys Gln Lys Ala Thr Leu
   145                 150                 155                 160
   Val Cys Leu Ala Arg Gly Phe Phe Pro Asp His Val Glu Leu Ser Trp
                   165                 170                 175
   Trp Val Asn Gly Lys Glu Val His Ser Gly Val Ser Thr Asp Pro Gln
               180                 185                 190
   Ala Tyr Lys Glu Ser Asn Tyr Ser Tyr Cys Leu Ser Ser Arg Leu Arg
           195                 200                 205
   Val Ser Ala Thr Phe Trp His Asn Pro Arg Asn His Phe Arg Cys Gln
       210                 215                 220
   Val Gln Phe His Gly Leu Ser Glu Glu Asp Lys Trp Pro Glu Gly Ser
   225                 230                 235                 240
   Pro Lys Pro Val Thr Gln Asn Ile Ser Ala Glu Ala Trp Gly Arg Ala
                   245                 250                 255
   Asp Cys Gly Ile Thr Ser Ala Ser Tyr Gln Gln Gly Val Leu Ser Ala
               260                 265                 270
   Thr Ile Leu Tyr Glu Ile Leu Leu Gly Lys Ala Thr Leu Tyr Ala Val
           275                 280                 285
   Leu Val Ser Thr Leu Val Val Met Ala Met Val Lys Arg Lys Asn Ser
       290                 295                 300
<210> 26
   <211> 304
   <212> PRT
   <213> Artificial
<220>
   <223> Synthetic
<400> 26
   Met Asp Ser Trp Thr Phe Cys Cys Val Ser Leu Cys Ile Leu Val Ala
   1               5                   10                  15
   Lys His Thr Asp Ala Gly Val Ile Gln Ser Pro Arg His Glu Val Thr
               20                  25                  30
   Glu Met Gly Gln Glu Val Thr Leu Arg Cys Lys Pro Ile Ser Gly His
           35                  40                  45
   Asn Ser Leu Phe Trp Tyr Arg Gln Thr Met Met Arg Gly Leu Glu Leu
       50                  55                  60
   Leu Ile Tyr Phe Asn Asn Asn Val Pro Ile Asp Asp Ser Gly Met Pro
   65                  70                  75                  80
   Glu Asp Arg Phe Ser Ala Lys Met Pro Asn Ala Ser Phe Ser Thr Leu
                   85                  90                  95
   Lys Ile Gln Pro Ser Glu Pro Arg Asp Ser Ala Val Tyr Phe Cys Ala
               100                 105                 110
   Ser Ser Pro Gly Gly Asn Glu Gln Phe Phe Gly Pro Gly Thr Arg Leu
           115                 120                 125
   Thr Val Leu Glu Asp Leu Arg Asn Val Thr Pro Pro Lys Val Ser Leu
       130                 135                 140
   Phe Glu Pro Ser Lys Ala Glu Ile Ala Asn Lys Gln Lys Ala Thr Leu
   145                 150                 155                 160
   Val Cys Leu Ala Arg Gly Phe Phe Pro Asp His Val Glu Leu Ser Trp
                   165                 170                 175
   Trp Val Asn Gly Lys Glu Val His Ser Gly Val Ser Thr Asp Pro Gln
               180                 185                 190
   Ala Tyr Lys Glu Ser Asn Tyr Ser Tyr Cys Leu Ser Ser Arg Leu Arg
           195                 200                 205
   Val Ser Ala Thr Phe Trp His Asn Pro Arg Asn His Phe Arg Cys Gln
       210                 215                 220
   Val Gln Phe His Gly Leu Ser Glu Glu Asp Lys Trp Pro Glu Gly Ser
   225                 230                 235                 240
   Pro Lys Pro Val Thr Gln Asn Ile Ser Ala Glu Ala Trp Gly Arg Ala
                   245                 250                 255
   Asp Cys Gly Ile Thr Ser Ala Ser Tyr His Gln Gly Val Leu Ser Ala
               260                 265                 270
   Thr Ile Leu Tyr Glu Ile Leu Leu Gly Lys Ala Thr Leu Tyr Ala Val
           275                 280                 285
   Leu Val Ser Gly Leu Val Leu Met Ala Met Val Lys Lys Lys Asn Ser
       290                 295                 300
<210> 27
   <211> 795
   <212> DNA
   <213> Artificial
<220>
   <223> Synthetic
<400> 27
   atgttgcttg aacatttatt aataatcttg tggatgcagc tgacatgggt cagtggtcaa     60
   cagctgaatc agagtcctca atctatgttt atccaggaag gagaagatgt ctccatgaac    120
   tgcacttctt caagcatatt taacacctgg ctatggtaca agcaggaccc tggggaaggt    180
   cctgtcctct tgatagcctt atataaggct ggtgaattga cctcaaatgg aagactgact    240
   gctcagtttg gtataaccag aaaggacagc ttcctgaata tctcagcatc catacctagt    300
   gatgtaggca tctacttctg tgctggtggg accggtaacc agttctattt tgggacaggg    360
   acaagtttga cggtcattcc aaatatccag aacccagaac ctgctgtgta ccagttaaaa    420
   gatcctcggt ctcaggacag caccctctgc ctgttcaccg actttgactc ccaaatcaat    480
   gtgccgaaaa ccatggaatc tggaacgttc atcactgaca aaactgtgct ggacatgaaa    540
   gctatggatt ccaagagcaa tggggccatt gcctggagca accagacaag cttcacctgc    600
   caagatatct tcaaagagac caacgccacc taccccagtt cagacgttcc ctgtgatgcc    660
   acgttgactg agaaaagctt tgaaacagat atgaacctaa actttcaaaa cctgtcagtt    720
   atgggactcc gaatcctcct gctgaaagta gccggattta acctgctcat gacgctgagg    780
   ctgtggtcca gttga                                                     795
<210> 28
   <211> 918
   <212> DNA
   <213> Artificial
<220>
   <223> Synthetic
<400> 28
   atgggcacaa ggttgttctt ctatgtggcc ctttgtctcc tgtggacagg acacatggat      60
   gctggaatca cccagagccc aagacacaag gtcacagaga caggaacacc agtgactctg     120
   agatgtcacc agactgagaa ccaccgctat atgtactggt atcgacaaga cccggggcat     180
   gggctgaggc tgatccatta ctcatatggt gttaaagata ctgacaaagg agaagtctca     240
   gatggctata gtgtctctag atcaaagaca gaggatttcc tcctcactct ggagtccgct     300
   accagctccc agacatctgt gtacttctgt gccatcagtg aggtaggggt tgggcagccc     360
   cagcattttg gtgatgggac tcgactctcc atcctagagg atctgagaaa tgtgactcca     420
   cccaaggtct ccttgtttga gccatcaaaa gcagagattg caaacaaaca aaaggctacc     480
   ctcgtgtgct tggccagggg cttcttccct gaccacgtgg agctgagctg gtgggtgaat     540
   ggcaaggagg tccacagtgg ggtcagcacg gaccctcagg cctacaagga gagcaattat     600
   agctactgcc tgagcagccg cctgagggtc tctgctacct tctggcacaa tcctcgcaac     660
   cacttccgct gccaagtgca gttccatggg ctttcagagg aggacaagtg gccagagggc     720
   tcacccaaac ctgtcacaca gaacatcagt gcagaggcct ggggccgagc agactgtggg     780
   attacctcag catcctatca acaaggggtc ttgtctgcca ccatcctcta tgagatcctg     840
   ctagggaaag ccaccctgta tgctgtgctt gtcagtacac tggtggtgat ggctatggtc     900
   aaaagaaaga attcatga                                                   918
<210> 29
   <211> 918
   <212> DNA
   <213> Artificial
<220>
   <223> Synthetic
<400> 29
   atgggcacaa ggttgttctt ctatgtggcc ctttgtctcc tgtggacagg acacatggat     60
   gctggaatca cccagagccc aagacacaag gtcacagaga caggaacacc agtgactctg    120
   agatgtcacc agactgagaa ccaccgctat atgtactggt atcgacaaga cccggggcat    180
   gggctgaggc tgatccatta ctcatatggt gttaaagata ctgacaaagg agaagtctca    240
   gatggctata gtgtctctag atcaaagaca gaggatttcc tcctcactct ggagtccgct    300
   accagctccc agacatctgt gtacttctgt gccatcagtg aggtaggggt tgggcagccc    360
   cagcattttg gtgatgggac tcgactctcc atcctagagg atctgagaaa tgtgactcca    420
   cccaaggtct ccttgtttga gccatcaaaa gcagagattg caaacaaaca aaaggctacc    480
   ctcgtgtgct tggccagggg cttcttccct gaccacgtgg agctgagctg gtgggtgaat    540
   ggcaaggagg tccacagtgg ggtcagcacg gaccctcagg cctacaagga gagcaattat    600
   agctactgcc tgagcagccg cctgagggtc tctgctacct tctggcacaa tcctcgaaac    660
   cacttccgct gccaagtgca gttccatggg ctttcagagg aggacaagtg gccagagggc    720
   tcacccaaac ctgtcacaca gaacatcagt gcagaggcct ggggccgagc agactgtgga    780
   atcacttcag catcctatca tcagggggtt ctgtctgcaa ccatcctcta tgagatccta    840
   ctggggaagg ccaccctata tgctgtgctg gtcagtggcc tggtgctgat ggccatggtc    900
   aagaaaaaaa attcctga                                                  918
<210> 30
   <211> 810
   <212> DNA
   <213> Artificial
<220>
   <223> Synthetic
<400> 30
   atgatgaaat ccttgagagt tttactagtg atcctgtggc ttcagttgag ctgggtttgg      60
   agccaacaga aggaggtgga gcagaattct ggacccctca gtgttccaga gggagccatt     120
   gcctctctca actgcactta cagtgaccga ggttcccagt ccttcttctg gtacagacaa     180
   tattctggga aaagccctga gttgataatg ttcatatact ccaatggtga caaagaagat     240
   ggaaggttta cagcacagct caataaagcc agccagtatg tttctctgct catcagagac     300
   tcccagccca gtgattcagc cacctacctc tgtgccgtga acttcggagg aggaaagctt     360
   atcttcggac agggaacgga gttatctgtg aaacccaata tccagaaccc agaacctgct     420
   gtgtaccagt taaaagatcc tcggtctcag gacagcaccc tctgcctgtt caccgacttt     480
   gactcccaaa tcaatgtgcc gaaaaccatg gaatctggaa cgttcatcac tgacaaaact     540
   gtgctggaca tgaaagctat ggattccaag agcaatgggg ccattgcctg gagcaaccag     600
   acaagcttca cctgccaaga tatcttcaaa gagaccaacg ccacctaccc cagttcagac     660
   gttccctgtg atgccacgtt gactgagaaa agctttgaaa cagatatgaa cctaaacttt     720
   caaaacctgt cagttatggg actccgaatc ctcctgctga aagtagccgg atttaacctg     780
   ctcatgacgc tgaggctgtg gtccagttga                                      810
<210> 31
   <211> 915
   <212> DNA
   <213> Artificial
<220>
   <223> Synthetic
<400> 31
   atgagaatca ggctcctgtg ctgtgtggcc ttttctctcc tgtgggcagg tccagtgatt      60
   gctgggatca cccaggcacc aacatctcag atcctggcag caggacggcg catgacactg     120
   agatgtaccc aggatatgag acataatgcc atgtactggt atagacaaga tctaggactg     180
   gggctaaggc tcatccatta ttcaaatact gcaggtacca ctggcaaagg agaagtccct     240
   gatggttata gtgtctccag agcaaacaca gatgatttcc ccctcacgtt ggcgtctgct     300
   gtaccctctc agacatctgt gtacttctgt gccagcagcc taagtttcgg cactgaagct     360
   ttctttggac aaggcaccag actcacagtt gtagaggatc tgagaaatgt gactccaccc     420
   aaggtctcct tgtttgagcc atcaaaagca gagattgcaa acaaacaaaa ggctaccctc     480
   gtgtgcttgg ccaggggctt cttccctgac cacgtggagc tgagctggtg ggtgaatggc     540
   aaggaggtcc acagtggggt cagcacggac cctcaggcct acaaggagag caattatagc 600
   tactgcctga gcagccgcct gagggtctct gctaccttct ggcacaatcc tcgcaaccac 660
   ttccgctgcc aagtgcagtt ccatgggctt tcagaggagg acaagtggcc agagggctca 720
   cccaaacctg tcacacagaa catcagtgca gaggcctggg gccgagcaga ctgtgggatt 780
   acctcagcat cctatcaaca aggggtcttg tctgccacca tcctctatga gatcctgcta 840
   gggaaagcca ccctgtatgc tgtgcttgtc agtacactgg tggtgatggc tatggtcaaa 900
   agaaagaatt catga 915
<210> 32
   <211> 810
   <212> DNA
   <213> Artificial
<220>
   <223> Synthetic
<400> 32
   atggtgaaga tccggcaatt tttgttggct attttgtggc ttcagctaag ctgtgtaagt 60
   gccgccaaaa atgaagtgga gcagagtcct cagaacctga ctgcccagga aggagaattt 120
   atcacaatca actgcagtta ctcggtagga ataagtgcct tacactggct gcaacagcat 180
   ccaggaggag gcattgtttc cttgtttatg ctgagctcag ggaagaagaa gcatggaaga 240
   ttaattgcca caataaacat acaggaaaag cacagctccc tgcacatcac agcctcccat 300
   cccagagact ctgccgtcta catctgtgct gcctcattaa ttcagggagc ccagaagctg 360
   gtatttggcc aaggaaccag gctgactatc aacccaaata tccagaaccc agaacctgct 420
   gtgtaccagt taaaagatcc tcggtctcag gacagcaccc tctgcctgtt caccgacttt 480
   gactcccaaa tcaatgtgcc gaaaaccatg gaatctggaa cgttcatcac tgacaaaact 540
   gtgctggaca tgaaagctat ggattccaag agcaatgggg ccattgcctg gagcaaccag 600
   acaagcttca cctgccaaga tatcttcaaa gagaccaacg ccacctaccc cagttcagac 660
   gttccctgtg atgccacgtt gactgagaaa agctttgaaa cagatatgaa cctaaacttt 720
   caaaacctgt cagttatggg actccgaatc ctcctgctga aagtagccgg atttaacctg 780
   ctcatgacgc tgaggctgtg gtccagttga 810
<210> 33
   <211> 915
   <212> DNA
   <213> Artificial
<220>
   <223> Synthetic
<400> 33
   atggactcct ggaccttctg ctgtgtgtcc ctttgcatcc tggtagcgaa gcatacagat      60
   gctggagtta tccagtcacc ccgccatgag gtgacagaga tgggacaaga agtgactctg     120
   agatgtaaac caatttcagg ccacaactcc cttttctggt acagacagac catgatgcgg     180
   ggactggagt tgctcattta ctttaacaac aacgttccga tagatgattc agggatgccc     240
   gaggatcgat tctcagctaa gatgcctaat gcatcattct ccactctgaa gatccagccc     300
   tcagaaccca gggactcagc tgtgtacttc tgtgccagca gccccggggg caatgagcag     360
   ttcttcgggc cagggacacg gctcaccgtg ctagaggatc tgagaaatgt gactccaccc     420
   aaggtctcct tgtttgagcc atcaaaagca gagattgcaa acaaacaaaa ggctaccctc     480
   gtgtgcttgg ccaggggctt cttccctgac cacgtggagc tgagctggtg ggtgaatggc     540
   aaggaggtcc acagtggggt cagcacggac cctcaggcct acaaggagag caattatagc     600
   tactgcctga gcagccgcct gagggtctct gctaccttct ggcacaatcc tcgcaaccac     660
   ttccgctgcc aagtgcagtt ccatgggctt tcagaggagg acaagtggcc agagggctca     720
   cccaaacctg tcacacagaa catcagtgca gaggcctggg gccgagcaga ctgtgggatt     780
   acctcagcat cctatcaaca aggggtcttg tctgccacca tcctctatga gatcctgcta     840
   gggaaagcca ccctgtatgc tgtgcttgtc agtacactgg tggtgatggc tatggtcaaa     900
   agaaagaatt catga                                                      915
<210> 34
   <211> 810
   <212> DNA
   <213> Artificial
<220>
   <223> Synthetic
<400> 34
   atgaactatt ctccaggctt agtatctctg atactcttac tgcttggaag aacccgtgga      60
   aattcagtga cccagatgga agggccagtg actctctcag aagaggcctt cctgactata     120
   aactgcacgt acacagccac aggataccct tcccttttct ggtatgtcca atatcctgga      180
   gaaggtctac agctcctcct gaaagccacg aaggctgatg acaagggaag caacaaaggt     240
   tttgaagcca cataccgtaa agaaaccact tctttccact tggagaaagg ctcagttcaa     300
   gtgtcagact cagcggtgta cttctgtgct ctgagcgcca accaggcagg aactgctctg     360
   atctttggga agggaaccac cttatcagtg agttccaata tccagaaccc agaacctgct     420
   gtgtaccagt taaaagatcc tcggtctcag gacagcaccc tctgcctgtt caccgacttt     480
   gactcccaaa tcaatgtgcc gaaaaccatg gaatctggaa cgttcatcac tgacaaaact     540
   gtgctggaca tgaaagctat ggattccaag agcaatgggg ccattgcctg gagcaaccag     600
   acaagcttca cctgccaaga tatcttcaaa gagaccaacg ccacctaccc cagttcagac     660
   gttccctgtg atgccacgtt gactgagaaa agctttgaaa cagatatgaa cctaaacttt     720
   caaaacctgt cagttatggg actccgaatc ctcctgctga aagtagccgg atttaacctg     780
   ctcatgacgc tgaggctgtg gtccagttga                                     810
<210> 35
   <211> 906
   <212> DNA
   <213> Artificial
<220>
   <223> Synthetic
<400> 35
   atgctgctgc ttctgctgct tctggggcca ggctccgggc ttggtgctgt cgtctctcaa     60
   catccgagca gggttatctg taagagtgga acctctgtga agatcgagtg ccgttccctg    120
   gactttcagg ccacaactat gttttggtat cgtcagttcc cgaaacagag tctcatgctg    180
   atggcaactt ccaatgaggg ctccaaggcc acatacgagc aaggcgtcga gaaggacaag    240
   tttctcatca accatgcaag cctgaccttg tccactctga cagtgaccag tgcccatcct    300
   gaagacagca gcttctacat ctgcagtgct agcgtagcca cggagaccca gtacttcggg    360
   ccaggcacgc ggctcctggt gctcgaggat ctgagaaatg tgactccacc caaggtctcc    420
   ttgtttgagc catcaaaagc agagattgca aacaaacaaa aggctaccct cgtgtgcttg    480
   gccaggggct tcttccctga ccacgtggag ctgagctggt gggtgaatgg caaggaggtc    540
   cacagtgggg tcagcacgga ccctcaggcc tacaaggaga gcaattatag ctactgcctg    600
   agcagccgcc tgagggtctc tgctaccttc tggcacaatc ctcgcaacca cttccgctgc    660
   caagtgcagt tccatgggct ttcagaggag gacaagtggc cagagggctc acccaaacct    720
   gtcacacaga acatcagtgc agaggcctgg ggccgagcag actgtgggat tacctcagca    780
   tcctatcaac aaggggtctt gtctgccacc atcctctatg agatcctgct agggaaagcc    840
   accctgtatg ctgtgcttgt cagtacactg gtggtgatgg ctatggtcaa aagaaagaat    900
   tcatga                                                               906
<210> 36
   <211> 36
   <212> DNA
   <213> Artificial
<220>
   <223> Synthetic
<400> 36
   atctagagcc gccatggctc ctggcgctcc tcccag 36
<210> 37
   <211> 42
   <212> DNA
   <213> Artificial
<220>
   <223> Synthetic
<400> 37
   ggcagggtca gggttctgga tgtctggctt tataattagc tt 42
<210> 38
   <211> 37
   <212> DNA
   <213> Artificial
<220>
   <223> Synthetic
<400> 38
   atctagagcc gccatggcta caaggctcct ctgttac 37
<210> 39
   <211> 45
   <212> DNA
   <213> Artificial
<220>
   <223> Synthetic
<400> 39
   tgggaacacc ttgttcaggt cctctacaac tgtgagtctg gttcc 45
<210> 40
   <211> 33
   <212> DNA
   <213> Artificial
<220>
   <223> Synthetic
<400> 40
   ctaggcggcc gctcagctgg accacagccg cag 33
<210> 41
   <211> 39
   <212> DNA
   <213> Artificial
<220>
   <223> Synthetic
<400> 41
   ctaggcggcc gctcagaaat cctttctctt gaccatggc 39
<210> 42
   <211> 39
   <212> DNA
   <213> Artificial
<220>
   <223> Synthetic
<400> 42
   gctctagagc cgccatgttg cttgaacatt tattaataa 39
<210> 43
   <211> 45
   <212> DNA
   <213> Artificial
<220>
   <223> Synthetic
<400> 43
   agcaggttct gggttctgga tatttggaat gaccgtcaaa cttgt 45
<210> 44
   <211> 39
   <212> DNA
   <213> Artificial
<220>
   <223> Synthetic
<400> 44
   gcaagcttgc cgccatgggc acaaggttgt tcttctatg 39
<210> 45
   <211> 46
   <212> DNA
   <213> Artificial
<220>
   <223> Synthetic
<400> 45
   agagtcacat ttctcagatc ctctaggatg gagagtcgag tcccat 46
<210> 46
   <211> 33
   <212> DNA
   <213> Artificial
<220>
   <223> Synthetic
<400> 46
   ccgcggccgc tcaactggac cacagcctca gcg 33
<210> 47
   <211> 37
   <212> DNA
   <213> Artificial
<220>
   <223> Synthetic
<400> 47
   ccgcggccgc tcatgaattc tttcttttga ccatagc 37
<210> 48
   <211> 51
   <212> DNA
   <213> Artificial
<220>
   <223> Synthetic
<400> 48
   taatacgact cactataggg agagccgcca tgaactattc tccaggctta g 51
<210> 49
   <211> 48
   <212> DNA
   <213> Artificial
<220>
   <223> Synthetic
<400> 49
   cagcaggttc tgggttctgg atattggaac tcactgataa ggtggttc 48
<210> 50
   <211> 32
   <212> DNA
   <213> Artificial
<220>
   <223> Synthetic
<400> 50
   aatgcggccg ctcaactgga ccacagcctc ag 32
<210> 51
   <211> 56
   <212> DNA
   <213> Artificial
<220>
   <223> Synthetic
<400> 51
   taatacgact cactataggg agaagcttgc cgccatgctg ctgcttctgc tgcttc 56
<210> 52
   <211> 42
   <212> DNA
   <213> Artificial
<220>
   <223> Synthetic
<400> 52
   ggagtcacat ttctcagatc ctcgagcacc aggagccgcg tg 42
<210> 53
   <211> 37
   <212> DNA
   <213> Artificial
<220>
   <223> Synthetic
<400> 53
   aatgcggccg ctcatgaatt ctttcttttg accatag 37
<210> 54
   <211> 41
   <212> DNA
   <213> Artificial
<220>
   <223> Synthetic
<400> 54
   gctctagagc cgccatgatg aaatccttga gagttttact a 41
<210> 55
   <211> 45
   <212> DNA
   <213> Artificial
<220>
   <223> Synthetic
<400> 55
   agcaggttct gggttctgga tattgggttt cacagataac tccgt 45
<210> 56
   <211> 35
   <212> DNA
   <213> Artificial
<220>
   <223> Synthetic
<400> 56
   gcaagcttgc cgccatgaga atcaggctcc tgtgc 35
<210> 57
   <211> 43
   <212> DNA
   <213> Artificial
<220>
   <223> Synthetic
<400> 57
   gagtcacatt tctcagatcc tctacaactg tgagtctggt gcc 43
<210> 58
   <211> 61
   <212> DNA
   <213> Artificial
<220>
   <223> Synthetic
<400> 58
   taatacgact cactataggg agagtttaaa cgccgccatg gtgaagatcc ggcaattttt 60
   g 61
<210> 59
   <211> 45
   <212> DNA
   <213> Artificial
<220>
   <223> Synthetic
<400> 59
   cagcaggttc tgggttctgg atatttgggt tgatagtcag cctgg 45
<210> 60
   <211> 56
   <212> DNA
   <213> Artificial
<220>
   <223> Synthetic
<400> 60
   taatacgact cactataggg agaaagcttg ccgccatgga ctcctggacc ttctgc 56
<210> 61
   <211> 43
   <212> DNA
   <213> Artificial
<220>
   <223> Synthetic
<400> 61
   ggagtcacat ttctcagatc ctctagcacg gtgagccgtg tcc 43
<210> 62
   <211> 85
   <212> DNA
   <213> Artificial
<220>
   <223> Synthetic
<400> 62
   tttttttttt tttttttttt tttttttttt tttttttttt tttttttttt tttttttttt     60
   tttttcaact ggaccacagc ctcag                                           85
<210> 63
   <211> 86
   <212> DNA
   <213> Artificial
<220>
   <223> Synthetic
<400> 63
   tttttttttt tttttttttt tttttttttt tttttttttt tttttttttt tttttttttt     60
   tttttcatga attctttctt ttgacc                                          86
<210> 64
   <211> 9
   <212> PRT
   <213> Artificial
<220>
   <223> Synthetic
<400> 64
   Leu Leu Gly Arg Asn Ser Phe Glu Val
   1               5
<210> 65
   <211> 10
   <212> PRT
   <213> Artificial
<220>
   <223> Synthetic
<400> 65
   Glu Leu Ala Gly Ile Gly Ile Leu Thr Val
   1               5                   10
<210> 66
   <211> 9
   <212> PRT
   <213> Artificial
<220>
   <223> Synthetic
<400> 66
   Ile Met Asp Gln Val Pro Phe Ser Val
   1               5
<210> 67
   <211> 9
   <212> PRT
   <213> Artificial
<220>
   <223> Synthetic
<400> 67
   Tyr Leu Glu Pro Gly Pro Val Thr Ala
   1 5
<210> 68
   <211> 10
   <212> PRT
   <213> Artificial
<220>
   <223> Synthetic
<400> 68
   Phe Leu Pro Ser Asp Tyr Phe Pro Ser Val
   1 5 10
<210> 69
   <211> 9
   <212> PRT
   <213> Artificial
<220>
   <223> Synthetic
<400> 69
   Ser Thr Pro Pro Pro Gly Thr Arg Val
   1 5
<210> 70
   <211> 9
   <212> PRT
   <213> Artificial
<220>
   <223> Synthetic
<400> 70
   Gly Ile Leu Gly Phe Val Phe Thr Leu
   1 5
<210> 71
   <211> 20
   <212> PRT
   <213> Artificial
<220>
   <223> Synthetic
<400> 71
   Trp Ile Thr Gln Cys Phe Leu Pro Val Phe Leu Ala Gln Pro Pro Ser
   1               5                   10                  15
   Gly Gln Arg Ala
               20
<210> 72
   <211> 273
   <212> PRT
   <213> Artificial
<220>
   <223> Synthetic
<400> 72
   Met Leu Leu Ala Leu Leu Pro Val Leu Gly Ile His Phe Val Leu Arg
   1               5                   10                  15
   Asp Ala Gln Ala Gln Ser Val Thr Gln Pro Asp Ala Arg Val Thr Val
               20                  25                  30
   Ser Glu Gly Ala Ser Leu Gln Leu Arg Cys Lys Tyr Ser Tyr Ser Gly
           35                  40                  45
   Thr Pro Tyr Leu Phe Trp Tyr Val Gln Tyr Pro Arg Gln Gly Leu Gln
       50                  55                  60
   Leu Leu Leu Lys Tyr Tyr Ser Gly Asp Pro Val Val Gln Gly Val Asn
   65                  70                  75                  80
   Gly Phe Glu Ala Glu Phe Ser Lys Ser Asn Ser Ser Phe His Leu Arg
                   85                  90                  95
   Lys Ala Ser Val His Trp Ser Asp Ser Ala Val Tyr Phe Cys Val Leu
               100                 105                 110
   Ser Glu Asp Ser Asn Tyr Gln Leu Ile Trp Gly Ser Gly Thr Lys Leu
           115                 120                 125
   Ile Ile Lys Pro Asp Ile Gln Asn Pro Asp Pro Ala Val Tyr Gln Leu
       130                 135                 140
   Arg Asp Ser Lys Ser Ser Asp Lys Ser Val Cys Leu Phe Thr Asp Phe
   145                 150                 155                 160
   Asp Ser Gln Thr Asn Val Ser Gln Ser Lys Asp Ser Asp Val Tyr Ile
                   165                 170                 175
   Thr Asp Lys Thr Val Leu Asp Met Arg Ser Met Asp Phe Lys Ser Asn
               180                 185                 190
   Ser Ala Val Ala Trp Ser Asn Lys Ser Asp Phe Ala Cys Ala Asn Ala
           195                 200                 205
   Phe Asn Asn Ser Ile Ile Pro Glu Asp Thr Phe Phe Pro Ser Pro Glu
       210                 215                 220
   Ser Ser Cys Asp Val Lys Leu Val Glu Lys Ser Phe Glu Thr Asp Thr
   225                 230                 235                 240
   Asn Leu Asn Phe Gln Asn Leu Ser Val Ile Gly Phe Arg Ile Leu Leu
                   245                 250                 255
   Leu Lys Val Ala Gly Phe Asn Leu Leu Met Thr Leu Arg Leu Trp Ser
               260                 265                 270
   Ser
<210> 73
   <211> 310
   <212> PRT
   <213> Artificial
<220>
   <223> Synthetic
<400> 73
   Met Ala Thr Arg Leu Leu Cys Tyr Thr Val Leu Cys Leu Leu Gly Ala
   1               5                   10                  15
   Arg Ile Leu Asn Ser Lys Val Ile Gln Thr Pro Arg Tyr Leu Val Lys
               20                  25                  30
   Gly Gln Gly Gln Lys Ala Lys Met Arg Cys Ile Pro Glu Lys Gly His
           35                  40                  45
   Pro Val Val Phe Trp Tyr Gln Gln Asn Lys Asn Asn Glu Phe Lys Phe
       50                  55                  60
   Leu Ile Asn Phe Gln Asn Gln Glu Val Leu Gln Gln Ile Asp Met Thr
   65                  70                  75                  80
   Glu Lys Arg Phe Ser Ala Glu Cys Pro Ser Asn Ser Pro Cys Ser Leu
                   85                  90                  95
   Glu Ile Gln Ser Ser Glu Ala Gly Asp Ser Ala Leu Tyr Leu Cys Ala
               100                 105                 110
   Ser Ser Leu Ser Gly Gly Gly Thr Glu Val Phe Phe Gly Lys Gly Thr
           115                 120                 125
   Arg Leu Thr Val Val Glu Asp Leu Asn Lys Val Phe Pro Pro Glu Val
       130                 135                 140
   Ala Val Phe Glu Pro Ser Glu Ala Glu Ile Ser His Thr Gln Lys Ala
   145                 150                 155                 160
   Thr Leu Val Cys Leu Ala Thr Gly Phe Phe Pro Asp His Val Glu Leu
                   165                 170                 175
   Ser Trp Trp Val Asn Gly Lys Glu Val His Ser Gly Val Ser Thr Asp
               180                 185                 190
   Pro Gln Pro Leu Lys Glu Gln Pro Ala Leu Asn Asp Ser Arg Tyr Cys
           195                 200                 205
   Leu Ser Ser Arg Leu Arg Val Ser Ala Thr Phe Trp Gln Asn Pro Arg
       210                 215                 220
   Asn His Phe Arg Cys Gln Val Gln Phe Tyr Gly Leu Ser Glu Asn Asp
   225                 230                 235                 240
   Glu Trp Thr Gln Asp Arg Ala Lys Pro Val Thr Gln Ile Val Ser Ala
                   245                 250                 255
   Glu Ala Trp Gly Arg Ala Asp Cys Gly Phe Thr Ser Val Ser Tyr Gln
               260                 265                 270
   Gln Gly Val Leu Ser Ala Thr Ile Leu Tyr Glu Ile Leu Leu Gly Lys
           275                 280                 285
   Ala Thr Leu Tyr Ala Val Leu Val Ser Ala Leu Val Leu Met Ala Met
       290                 295                 300
   Val Lys Arg Lys Asp Phe
   305                 310
<210> 74
   <211> 822
   <212> DNA
   <213> Artificial
<220>
   <223> Synthetic
<400> 74
   atgctcctgg cgctcctccc agtgctgggg atacactttg tcctgagaga tgcccaagct     60
   cagtcagtga cgcagcccga tgctcgcgtc actgtctctg aaggagcctc tctgcagctg    120
   agatgcaagt attcctactc tgggacacct tatctgttct ggtatgtcca gtacccgcgg    180
   caggggctgc agctgctcct caagtactat tcaggagacc cagtggttca aggagtgaat    240
   ggcttcgagg ctgagttcag caagagtaac tcttccttcc acctgcggaa agcctctgtg    300
   cactggagcg actctgctgt gtacttctgt gttttgagcg aggatagcaa ctatcagttg    360
   atctggggct ctgggaccaa gctaattata aagccagaca tccagaaccc tgaccctgcc    420
   gtgtaccagc tgagagactc taaatccagt gacaagtctg tctgcctatt caccgatttt    480
   gattctcaaa caaatgtgtc acaaagtaag gattctgatg tgtatatcac agacaaaact    540
   gtgctagaca tgaggtctat ggacttcaag agcaacagtg ctgtggcctg gagcaacaaa    600
   tctgactttg catgtgcaaa cgccttcaac aacagcatta ttccagaaga caccttcttc    660
   cccagcccag aaagttcctg tgatgtcaag ctggtcgaga aaagctttga aacagatacg    720
   aacctaaact ttcaaaacct gtcagtgatt gggttccgaa tcctcctcct gaaggtggcc    780
   gggtttaatc tgctcatgac gctgcggctg tggtccagct ga                        822
<210> 75
   <211> 933
   <212> DNA
   <213> Artificial
<220>
   <223> Synthetic
<400> 75
   atggctacaa ggctcctctg ttacacagta ctttgtctcc tgggtgcaag aattttgaat      60
   tcaaaagtca ttcagactcc aagatatctg gtgaaagggc aaggacaaaa agcaaagatg     120
   aggtgtatcc ctgaaaaggg acatccagtt gtattctggt atcaacaaaa taagaacaat     180
   gagtttaaat ttttgattaa ctttcagaat caagaagttc ttcagcaaat agacatgact     240
   gaaaaacgat tctctgctga gtgtccttca aactcacctt gcagcctaga aattcagtcc     300
   tctgaggcag gagactcagc actgtacctc tgtgccagca gtctgtcagg gggcggcaca     360
   gaagtcttct ttggtaaagg aaccagactc acagttgtag aggacctgaa caaggtgttc     420
   ccacccgagg tcgctgtgtt tgagccatca gaagcagaga tctcccacac ccaaaaggcc     480
   acactggtgt gcctggccac aggcttcttc cctgaccacg tggagctgag ctggtgggtg     540
   aatgggaagg aggtgcacag tggggtcagc acggacccgc agcccctcaa ggagcagccc     600
   gccctcaatg actccagata ctgcctgagc agccgcctga gggtctcggc caccttctgg     660
   cagaaccccc gcaaccactt ccgctgtcaa gtccagttct acgggctctc ggagaatgac     720
   gagtggaccc aggatagggc caaacccgtc acccagatcg tcagcgccga ggcctggggt     780
   agagcagact gtggctttac ctcggtgtcc taccagcaag gggtcctgtc tgccaccatc     840
   ctctatgaga tcctgctagg gaaggccacc ctgtatgctg tgctggtcag cgcccttgtg     900
   ttgatggcca tggtcaagag aaaggatttc tga                                  933
<210> 76
   <211> 268
   <212> PRT
   <213> Homo sapiens
<220>
   <221> misc_feature
   <223> amino acid sequence of fully human MART F4 TCR - alpha chain
<400> 76
   Met Leu Leu Glu His Leu Leu Ile Ile Leu Trp Met Gln Leu Thr Trp
   1               5                   10                  15
   Val Ser Gly Gln Gln Leu Asn Gln Ser Pro Gln Ser Met Phe Ile Gln
               20                  25                  30
   Glu Gly Glu Asp Val Ser Met Asn Cys Thr Ser Ser Ser Ile Phe Asn
           35                  40                  45
   Thr Trp Leu Trp Tyr Lys Gln Asp Pro Gly Glu Gly Pro Val Leu Leu
       50                  55                  60
   Ile Ala Leu Tyr Lys Ala Gly Glu Leu Thr Ser Asn Gly Arg Leu Thr
   65                  70                  75                  80
   Ala Gln Phe Gly Ile Thr Arg Lys Asp Ser Phe Leu Asn Ile Ser Ala
                   85                  90                  95
   Ser Ile Pro Ser Asp Val Gly Ile Tyr Phe Cys Ala Gly Gly Thr Gly
               100                 105                 110
   Asn Gln Phe Tyr Phe Gly Thr Gly Thr Ser Leu Thr Val Ile Pro Asn
           115                 120                 125
   Ile Gln Asn Pro Asp Pro Ala Val Tyr Gln Leu Arg Asp Ser Lys Ser
       130                 135                 140
   Ser Asp Lys Ser Val Cys Leu Phe Thr Asp Phe Asp Ser Gln Thr Asn
   145                 150                 155                 160
   Val Ser Gln Ser Lys Asp Ser Asp Val Tyr Ile Thr Asp Lys Thr Val
                   165                 170                 175
   Leu Asp Met Arg Ser Met Asp Phe Lys Ser Asn Ser Ala Val Ala Trp
               180                 185                 190
   Ser Asn Lys Ser Asp Phe Ala Cys Ala Asn Ala Phe Asn Asn Ser Ile
           195                 200                 205
   Ile Pro Glu Asp Thr Phe Phe Pro Ser Pro Glu Ser Ser Cys Asp Val
       210                 215                 220
   Lys Leu Val Glu Lys Ser Phe Glu Thr Asp Thr Asn Leu Asn Phe Gln
   225                 230                 235                 240
   Asn Leu Ser Val Ile Gly Phe Arg Ile Leu Leu Leu Lys Val Ala Gly
                   245                 250                 255
   Phe Asn Leu Leu Met Thr Leu Arg Leu Trp Ser Ser
               260                 265
<210> 77
   <211> 309
   <212> PRT
   <213> Homo sapiens
<220>
   <221> misc_feature
   <223> amino acid sequence of fully human MART F4 TCR - beta chain
<400> 77
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Asn Lys Val Phe Pro Pro Glu Val Ala
       130                 135                 140
   Val Phe Glu Pro Ser Glu Ala Glu Ile Ser His Thr Gln Lys Ala Thr
   145                 150                 155                 160
   Leu Val Cys Leu Ala Thr Gly Phe Phe Pro Asp His Val Glu Leu Ser
                   165                 170                 175
   Trp Trp Val Asn Gly Lys Glu Val His Ser Gly Val Ser Thr Asp Pro
               180                 185                 190
   Gln Pro Leu Lys Glu Gln Pro Ala Leu Asn Asp Ser Arg Tyr Cys Leu
           195                 200                 205
   Ser Ser Arg Leu Arg Val Ser Ala Thr Phe Trp Gln Asn Pro Arg Asn
       210                 215                 220
   His Phe Arg Cys Gln Val Gln Phe Tyr Gly Leu Ser Glu Asn Asp Glu
   225                 230                 235                 240
   Trp Thr Gln Asp Arg Ala Lys Pro Val Thr Gln Ile Val Ser Ala Glu
   245 250 255
   Ala Trp Gly Arg Ala Asp Cys Gly Phe Thr Ser Val Ser Tyr Gln Gln
   260 265 270
   Gly Val Leu Ser Ala Thr Ile Leu Tyr Glu Ile Leu Leu Gly Lys Ala
   275 280 285
   Thr Leu Tyr Ala Val Leu Val Ser Ala Leu Val Leu Met Ala Met Val
   290 295 300
   Lys Arg Lys Asp Phe
   305
<210> 78
   <211> 273
   <212> PRT
   <213> Homo sapiens
<220>
   <221> misc_feature
   <223> amino acid sequence of fully human MART F5 TCR - alpha chain
<400> 78
   Met Met Lys Ser Leu Arg Val Leu Leu Val Ile Leu Trp Leu Gln Leu
   1               5                   10                  15
   Ser Trp Val Trp Ser Gln Gln Lys Glu Val Glu Gln Asn Ser Gly Pro
               20                  25                  30
   Leu Ser Val Pro Glu Gly Ala Ile Ala Ser Leu Asn Cys Thr Tyr Ser
           35                  40                  45
   Asp Arg Gly Ser Gln Ser Phe Phe Trp Tyr Arg Gln Tyr Ser Gly Lys
       50                  55                  60
   Ser Pro Glu Leu Ile Met Phe Ile Tyr Ser Asn Gly Asp Lys Glu Asp
   65                  70                  75                  80
   Gly Arg Phe Thr Ala Gln Leu Asn Lys Ala Ser Gln Tyr Val Ser Leu
                   85                  90                  95
   Leu Ile Arg Asp Ser Gln Pro Ser Asp Ser Ala Thr Tyr Leu Cys Ala
               100                 105                 110
   Val Asn Phe Gly Gly Gly Lys Leu Ile Phe Gly Gln Gly Thr Glu Leu
   115 120 125
   Ser Val Lys Pro Asn Ile Gln Asn Pro Asp Pro Ala Val Tyr Gln Leu
   130 135 140
   Arg Asp Ser Lys Ser Ser Asp Lys Ser Val Cys Leu Phe Thr Asp Phe
   145 150 155 160
   Asp Ser Gln Thr Asn Val Ser Gln Ser Lys Asp Ser Asp Val Tyr Ile
   165 170 175
   Thr Asp Lys Thr Val Leu Asp Met Arg Ser Met Asp Phe Lys Ser Asn
   180 185 190
   Ser Ala Val Ala Trp Ser Asn Lys Ser Asp Phe Ala Cys Ala Asn Ala
   195 200 205
   Phe Asn Asn Ser Ile Ile Pro Glu Asp Thr Phe Phe Pro Ser Pro Glu
   210 215 220
   Ser Ser Cys Asp Val Lys Leu Val Glu Lys Ser Phe Glu Thr Asp Thr
   225 230 235 240
   Asn Leu Asn Phe Gln Asn Leu Ser Val Ile Gly Phe Arg Ile Leu Leu
   245 250 255
   Leu Lys Val Ala Gly Phe Asn Leu Leu Met Thr Leu Arg Leu Trp Ser
   260 265 270
   Ser
<210> 79
   <211> 308
   <212> PRT
   <213> Homo sapiens
<220>
   <221> misc_feature
   <223> amino acid sequence of fully human MART F5 TCR - beta chain
<400> 79
   Met Arg Ile Arg Leu Leu Cys Cys Val Ala Phe Ser Leu Leu Trp Ala
   1 5 10 15
   Gly Pro Val Ile Ala Gly Ile Thr Gln Ala Pro Thr Ser Gln Ile Leu
   20 25 30
   Ala Ala Gly Arg Arg Met Thr Leu Arg Cys Thr Gln Asp Met Arg His
           35                  40                  45
   Asn Ala Met Tyr Trp Tyr Arg Gln Asp Leu Gly Leu Gly Leu Arg Leu
       50                  55                  60
   Ile His Tyr Ser Asn Thr Ala Gly Thr Thr Gly Lys Gly Glu Val Pro
   65                  70                  75                  80
   Asp Gly Tyr Ser Val Ser Arg Ala Asn Thr Asp Asp Phe Pro Leu Thr
                   85                  90                  95
   Leu Ala Ser Ala Val Pro Ser Gln Thr Ser Val Tyr Phe Cys Ala Ser
               100                 105                 110
   Ser Leu Ser Phe Gly Thr Glu Ala Phe Phe Gly Gln Gly Thr Arg Leu
           115                 120                 125
   Thr Val Val Glu Asp Leu Asn Lys Val Phe Pro Pro Glu Val Ala Val
       130                 135                 140
   Phe Glu Pro Ser Glu Ala Glu Ile Ser His Thr Gln Lys Ala Thr Leu
   145                 150                 155                 160
   Val Cys Leu Ala Thr Gly Phe Phe Pro Asp His Val Glu Leu Ser Trp
                   165                 170                 175
   Trp Val Asn Gly Lys Glu Val His Ser Gly Val Ser Thr Asp Pro Gln
               180                 185                 190
   Pro Leu Lys Glu Gln Pro Ala Leu Asn Asp Ser Arg Tyr Cys Leu Ser
           195                 200                 205
   Ser Arg Leu Arg Val Ser Ala Thr Phe Trp Gln Asn Pro Arg Asn His
       210                 215                 220
   Phe Arg Cys Gln Val Gln Phe Tyr Gly Leu Ser Glu Asn Asp Glu Trp
   225                 230                 235                 240
   Thr Gln Asp Arg Ala Lys Pro Val Thr Gln Ile Val Ser Ala Glu Ala
                   245                 250                 255
   Trp Gly Arg Ala Asp Cys Gly Phe Thr Ser Val Ser Tyr Gln Gln Gly
               260                 265                 270
   Val Leu Ser Ala Thr Ile Leu Tyr Glu Ile Leu Leu Gly Lys Ala Thr
   275 280 285
   Leu Tyr Ala Val Leu Val Ser Ala Leu Val Leu Met Ala Met Val Lys
   290 295 300
   Arg Lys Asp Phe
   305
<210> 80
   <211> 273
   <212> PRT
   <213> Homo sapiens
<220>
   <221> misc_feature
   <223> amino acid sequence of fully human gp100 TCR - alpha chain
<400> 80
   Met Val Lys Ile Arg Gln Phe Leu Leu Ala Ile Leu Trp Leu Gln Leu
   1 5 10 15
   Ser Cys Val Ser Ala Ala Lys Asn Glu Val Glu Gln Ser Pro Gln Asn
   20 25 30
   Leu Thr Ala Gln Glu Gly Glu Phe Ile Thr Ile Asn Cys Ser Tyr Ser
   35 40 45
   Val Gly Ile Ser Ala Leu His Trp Leu Gln Gln His Pro Gly Gly Gly
   50 55 60
   Ile Val Ser Leu Phe Met Leu Ser Ser Gly Lys Lys Lys His Gly Arg
   65 70 75 80
   Leu Ile Ala Thr Ile Asn Ile Gln Glu Lys His Ser Ser Leu His Ile
   85 90 95
   Thr Ala Ser His Pro Arg Asp Ser Ala Val Tyr Ile Cys Ala Ala Ser
   100 105 110
   Leu Ile Gln Gly Ala Gln Lys Leu Val Phe Gly Gln Gly Thr Arg Leu
   115 120 125
   Thr Ile Asn Pro Asn Ile Gln Asn Pro Asp Pro Ala Val Tyr Gln Leu
   130 135 140
   Arg Asp Ser Lys Ser Ser Asp Lys Ser Val Cys Leu Phe Thr Asp Phe
   145                 150                 155                 160
   Asp Ser Gln Thr Asn Val Ser Gln Ser Lys Asp Ser Asp Val Tyr Ile
                   165                 170                 175
   Thr Asp Lys Thr Val Leu Asp Met Arg Ser Met Asp Phe Lys Ser Asn
               180                 185                 190
   Ser Ala Val Ala Trp Ser Asn Lys Ser Asp Phe Ala Cys Ala Asn Ala
           195                 200                 205
   Phe Asn Asn Ser Ile Ile Pro Glu Asp Thr Phe Phe Pro Ser Pro Glu
       210                 215                 220
   Ser Ser Cys Asp Val Lys Leu Val Glu Lys Ser Phe Glu Thr Asp Thr
   225                 230                 235                 240
   Asn Leu Asn Phe Gln Asn Leu Ser Val Ile Gly Phe Arg Ile Leu Leu
                   245                 250                 255
   Leu Lys Val Ala Gly Phe Asn Leu Leu Met Thr Leu Arg Leu Trp Ser
               260                 265                 270
   Ser
<210> 81
   <211> 310
   <212> PRT
   <213> Homo sapiens
<220>
   <221> misc_feature
   <223> amino acid sequence of fully human gp100 TCR - beta chain
<400> 81
   Met Asp Ser Trp Thr Phe Cys Cys Val Ser Leu Cys Ile Leu Val Ala
   1               5                   10                  15
   Lys His Thr Asp Ala Gly Val Ile Gln Ser Pro Arg His Glu Val Thr
               20                  25                  30
   Glu Met Gly Gln Glu Val Thr Leu Arg Cys Lys Pro Ile Ser Gly His
           35                  40                  45
   Asn Ser Leu Phe Trp Tyr Arg Gln Thr Met Met Arg Gly Leu Glu Leu
   50 55 60
   Leu Ile Tyr Phe Asn Asn Asn Val Pro Ile Asp Asp Ser Gly Met Pro
   65 70 75 80
   Glu Asp Arg Phe Ser Ala Lys Met Pro Asn Ala Ser Phe Ser Thr Leu
   85 90 95
   Lys Ile Gln Pro Ser Glu Pro Arg Asp Ser Ala Val Tyr Phe Cys Ala
   100 105 110
   Ser Ser Pro Gly Gly Asn Glu Gln Phe Phe Gly Pro Gly Thr Arg Leu
   115 120 125
   Thr Val Leu Glu Asp Leu Lys Asn Val Phe Pro Pro Glu Val Ala Val
   130 135 140
   Phe Glu Pro Ser Glu Ala Glu Ile Ser His Thr Gln Lys Ala Thr Leu
   145 150 155 160
   Val Cys Leu Ala Thr Gly Phe Tyr Pro Asp His Val Glu Leu Ser Trp
   165 170 175
   Trp Val Asn Gly Lys Glu Val His Ser Gly Val Ser Thr Asp Pro Gln
   180 185 190
   Pro Leu Lys Glu Gln Pro Ala Leu Asn Asp Ser Arg Tyr Cys Leu Ser
   195 200 205
   Ser Arg Leu Arg Val Ser Ala Thr Phe Trp Gln Asn Pro Arg Asn His
   210 215 220
   Phe Arg Cys Gln Val Gln Phe Tyr Gly Leu Ser Glu Asn Asp Glu Trp
   225 230 235 240
   Thr Gln Asp Arg Ala Lys Pro Val Thr Gln Ile Val Ser Ala Glu Ala
   245 250 255
   Trp Gly Arg Ala Asp Cys Gly Phe Thr Ser Glu Ser Tyr Gln Gln Gly
   260 265 270
   Val Leu Ser Ala Thr Ile Leu Tyr Glu Ile Leu Leu Gly Lys Ala Thr
   275 280 285
   Leu Tyr Ala Val Leu Val Ser Ala Leu Val Leu Met Ala Met Val Lys
   290 295 300
   Arg Lys Asp Ser Arg Gly
   305 310
<210> 82
   <211> 273
   <212> PRT
   <213> Homo sapiens
<220>
   <221> misc_feature
   <223> amino acid sequence of fully human NY-ESO-1 TCR - alpha chain
<400> 82
<210> 83
   <211> 307
   <212> PRT
   <213> Homo sapiens
<220>
   <221> misc_feature
   <223> amino acid sequence of fully human NY-ESO-1 TCR - beta chain
<400> 83
   Met Leu Leu Leu Leu Leu Leu Leu Gly Pro Gly Ser Gly Leu Gly Ala
   1 5 10 15
   Val Val Ser Gln His Pro Ser Arg Val Ile Cys Lys Ser Gly Thr Ser
   20 25 30
   Val Lys Ile Glu Cys Arg Ser Leu Asp Phe Gln Ala Thr Thr Met Phe
   35 40 45
   Trp Tyr Arg Gln Phe Pro Lys Gln Ser Leu Met Leu Met Ala Thr Ser
   50 55 60
   Asn Glu Gly Ser Lys Ala Thr Tyr Glu Gln Gly Val Glu Lys Asp Lys
   65 70 75 80
   Phe Leu Ile Asn His Ala Ser Leu Thr Leu Ser Thr Leu Thr Val Thr
   85 90 95
   Ser Ala His Pro Glu Asp Ser Ser Phe Tyr Ile Cys Ser Ala Ser Val
   100 105 110
   Ala Thr Glu Thr Gln Tyr Phe Gly Pro Gly Thr Arg Leu Leu Val Leu
   115 120 125
   Glu Asp Leu Lys Asn Val Phe Pro Pro Glu Val Ala Val Phe Glu Pro
   130 135 140
   Ser Glu Ala Glu Ile Ser His Thr Gln Lys Ala Thr Leu Val Cys Leu
   145 150 155 160
   Ala Thr Gly Phe Tyr Pro Asp His Val Glu Leu Ser Trp Trp Val Asn
   165 170 175
   Gly Lys Glu Val His Ser Gly Val Ser Thr Asp Pro Gln Pro Leu Lys
   180 185 190
   Glu Gln Pro Ala Leu Asn Asp Ser Arg Tyr Cys Leu Ser Ser Arg Leu
   195 200 205
   Arg Val Ser Ala Thr Phe Trp Gln Asn Pro Arg Asn His Phe Arg Cys
   210 215 220
   Gln Val Gln Phe Tyr Gly Leu Ser Glu Asn Asp Glu Trp Thr Gln Asp
   225 230 235 240
   Arg Ala Lys Pro Val Thr Gln Ile Val Ser Ala Glu Ala Trp Gly Arg
   245 250 255
   Ala Asp Cys Gly Phe Thr Ser Glu Ser Tyr Gln Gln Gly Val Leu Ser
   260 265 270
   Ala Thr Ile Leu Tyr Glu Ile Leu Leu Gly Lys Ala Thr Leu Tyr Ala
   275 280 285
   Val Leu Val Ser Ala Leu Val Leu Met Ala Met Val Lys Arg Lys Asp
   290 295 300
   Ser Arg Gly
   305
<210> 84
   <211> 269
   <212> PRT
   <213> Mus musculus
<220>
   <221> misc_feature
   <223> amino acid sequence of fully murine p53 TCR - alpha chain
<400> 84
   Met Leu Leu Ala Leu Leu Pro Val Leu Gly Ile His Phe Val Leu Arg
   1               5                   10                  15
   Asp Ala Gln Ala Gln Ser Val Thr Gln Pro Asp Ala Arg Val Thr Val
               20                  25                  30
   Ser Glu Gly Ala Ser Leu Gln Leu Arg Cys Lys Tyr Ser Tyr Ser Gly
           35                  40                  45
   Thr Pro Tyr Leu Phe Trp Tyr Val Gln Tyr Pro Arg Gln Gly Leu Gln
       50                  55                  60
   Leu Leu Leu Lys Tyr Tyr Ser Gly Asp Pro Val Val Gln Gly Val Asn
   65                  70                  75                  80
   Gly Phe Glu Ala Glu Phe Ser Lys Ser Asn Ser Ser Phe His Leu Arg
                   85                  90                  95
   Lys Ala Ser Val His Trp Ser Asp Ser Ala Val Tyr Phe Cys Val Leu
               100                 105                 110
   Ser Glu Asp Ser Asn Tyr Gln Leu Ile Trp Gly Ser Gly Thr Lys Leu
           115                 120                 125
   Ile Ile Lys Pro Asp Ile Gln Asn Pro Glu Pro Ala Val Tyr Gln Leu
       130                 135                 140
   Lys Asp Pro Arg Ser Gln Asp Ser Thr Leu Cys Leu Phe Thr Asp Phe
   145                 150                 155                 160
   Asp Ser Gln Ile Asn Val Pro Lys Thr Met Glu Ser Gly Thr Phe Ile
                   165                 170                 175
   Thr Asp Lys Thr Val Leu Asp Met Lys Ala Met Asp Ser Lys Ser Asn
               180                 185                 190
   Gly Ala Ile Ala Trp Ser Asn Gln Thr Ser Phe Thr Cys Gln Asp Ile
           195                 200                 205
   Phe Lys Glu Thr Asn Ala Thr Tyr Pro Ser Ser Asp Val Pro Cys Asp
       210                 215                 220
   Ala Thr Leu Thr Glu Lys Ser Phe Glu Thr Asp Met Asn Leu Asn Phe
   225                 230                 235                 240
   Gln Asn Leu Ser Val Met Gly Leu Arg Ile Leu Leu Leu Lys Val Ala
                   245                 250                 255
   Gly Phe Asn Leu Leu Met Thr Leu Arg Leu Trp Ser Ser
               260                 265
<210> 85
   <211> 306
   <212> PRT
   <213> Mus musculus
<220>
   <221> misc_feature
   <223> amino acid sequence of fully murine p53 TCR - beta chain
<400> 85
   Met Ala Thr Arg Leu Leu Cys Tyr Thr Val Leu Cys Leu Leu Gly Ala
   1               5                   10                  15
   Arg Ile Leu Asn Ser Lys Val Ile Gln Thr Pro Arg Tyr Leu Val Lys
               20                  25                  30
   Gly Gln Gly Gln Lys Ala Lys Met Arg Cys Ile Pro Glu Lys Gly His
           35                  40                  45
   Pro Val Val Phe Trp Tyr Gln Gln Asn Lys Asn Asn Glu Phe Lys Phe
       50                  55                  60
   Leu Ile Asn Phe Gln Asn Gln Glu Val Leu Gln Gln Ile Asp Met Thr
   65                  70                  75                  80
   Glu Lys Arg Phe Ser Ala Glu Cys Pro Ser Asn Ser Pro Cys Ser Leu
                   85                  90                  95
   Glu Ile Gln Ser Ser Glu Ala Gly Asp Ser Ala Leu Tyr Leu Cys Ala
               100                 105                 110
   Ser Ser Leu Ser Gly Gly Gly Thr Glu Val Phe Phe Gly Lys Gly Thr
           115                 120                 125
   Arg Leu Thr Val Val Glu Asp Leu Arg Asn Val Thr Pro Pro Lys Val
       130                 135                 140
   Ser Leu Phe Glu Pro Ser Lys Ala Glu Ile Ala Asn Lys Gln Lys Ala
   145                 150                 155                 160
   Thr Leu Val Cys Leu Ala Arg Gly Phe Phe Pro Asp His Val Glu Leu
                   165                 170                 175
   Ser Trp Trp Val Asn Gly Lys Glu Val His Ser Gly Val Ser Thr Asp
               180                 185                 190
   Pro Gln Ala Tyr Lys Glu Ser Asn Tyr Ser Tyr Cys Leu Ser Ser Arg
           195                 200                 205
   Leu Arg Val Ser Ala Thr Phe Trp His Asn Pro Arg Asn His Phe Arg
       210                 215                 220
   Cys Gln Val Gln Phe His Gly Leu Ser Glu Glu Asp Lys Trp Pro Glu
   225                 230                 235                 240
   Gly Ser Pro Lys Pro Val Thr Gln Asn Ile Ser Ala Glu Ala Trp Gly
                   245                 250                 255
   Arg Ala Asp Cys Gly Ile Thr Ser Ala Ser Tyr Gln Gln Gly Val Leu
               260                 265                 270
   Ser Ala Thr Ile Leu Tyr Glu Ile Leu Leu Gly Lys Ala Thr Leu Tyr
           275                 280                 285
   Ala Val Leu Val Ser Thr Leu Val Val Met Ala Met Val Lys Arg Lys
       290                 295                 300
   Asn Ser
   305
<210> 86
   <211> 116
   <212> PRT
   <213> Artificial
<220>
   <223> Synthetic
<220>
   <221> misc_feature
   <223> amino acid sequence of extracellular domain of constant region of F4 Mut31 alpha
   116 aa
<400> 86
   Ile Gln Asn Pro Asp Pro Ala Val Tyr Gln Leu Arg Asp Ser Lys Ser
   1               5                   10                  15
   Ser Asp Lys Ser Val Cys Leu Phe Thr Asp Phe Asp Ser Gln Ile Asn
               20                  25                  30
   Val Pro Lys Thr Met Glu Ser Gly Thr Phe Ile Thr Asp Lys Thr Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Ser Asp Val Pro Cys Asp Ala Thr Leu Thr Glu
                   85                  90                  95
   Lys Ser Phe Glu Thr Asp Met Asn Leu Asn Phe Gln Asn Leu Ser Val
               100                 105                 110
   Met Gly Leu Arg
           115
<210> 87
   <211> 116
   <212> PRT
   <213> Artificial
<220>
   <223> Synthetic
<220>
   <221> misc_feature
   <223> amino acid sequence of extracellular domain of constant region of F4-Mut74 alpha
   116 aa
<400> 87
   Ile Gln Asn Pro Asp Pro Ala Val Tyr Gln Leu Arg Asp Ser Lys Ser
   1               5                   10                  15
   Ser Asp Lys Ser Val Cys Leu Phe Thr Asp Phe Asp Ser Gln Thr Asn
   20 25 30
   Val Ser Gln Ser Lys Asp Ser Asp Val Tyr Ile Thr Asp Lys Thr Val
   35 40 45
   Leu Asp Met Arg Ser Met Asp Phe Lys Ser Asn Ser Ala Val Ala Trp
   50 55 60
   Ser Asn Lys Ser Asp Phe Ala Cys Gln Asp Ile Phe Lys Glu Thr Asn
   65 70 75 80
   Ala Thr Tyr Pro Ser Ser Asp Val Pro Cys Asp Ala Thr Leu Thr Glu
   85 90 95
   Lys Ser Phe Glu Thr Asp Met Asn Leu Asn Phe Gln Asn Leu Ser Val
   100 105 110
   Met Gly Leu Arg
   115
<210> 88
   <211> 118
   <212> PRT
   <213> Artificial
<220>
   <223> Synthetic
<220>
   <221> misc_feature
   <223> amino acid sequence of extracellular domain of constant region of F4-AEK-Mut31a/Cpa:
   118 aa
<400> 88
   Ile Gln Asn Pro Asp Pro Ala Val Tyr Gln Leu Arg Asp Ser Lys Ser
   1 5 10 15
   Ser Asp Lys Ser Val Cys Leu Phe Thr Asp Phe Asp Ser Gln Ile Asn
   20 25 30
   Val Pro Lys Thr Met Glu Thr Glu Ser Gly Thr Phe Ile Thr Asp Lys
   35 40 45
   Thr Val Leu Asp Met Lys Ala Met Asp Ser Lys Ser Asn Gly Ala Ile
   50 55 60
   Ala Trp Ser Asn Gln Thr Ser Phe Thr Cys Gln Asp Ile Phe Lys Glu
   65 70 75 80
   Thr Asn Ala Thr Tyr Pro Ser Pro Glu Ser Ser Cys Asp Val Lys Leu
   85 90 95
   Val Glu Lys Ser Phe Glu Thr Asp Thr Asn Leu Asn Phe Gln Asn Leu
   100 105 110
   Ser Val Ile Gly Phe Arg
   115
<210> 89
   <211> 116
   <212> PRT
   <213> Artificial
<220>
   <223> Synthetic
<220>
   <221> misc_feature
   <223> amino acid sequence of extracellular domain of constant region of F4-BFK-Mut74a/Cpa:
   116 aa
<400> 89
   Ile Gln Asn Pro Asp Pro Ala Val Tyr Gln Leu Arg Asp Ser Lys Ser
   1 5 10 15
   Ser Asp Lys Ser Val Cys Leu Phe Thr Asp Phe Asp Ser Gln Thr Asn
   20 25 30
   Val Ser Gln Ser Lys Asp Ser Asp Val Tyr Ile Thr Asp Lys Thr Val
   35 40 45
   Leu Asp Met Arg Ser Met Asp Phe Lys Ser Asn Ser Ala Val Ala Trp
   50 55 60
   Ser Asn Lys Ser Asp Phe Ala Cys Gln Asp Ile Phe Lys Glu Thr Asn
   65 70 75 80
   Ala Thr Tyr Pro Ser Pro Glu Ser Ser Cys Asp Val Lys Leu Val Glu
   85 90 95
   Lys Ser Phe Glu Thr Asp Thr Asn Leu Asn Phe Gln Asn Leu Ser Val
   100 105 110
   Ile Gly Phe Arg
   115
<210> 90
   <211> 116
   <212> PRT
   <213> Artificial
<220>
   <223> Synthetic
<220>
   <221> misc_feature
   <223> amino acid sequence of extracellular domain of constant region of Cp-alpha:
   116 aa
<400> 90
   Ile Gln Asn Pro Glu Pro Ala Val Tyr Gln Leu Lys Asp Pro Arg Ser
   1               5                   10                  15
   Gln Asp Ser Thr Leu Cys Leu Phe Thr Asp Phe Asp Ser Gln Ile Asn
               20                  25                  30
   Val Pro Lys Thr Met Glu Ser Gly Thr Phe Ile Thr Asp Lys Thr Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Pro Glu Ser Ser Cys Asp Val Lys Leu Val Glu
                   85                  90                  95
   Lys Ser Phe Glu Thr Asp Thr Asn Leu Asn Phe Gln Asn Leu Ser Val
               100                 105                 110
   Ile Gly Phe Arg
           115
<210> 91
   <211> 146
   <212> PRT
   <213> Artificial
<220>
   <223> Synthetic
<220>
   <221> misc_feature
   <223> amino acid sequence of extracellular domain of constant region of F4-Mut62 beta
   146 aa
<400> 91
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Ile Thr Ser Ala Ser Tyr Gln Gln Gly Val Leu Ser Ala Thr Ile Leu
       130                 135                 140
   Tyr Glu
   145
<210> 92
   <211> 150
   <212> PRT
   <213> Artificial
<220>
   <223> Synthetic
<220>
   <221> misc_feature
   <223> amino acid sequence of extracellular domain of constant region of F4-Mut97 beta:
   150 aa
<400> 92
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe His Gly Leu Ser Glu Glu Asp Lys Trp Pro Glu Gly
               100                 105                 110
   Ser Pro Lys Pro Val Thr Gln Asn Ile Ser Ala Glu Ala Trp Gly Arg
           115                 120                 125
   Ala Asp Cys Gly Ile Thr Ser Ala Ser Tyr Gln Gln Gly Val Leu Ser
       130                 135                 140
   Ala Thr Ile Leu Tyr Glu
   145                 150
<210> 93
   <211> 146
   <212> PRT
   <213> Artificial
<220>
   <223> Synthetic
<220>
   <221> misc_feature
   <223> amino acid sequence of extracellular domain of constant region of F4-CGH-Mut62b/Cpb:
   146 aa
<400> 93
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Phe Thr Ser Val Ser Tyr Gln Gln Gly Val Leu Ser Ala Thr Ile Leu
       130                 135                 140
   Tyr Glu
   145
<210> 94
   <211> 146
   <212> PRT
   <213> Artificial
<220>
   <223> Synthetic
<220>
   <221> misc_feature
   <223> amino acid sequence of extracellular domain of constant region of Cp-beta:
   146 aa
<400> 94
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Phe Thr Ser Val Ser Tyr Gln Gln Gly Val Leu Ser Ala Thr Ile Leu
       130                 135                 140
   Tyr Glu
   145
<210> 95
   <211> 150
   <212> PRT
   <213> Artificial
<220>
   <223> Synthetic
<220>
   <221> misc_feature
   <223> amino acid sequence of extracellular domain of constant region of Mutant m40b:
   150 aa
<400> 95
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe Tyr Gly Leu Ser Glu Asn Asp Glu Trp Thr Gln Asp
               100                 105                 110
   Arg Ala Lys Pro Val Thr Gln Ile Val Ser Ala Glu Ala Trp Gly Arg
           115                 120                 125
   Ala Asp Cys Gly Phe Thr Ser Val Ser Tyr Gln Gln Gly Val Leu Ser
       130                 135                 140
   Ala Thr Ile Leu Tyr Glu
   145                 150
<210> 96
   <211> 116
   <212> PRT
   <213> Artificial
<220>
   <223> Synthetic
<220>
   <221> misc_feature
   <223> amino acid sequence of extracellular domain of constant region of MURINE-CYS alpha
   116 aa
<400> 96
   Ile Gln Asn Pro Glu Pro Ala Val Tyr Gln Leu Lys Asp Pro Arg Ser
   1               5                   10                  15
   Gln Asp Ser Thr Leu Cys Leu Phe Thr Asp Phe Asp Ser Gln Ile Asn
               20                  25                  30
   Val Pro Lys Thr Met Glu Ser Gly Thr Phe Ile Thr Asp Lys Cys Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Ser Asp Val Pro Cys Asp Ala Thr Leu Thr Glu
                   85                  90                  95
   Lys Ser Phe Glu Thr Asp Met Asn Leu Asn Phe Gln Asn Leu Ser Val
               100                 105                 110
   Met Gly Leu Arg
           115
<210> 97
   <211> 146
   <212> PRT
   <213> Artificial
<220>
   <223> Synthetic
<220>
   <221> misc_feature
   <223> amino acid sequence of extracellular domain of constant region of MURINE-CYS beta1
   146 aa
<400> 97
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Cys Thr Asp Pro Gln Ala Tyr Lys
       50                  55                  60
   Glu Ser Asn Tyr Ser Tyr Cys Leu Ser Ser Arg Leu Arg Val Ser Ala
   65 70 75 80
   Thr Phe Trp His Asn Pro Arg Asn His Phe Arg Cys Gln Val Gln Phe
   85 90 95
   His Gly Leu Ser Glu Glu Asp Lys Trp Pro Glu Gly Ser Pro Lys Pro
   100 105 110
   Val Thr Gln Asn Ile Ser Ala Glu Ala Trp Gly Arg Ala Asp Cys Gly
   115 120 125
   Ile Thr Ser Ala Ser Tyr Gln Gln Gly Val Leu Ser Ala Thr Ile Leu
   130 135 140
   Tyr Glu
   145
<210> 98
   <211> 146
   <212> PRT
   <213> Artificial
<220>
   <223> Synthetic
<220>
   <221> misc_feature
   <223> amino acid sequence of extracellular domain of constant region of MURINE-CYS beta2
   146 aa
<400> 98
   Glu Asp Leu Arg Asn Val Thr Pro Pro Lys Val Ser Leu Phe Glu Pro
   1 5 10 15
   Ser Lys Ala Glu Ile Ala Asn Lys Gln Lys Ala Thr Leu Val Cys Leu
   20 25 30
   Ala Arg Gly Phe Phe Pro Asp His Val Glu Leu Ser Trp Trp Val Asn
   35 40 45
   Gly Lys Glu Val His Ser Gly Val Cys Thr Asp Pro Gln Ala Tyr Lys
   50 55 60
   Glu Ser Asn Tyr Ser Tyr Cys Leu Ser Ser Arg Leu Arg Val Ser Ala
   65 70 75 80
   Thr Phe Trp His Asn Pro Arg Asn His Phe Arg Cys Gln Val Gln Phe
   85 90 95
   His Gly Leu Ser Glu Glu Asp Lys Trp Pro Glu Gly Ser Pro Lys Pro
   100 105 110
   Val Thr Gln Asn Ile Ser Ala Glu Ala Trp Gly Arg Ala Asp Cys Gly
   115 120 125
   Ile Thr Ser Ala Ser Tyr His Gln Gly Val Leu Ser Ala Thr Ile Leu
   130 135 140
   Tyr Glu
   145
<210> 99
   <211> 136
   <212> PRT
   <213> Artificial
<220>
   <223> Synthetic
<220>
   <221> misc_feature
   <223> amino acid sequence of constant region of F4 Mut31 alpha
   136 aa
<400> 99
   Ile Gln Asn Pro Asp Pro Ala Val Tyr Gln Leu Arg Asp Ser Lys Ser
   1 5 10 15
   Ser Asp Lys Ser Val Cys Leu Phe Thr Asp Phe Asp Ser Gln Ile Asn
   20 25 30
   Val Pro Lys Thr Met Glu Ser Gly Thr Phe Ile Thr Asp Lys Thr Val
   35 40 45
   Leu Asp Met Lys Ala Met Asp Ser Lys Ser Asn Gly Ala Ile Ala Trp
   50 55 60
   Ser Asn Gln Thr Ser Phe Thr Cys Gln Asp Ile Phe Lys Glu Thr Asn
   65 70 75 80
   Ala Thr Tyr Pro Ser Ser Asp Val Pro Cys Asp Ala Thr Leu Thr Glu
   85 90 95
   Lys Ser Phe Glu Thr Asp Met Asn Leu Asn Phe Gln Asn Leu Ser Val
   100 105 110
   Met Gly Leu Arg Ile Leu Leu Leu Lys Val Ala Gly Phe Asn Leu Leu
   115 120 125
   Met Thr Leu Arg Leu Trp Ser Ser
   130 135
<210> 100
   <211> 136
   <212> PRT
   <213> Artificial
<220>
   <223> Synthetic
<220>
   <221> misc_feature
   <223> amino acid sequence of constant region of F4-Mut74 alpha
   136 aa
<400> 100
   Ile Gln Asn Pro Asp Pro Ala Val Tyr Gln Leu Arg Asp Ser Lys Ser
   1 5 10 15
   Ser Asp Lys Ser Val Cys Leu Phe Thr Asp Phe Asp Ser Gln Thr Asn
   20 25 30
   Val Ser Gln Ser Lys Asp Ser Asp Val Tyr Ile Thr Asp Lys Thr Val
   35 40 45
   Leu Asp Met Arg Ser Met Asp Phe Lys Ser Asn Ser Ala Val Ala Trp
   50 55 60
   Ser Asn Lys Ser Asp Phe Ala Cys Gln Asp Ile Phe Lys Glu Thr Asn
   65 70 75 80
   Ala Thr Tyr Pro Ser Ser Asp Val Pro Cys Asp Ala Thr Leu Thr Glu
   85 90 95
   Lys Ser Phe Glu Thr Asp Met Asn Leu Asn Phe Gln Asn Leu Ser Val
   100 105 110
   Met Gly Leu Arg Ile Leu Leu Leu Lys Val Ala Gly Phe Asn Leu Leu
   115 120 125
   Met Thr Leu Arg Leu Trp Ser Ser
   130 135
<210> 101
   <211> 138
   <212> PRT
   <213> Artificial
<220>
   <223> Synthetic
<220>
   <221> misc_feature
   <223> amino acid sequence of constant region of F4-AEK-Mut31a/Cpa:
   138 aa
<400> 101
   Ile Gln Asn Pro Asp Pro Ala Val Tyr Gln Leu Arg Asp Ser Lys Ser
   1               5                   10                  15
   Ser Asp Lys Ser Val Cys Leu Phe Thr Asp Phe Asp Ser Gln Ile Asn
               20                  25                  30
   Val Pro Lys Thr Met Glu Thr Glu Ser Gly Thr Phe Ile Thr Asp Lys
           35                  40                  45
   Thr Val Leu Asp Met Lys Ala Met Asp Ser Lys Ser Asn Gly Ala Ile
       50                  55                  60
   Ala Trp Ser Asn Gln Thr Ser Phe Thr Cys Gln Asp Ile Phe Lys Glu
   65                  70                  75                  80
   Thr Asn Ala Thr Tyr Pro Ser Pro Glu Ser Ser Cys Asp Val Lys Leu
                   85                  90                  95
   Val Glu Lys Ser Phe Glu Thr Asp Thr Asn Leu Asn Phe Gln Asn Leu
               100                 105                 110
   Ser Val Ile Gly Phe Arg Ile Leu Leu Leu Lys Val Ala Gly Phe Asn
           115                 120                 125
   Leu Leu Met Thr Leu Arg Leu Trp Ser Ser
       130                 135
<210> 102
   <211> 136
   <212> PRT
   <213> Artificial
<220>
   <223> Synthetic
<220>
   <221> misc_feature <223> amino acid sequence of constant region of F4-BFK-Mut74a/Cpa:
   136 aa
<400> 102
   Ile Gln Asn Pro Asp Pro Ala Val Tyr Gln Leu Arg Asp Ser Lys Ser
   1               5                   10                  15
   Ser Asp Lys Ser Val Cys Leu Phe Thr Asp Phe Asp Ser Gln Thr Asn
               20                  25                  30
   Val Ser Gln Ser Lys Asp Ser Asp Val Tyr Ile Thr Asp Lys Thr Val
           35                  40                  45
   Leu Asp Met Arg Ser Met Asp Phe Lys Ser Asn Ser Ala Val Ala Trp
       50                  55                  60
   Ser Asn Lys Ser Asp Phe Ala Cys Gln Asp Ile Phe Lys Glu Thr Asn
   65                  70                  75                  80
   Ala Thr Tyr Pro Ser Pro Glu Ser Ser Cys Asp Val Lys Leu Val Glu
                   85                  90                  95
   Lys Ser Phe Glu Thr Asp Thr Asn Leu Asn Phe Gln Asn Leu Ser Val
               100                 105                 110
   Ile Gly Phe Arg Ile Leu Leu Leu Lys Val Ala Gly Phe Asn Leu Leu
           115                 120                 125
   Met Thr Leu Arg Leu Trp Ser Ser
       130                 135
<210> 103
   <211> 136
   <212> PRT
   <213> Artificial
<220>
   <223> Synthetic
<220>
   <221> misc_feature
   <223> amino acid sequence of constant region of Cp-alpha:
   136 aa
<400> 103
   Ile Gln Asn Pro Glu Pro Ala Val Tyr Gln Leu Lys Asp Pro Arg Ser
   1               5                   10                  15
   Gln Asp Ser Thr Leu Cys Leu Phe Thr Asp Phe Asp Ser Gln Ile Asn
               20                  25                  30
   Val Pro Lys Thr Met Glu Ser Gly Thr Phe Ile Thr Asp Lys Thr Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Pro Glu Ser Ser Cys Asp Val Lys Leu Val Glu
                   85                  90                  95
   Lys Ser Phe Glu Thr Asp Thr Asn Leu Asn Phe Gln Asn Leu Ser Val
               100                 105                 110
   Ile Gly Phe Arg Ile Leu Leu Leu Lys Val Ala Gly Phe Asn Leu Leu
           115                 120                 125
   Met Thr Leu Arg Leu Trp Ser Ser
       130                 135
<210> 104
   <211> 173
   <212> PRT
   <213> Artificial
<220>
   <223> Synthetic
<220>
   <221> misc_feature
   <223> amino acid sequence of constant region of F4-Mut62 beta
   173 aa
<400> 104
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Ile Thr Ser Ala Ser Tyr Gln Gln Gly Val Leu Ser Ala Thr Ile Leu
       130                 135                 140
   Tyr Glu Ile Leu Leu Gly Lys Ala Thr Leu Tyr Ala Val Leu Val Ser
   145                 150                 155                 160
   Thr Leu Val Val Met Ala Met Val Lys Arg Lys Asn Ser
                   165                 170
<210> 105
   <211> 177
   <212> PRT
   <213> Artificial
<220>
   <223> Synthetic
<220>
   <221> misc_feature
   <223> amino acid sequence constant region of F4-Mut97 beta:
   177 aa
<400> 105
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe His Gly Leu Ser Glu Glu Asp Lys Trp Pro Glu Gly
               100                 105                 110
   Ser Pro Lys Pro Val Thr Gln Asn Ile Ser Ala Glu Ala Trp Gly Arg
           115                 120                 125
   Ala Asp Cys Gly Ile Thr Ser Ala Ser Tyr Gln Gln Gly Val Leu Ser
       130                 135                 140
   Ala Thr Ile Leu Tyr Glu Ile Leu Leu Gly Lys Ala Thr Leu Tyr Ala
   145                 150                 155                 160
   Val Leu Val Ser Thr Leu Val Val Met Ala Met Val Lys Arg Lys Asn
                   165                 170                 175
   Ser
<210> 106
   <211> 173
   <212> PRT
   <213> Artificial
<220>
   <223> Synthetic
<220>
   <221> misc_feature
   <223> amino acid sequence of constant region of F4-CGH-Mut62b/Cpb:
   173 aa
<400> 106
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Phe Thr Ser Val Ser Tyr Gln Gln Gly Val Leu Ser Ala Thr Ile Leu
       130                 135                 140
   Tyr Glu Ile Leu Leu Gly Lys Ala Thr Leu Tyr Ala Val Leu Val Ser
   145                 150                 155                 160
   Ala Leu Val Leu Met Ala Met Val Lys Arg Lys Asp Phe
                   165                 170
<210> 107
   <211> 173
   <212> PRT
   <213> Artificial
<220>
   <223> Synthetic
<220>
   <221> misc_feature
   <223> amino acid sequence of constant region of Cp-beta:
   173 aa
<400> 107
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Ser Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Phe Thr Ser Val Ser Tyr Gln Gln Gly Val Leu Ser Ala Thr Ile Leu
       130                 135                 140
   Tyr Glu Ile Leu Leu Gly Lys Ala Thr Leu Tyr Ala Val Leu Val Ser
   145                 150                 155                 160
   Ala Leu Val Leu Met Ala Met Val Lys Arg Lys Asp Phe
                   165                 170
<210> 108
   <211> 177
   <212> PRT
   <213> Artificial
<220>
   <223> Synthetic
<220>
   <221> misc_feature
   <223> amino acid sequence of constant region of Mutant m40b:
   177 aa
<400> 108
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe Tyr Gly Leu Ser Glu Asn Asp Glu Trp Thr Gln Asp
               100                 105                 110
   Arg Ala Lys Pro Val Thr Gln Ile Val Ser Ala Glu Ala Trp Gly Arg
           115                 120                 125
   Ala Asp Cys Gly Phe Thr Ser Val Ser Tyr Gln Gln Gly Val Leu Ser
       130                 135                 140
   Ala Thr Ile Leu Tyr Glu Ile Leu Leu Gly Lys Ala Thr Leu Tyr Ala
   145                 150                 155                 160
   Val Leu Val Ser Ala Leu Val Leu Met Ala Met Val Lys Arg Lys Asp
                   165                 170                 175
   Phe
<210> 109
   <211> 136
   <212> PRT
   <213> Artificial
<220>
   <223> Synthetic
<220>
   <221> misc_feature
   <223> amino acid sequence of constant region of MURINE-CYS alpha
   136 aa
<400> 109
   Ile Gln Asn Pro Glu Pro Ala Val Tyr Gln Leu Lys Asp Pro Arg Ser
   1               5                   10                  15
   Gln Asp Ser Thr Leu Cys Leu Phe Thr Asp Phe Asp Ser Gln Ile Asn
               20                  25                  30
   Val Pro Lys Thr Met Glu Ser Gly Thr Phe Ile Thr Asp Lys Cys Val
           35                  40                  45
   Leu Asp Met Lys Ala Met Asp Ser Lys Ser Asn Gly Ala Ile Ala Trp
       50                  55                  60
   Ser Asn Gln Thr Ser Phe Thr Cys Gln Asp Ile Phe Lys Glu Thr Asn
   65                  70                  75                  80
   Ala Thr Tyr Pro Ser Ser Asp Val Pro Cys Asp Ala Thr Leu Thr Glu
                   85                  90                  95
   Lys Ser Phe Glu Thr Asp Met Asn Leu Asn Phe Gln Asn Leu Ser Val
               100                 105                 110
   Met Gly Leu Arg Ile Leu Leu Leu Lys Val Ala Gly Phe Asn Leu Leu
           115                 120                 125
   Met Thr Leu Arg Leu Trp Ser Ser
       130                 135
<210> 110
   <211> 173
   <212> PRT
   <213> Artificial
<220>
   <223> Synthetic
<220>
   <221> misc_feature
   <223> amino acid sequence of constant region of MURINE-CYS beta1
   173 aa
<400> 110
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Cys Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Ile Thr Ser Ala Ser Tyr Gln Gln Gly Val Leu Ser Ala Thr Ile Leu
       130                 135                 140
   Tyr Glu Ile Leu Leu Gly Lys Ala Thr Leu Tyr Ala Val Leu Val Ser
   145                 150                 155                 160
   Thr Leu Val Val Met Ala Met Val Lys Arg Lys Asn Ser
                   165                 170
<210> 111
   <211> 173
   <212> PRT
   <213> Artificial
<220>
   <223> Synthetic
<220>
   <221> misc_feature
   <223> amino acid sequence of constant region of MURINE-CYS beta2
   173 aa
<400> 111
   Glu Asp Leu Arg Asn Val Thr Pro Pro Lys Val Ser Leu Phe Glu Pro
   1               5                   10                  15
   Ser Lys Ala Glu Ile Ala Asn Lys Gln Lys Ala Thr Leu Val Cys Leu
               20                  25                  30
   Ala Arg Gly Phe Phe Pro Asp His Val Glu Leu Ser Trp Trp Val Asn
           35                  40                  45
   Gly Lys Glu Val His Ser Gly Val Cys Thr Asp Pro Gln Ala Tyr Lys
       50                  55                  60
   Glu Ser Asn Tyr Ser Tyr Cys Leu Ser Ser Arg Leu Arg Val Ser Ala
   65                  70                  75                  80
   Thr Phe Trp His Asn Pro Arg Asn His Phe Arg Cys Gln Val Gln Phe
                   85                  90                  95
   His Gly Leu Ser Glu Glu Asp Lys Trp Pro Glu Gly Ser Pro Lys Pro
               100                 105                 110
   Val Thr Gln Asn Ile Ser Ala Glu Ala Trp Gly Arg Ala Asp Cys Gly
           115                 120                 125
   Ile Thr Ser Ala Ser Tyr His Gln Gly Val Leu Ser Ala Thr Ile Leu
       130                 135                 140
   Tyr Glu Ile Leu Leu Gly Lys Ala Thr Leu Tyr Ala Val Leu Val Ser
   145                 150                 155                 160
   Gly Leu Val Leu Met Ala Met Val Lys Lys Lys Asn Ser
                   165                 170
<210> 112
   <211> 264
   <212> PRT
   <213> Artificial
<220>
   <223> Synthetic
<220>
   <221> misc_feature
   <223> amino acid sequence of full length chain of F4 Mut31 alpha
   264 aa
<400> 112
   Met Leu Leu Glu His Leu Leu Ile Ile Leu Trp Met Gln Leu Thr Trp
   1               5                   10                  15
   Val Ser Gly Gln Gln Leu Asn Gln Ser Pro Gln Ser Met Phe Ile Gln
               20                  25                  30
   Glu Gly Glu Asp Val Ser Met Asn Cys Thr Ser Ser Ser Ile Phe Asn
           35                  40                  45
   Thr Trp Leu Trp Tyr Lys Gln Asp Pro Gly Glu Gly Pro Val Leu Leu
       50                  55                  60
   Ile Ala Leu Tyr Lys Ala Gly Glu Leu Thr Ser Asn Gly Arg Leu Thr
   65                  70                  75                  80
   Ala Gln Phe Gly Ile Thr Arg Lys Asp Ser Phe Leu Asn Ile Ser Ala
                   85                  90                  95
   Ser Ile Pro Ser Asp Val Gly Ile Tyr Phe Cys Ala Gly Gly Thr Gly
               100                 105                 110
   Asn Gln Phe Tyr Phe Gly Thr Gly Thr Ser Leu Thr Val Ile Pro Asn
           115                 120                 125
   Ile Gln Asn Pro Asp Pro Ala Val Tyr Gln Leu Arg Asp Ser Lys Ser
       130                 135                 140
   Ser Asp Lys Ser Val Cys Leu Phe Thr Asp Phe Asp Ser Gln Ile Asn
   145                 150                 155                 160
   Val Pro Lys Thr Met Glu Ser Gly Thr Phe Ile Thr Asp Lys Thr Val
                   165                 170                 175
   Leu Asp Met Lys Ala Met Asp Ser Lys Ser Asn Gly Ala Ile Ala Trp
               180                 185                 190
   Ser Asn Gln Thr Ser Phe Thr Cys Gln Asp Ile Phe Lys Glu Thr Asn
           195                 200                 205
   Ala Thr Tyr Pro Ser Ser Asp Val Pro Cys Asp Ala Thr Leu Thr Glu
       210                 215                 220
   Lys Ser Phe Glu Thr Asp Met Asn Leu Asn Phe Gln Asn Leu Ser Val
   225                 230                 235                 240
   Met Gly Leu Arg Ile Leu Leu Leu Lys Val Ala Gly Phe Asn Leu Leu
   245 250 255
   Met Thr Leu Arg Leu Trp Ser Ser
   260
<210> 113
   <211> 264
   <212> PRT
   <213> Artificial
<220>
   <223> Synthetic
<220>
   <221> misc_feature
   <223> amino acid sequence of full length chain of F4-Mut74 alpha
   264 aa
<400> 113
   Met Leu Leu Glu His Leu Leu Ile Ile Leu Trp Met Gln Leu Thr Trp
   1 5 10 15
   Val Ser Gly Gln Gln Leu Asn Gln Ser Pro Gln Ser Met Phe Ile Gln
   20 25 30
   Glu Gly Glu Asp Val Ser Met Asn Cys Thr Ser Ser Ser Ile Phe Asn
   35 40 45
   Thr Trp Leu Trp Tyr Lys Gln Asp Pro Gly Glu Gly Pro Val Leu Leu
   50 55 60
   Ile Ala Leu Tyr Lys Ala Gly Glu Leu Thr Ser Asn Gly Arg Leu Thr
   65 70 75 80
   Ala Gln Phe Gly Ile Thr Arg Lys Asp Ser Phe Leu Asn Ile Ser Ala
   85 90 95
   Ser Ile Pro Ser Asp Val Gly Ile Tyr Phe Cys Ala Gly Gly Thr Gly
   100 105 110
   Asn Gln Phe Tyr Phe Gly Thr Gly Thr Ser Leu Thr Val Ile Pro Asn
   115 120 125
   Ile Gln Asn Pro Asp Pro Ala Val Tyr Gln Leu Arg Asp Ser Lys Ser
   130 135 140
   Ser Asp Lys Ser Val Cys Leu Phe Thr Asp Phe Asp Ser Gln Thr Asn
   145 150 155 160
   Val Ser Gln Ser Lys Asp Ser Asp Val Tyr Ile Thr Asp Lys Thr Val
   165 170 175
   Leu Asp Met Arg Ser Met Asp Phe Lys Ser Asn Ser Ala Val Ala Trp
   180 185 190
   Ser Asn Lys Ser Asp Phe Ala Cys Gln Asp Ile Phe Lys Glu Thr Asn
   195 200 205
   Ala Thr Tyr Pro Ser Ser Asp Val Pro Cys Asp Ala Thr Leu Thr Glu
   210 215 220
   Lys Ser Phe Glu Thr Asp Met Asn Leu Asn Phe Gln Asn Leu Ser Val
   225 230 235 240
   Met Gly Leu Arg Ile Leu Leu Leu Lys Val Ala Gly Phe Asn Leu Leu
   245 250 255
   Met Thr Leu Arg Leu Trp Ser Ser
   260
<210> 114
   <211> 266
   <212> PRT
   <213> Artificial
<220>
   <223> Synthetic
<220>
   <221> misc_feature
   <223> amino acid sequence of full length chain of F4-AEK-Mut31a/Cpa:
   266 aa
<400> 114
   Met Leu Leu Glu His Leu Leu Ile Ile Leu Trp Met Gln Leu Thr Trp
   1 5 10 15
   Val Ser Gly Gln Gln Leu Asn Gln Ser Pro Gln Ser Met Phe Ile Gln
   20 25 30
   Glu Gly Glu Asp Val Ser Met Asn Cys Thr Ser Ser Ser Ile Phe Asn
   35 40 45
   Thr Trp Leu Trp Tyr Lys Gln Asp Pro Gly Glu Gly Pro Val Leu Leu
           50                  55                  60
   Ile Ala Leu Tyr Lys Ala Gly Glu Leu Thr Ser Asn Gly Arg Leu Thr
   65                  70                  75                  80
   Ala Gln Phe Gly Ile Thr Arg Lys Asp Ser Phe Leu Asn Ile Ser Ala
                   85                  90                  95
   Ser Ile Pro Ser Asp Val Gly Ile Tyr Phe Cys Ala Gly Gly Thr Gly
               100                 105                 110
   Asn Gln Phe Tyr Phe Gly Thr Gly Thr Ser Leu Thr Val Ile Pro Asn
           115                 120                 125
   Ile Gln Asn Pro Asp Pro Ala Val Tyr Gln Leu Arg Asp Ser Lys Ser
       130                 135                 140
   Ser Asp Lys Ser Val Cys Leu Phe Thr Asp Phe Asp Ser Gln Ile Asn
   145                 150                 155                 160
   Val Pro Lys Thr Met Glu Thr Glu Ser Gly Thr Phe Ile Thr Asp Lys
                   165                 170                 175
   Thr Val Leu Asp Met Lys Ala Met Asp Ser Lys Ser Asn Gly Ala Ile
               180                 185                 190
   Ala Trp Ser Asn Gln Thr Ser Phe Thr Cys Gln Asp Ile Phe Lys Glu
           195                 200                 205
   Thr Asn Ala Thr Tyr Pro Ser Pro Glu Ser Ser Cys Asp Val Lys Leu
       210                 215                 220
   Val Glu Lys Ser Phe Glu Thr Asp Thr Asn Leu Asn Phe Gln Asn Leu
   225                 230                 235                 240
   Ser Val Ile Gly Phe Arg Ile Leu Leu Leu Lys Val Ala Gly Phe Asn
                   245                 250                 255
   Leu Leu Met Thr Leu Arg Leu Trp Ser Ser
               260                 265
<210> 115
   <211> 264
   <212> PRT
   <213> Artificial
<220>
   <223> Synthetic
<220>
   <221> misc_feature
   <223> amino acid sequence of full length chain of F4-BFK-Mut74a/Cpa:
   264 aa
<400> 115
   Met Leu Leu Glu His Leu Leu Ile Ile Leu Trp Met Gln Leu Thr Trp
   1               5                   10                  15
   Val Ser Gly Gln Gln Leu Asn Gln Ser Pro Gln Ser Met Phe Ile Gln
               20                  25                  30
   Glu Gly Glu Asp Val Ser Met Asn Cys Thr Ser Ser Ser Ile Phe Asn
           35                  40                  45
   Thr Trp Leu Trp Tyr Lys Gln Asp Pro Gly Glu Gly Pro Val Leu Leu
       50                  55                  60
   Ile Ala Leu Tyr Lys Ala Gly Glu Leu Thr Ser Asn Gly Arg Leu Thr
   65                  70                  75                  80
   Ala Gln Phe Gly Ile Thr Arg Lys Asp Ser Phe Leu Asn Ile Ser Ala
                   85                  90                  95
   Ser Ile Pro Ser Asp Val Gly Ile Tyr Phe Cys Ala Gly Gly Thr Gly
               100                 105                 110
   Asn Gln Phe Tyr Phe Gly Thr Gly Thr Ser Leu Thr Val Ile Pro Asn
           115                 120                 125
   Ile Gln Asn Pro Asp Pro Ala Val Tyr Gln Leu Arg Asp Ser Lys Ser
       130                 135                 140
   Ser Asp Lys Ser Val Cys Leu Phe Thr Asp Phe Asp Ser Gln Thr Asn
   145                 150                 155                 160
   Val Ser Gln Ser Lys Asp Ser Asp Val Tyr Ile Thr Asp Lys Thr Val
                   165                 170                 175
   Leu Asp Met Arg Ser Met Asp Phe Lys Ser Asn Ser Ala Val Ala Trp
               180                 185                 190
   Ser Asn Lys Ser Asp Phe Ala Cys Gln Asp Ile Phe Lys Glu Thr Asn
           195                 200                 205
   Ala Thr Tyr Pro Ser Pro Glu Ser Ser Cys Asp Val Lys Leu Val Glu
   210 215 220
   Lys Ser Phe Glu Thr Asp Thr Asn Leu Asn Phe Gln Asn Leu Ser Val
   225 230 235 240
   Ile Gly Phe Arg Ile Leu Leu Leu Lys Val Ala Gly Phe Asn Leu Leu
   245 250 255
   Met Thr Leu Arg Leu Trp Ser Ser
   260
<210> 116
   <211> 264
   <212> PRT
   <213> Artificial
<220>
   <223> Synthetic
<220>
   <221> misc_feature
   <223> amino acid sequence of full length chain of Cp-alpha:
   264 aa
<400> 116
   Met Leu Leu Glu His Leu Leu Ile Ile Leu Trp Met Gln Leu Thr Trp
   1 5 10 15
   Val Ser Gly Gln Gln Leu Asn Gln Ser Pro Gln Ser Met Phe Ile Gln
   20 25 30
   Glu Gly Glu Asp Val Ser Met Asn Cys Thr Ser Ser Ser Ile Phe Asn
   35 40 45
   Thr Trp Leu Trp Tyr Lys Gln Asp Pro Gly Glu Gly Pro Val Leu Leu
   50 55 60
   Ile Ala Leu Tyr Lys Ala Gly Glu Leu Thr Ser Asn Gly Arg Leu Thr
   65 70 75 80
   Ala Gln Phe Gly Ile Thr Arg Lys Asp Ser Phe Leu Asn Ile Ser Ala
   85 90 95
   Ser Ile Pro Ser Asp Val Gly Ile Tyr Phe Cys Ala Gly Gly Thr Gly
   100 105 110
   Asn Gln Phe Tyr Phe Gly Thr Gly Thr Ser Leu Thr Val Ile Pro Asn
           115                 120                 125
   Ile Gln Asn Pro Glu Pro Ala Val Tyr Gln Leu Lys Asp Pro Arg Ser
       130                 135                 140
   Gln Asp Ser Thr Leu Cys Leu Phe Thr Asp Phe Asp Ser Gln Ile Asn
   145                 150                 155                 160
   Val Pro Lys Thr Met Glu Ser Gly Thr Phe Ile Thr Asp Lys Thr Val
                   165                 170                 175
   Leu Asp Met Lys Ala Met Asp Ser Lys Ser Asn Gly Ala Ile Ala Trp
               180                 185                 190
   Ser Asn Gln Thr Ser Phe Thr Cys Gln Asp Ile Phe Lys Glu Thr Asn
           195                 200                 205
   Ala Thr Tyr Pro Ser Pro Glu Ser Ser Cys Asp Val Lys Leu Val Glu
       210                 215                 220
   Lys Ser Phe Glu Thr Asp Thr Asn Leu Asn Phe Gln Asn Leu Ser Val
   225                 230                 235                 240
   Ile Gly Phe Arg Ile Leu Leu Leu Lys Val Ala Gly Phe Asn Leu Leu
                   245                 250                 255
   Met Thr Leu Arg Leu Trp Ser Ser
               260
<210> 117
   <211> 305
   <212> PRT
   <213> Artificial
<220>
   <223> Synthetic
<220>
   <221> misc_feature
   <223> amino acid sequence of full length chain of F4-Mut62 beta
   305 aa
<400> 117
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
   20 25 30
   Glu Thr Gly Thr Pro Val Thr Leu Arg Cys His Gln Thr Glu Asn His
   35 40 45
   Arg Tyr Met Tyr Trp Tyr Arg Gln Asp Pro Gly His Gly Leu Arg Leu
   50 55 60
   Ile His Tyr Ser Tyr Gly Val Lys Asp Thr Asp Lys Gly Glu Val Ser
   65 70 75 80
   Asp Gly Tyr Ser Val Ser Arg Ser Lys Thr Glu Asp Phe Leu Leu Thr
   85 90 95
   Leu Glu Ser Ala Thr Ser Ser Gln Thr Ser Val Tyr Phe Cys Ala Ile
   100 105 110
   Ser Glu Val Gly Val Gly Gln Pro Gln His Phe Gly Asp Gly Thr Arg
   115 120 125
   Leu Ser Ile Leu Glu Asp Leu Asn Lys Val Phe Pro Pro Glu Val Ala
   130 135 140
   Val Phe Glu Pro Ser Glu Ala Glu Ile Ser His Thr Gln Lys Ala Thr
   145 150 155 160
   Leu Val Cys Leu Ala Thr Gly Phe Phe Pro Asp His Val Glu Leu Ser
   165 170 175
   Trp Trp Val Asn Gly Lys Glu Val His Ser Gly Val Ser Thr Asp Pro
   180 185 190
   Gln Ala Tyr Lys Glu Ser Asn Tyr Ser Tyr Cys Leu Ser Ser Arg Leu
   195 200 205
   Arg Val Ser Ala Thr Phe Trp His Asn Pro Arg Asn His Phe Arg Cys
   210 215 220
   Gln Val Gln Phe His Gly Leu Ser Glu Glu Asp Lys Trp Pro Glu Gly
   225 230 235 240
   Ser Pro Lys Pro Val Thr Gln Asn Ile Ser Ala Glu Ala Trp Gly Arg
   245 250 255
   Ala Asp Cys Gly Ile Thr Ser Ala Ser Tyr Gln Gln Gly Val Leu Ser
   260 265 270
   Ala Thr Ile Leu Tyr Glu Ile Leu Leu Gly Lys Ala Thr Leu Tyr Ala
   275 280 285
   Val Leu Val Ser Thr Leu Val Val Met Ala Met Val Lys Arg Lys Asn
   290 295 300
   Ser
   305
<210> 118
   <211> 309
   <212> PRT
   <213> Artificial
<220>
   <223> Synthetic
<220>
   <221> misc_feature
   <223> amino acid sequence full length chain of F4-Mut97 beta:
   309 aa
<400> 118
   Met Gly Thr Arg Leu Phe Phe Tyr Val Ala Leu Cys Leu Leu Trp Thr
   1 5 10 15
   Gly His Met Asp Ala Gly Ile Thr Gln Ser Pro Arg His Lys Val Thr
   20 25 30
   Glu Thr Gly Thr Pro Val Thr Leu Arg Cys His Gln Thr Glu Asn His
   35 40 45
   Arg Tyr Met Tyr Trp Tyr Arg Gln Asp Pro Gly His Gly Leu Arg Leu
   50 55 60
   Ile His Tyr Ser Tyr Gly Val Lys Asp Thr Asp Lys Gly Glu Val Ser
   65 70 75 80
   Asp Gly Tyr Ser Val Ser Arg Ser Lys Thr Glu Asp Phe Leu Leu Thr
   85 90 95
   Leu Glu Ser Ala Thr Ser Ser Gln Thr Ser Val Tyr Phe Cys Ala Ile
   100 105 110
   Ser Glu Val Gly Val Gly Gln Pro Gln His Phe Gly Asp Gly Thr Arg
   115 120 125
   Leu Ser Ile Leu Glu Asp Leu Asn Lys Val Phe Pro Pro Glu Val Ala
   130 135 140
   Val Phe Glu Pro Ser Glu Ala Glu Ile Ser His Thr Gln Lys Ala Thr
   145 150 155 160
   Leu Val Cys Leu Ala Thr Gly Phe Phe Pro Asp His Val Glu Leu Ser
   165 170 175
   Trp Trp Val Asn Gly Lys Glu Val His Ser Gly Val Ser Thr Asp Pro
   180 185 190
   Gln Pro Leu Lys Glu Gln Pro Ala Leu Asn Asp Ser Arg Tyr Cys Leu
   195 200 205
   Ser Ser Arg Leu Arg Val Ser Ala Thr Phe Trp Gln Asn Pro Arg Asn
   210 215 220
   His Phe Arg Cys Gln Val Gln Phe His Gly Leu Ser Glu Glu Asp Lys
   225 230 235 240
   Trp Pro Glu Gly Ser Pro Lys Pro Val Thr Gln Asn Ile Ser Ala Glu
   245 250 255
   Ala Trp Gly Arg Ala Asp Cys Gly Ile Thr Ser Ala Ser Tyr Gln Gln
   260 265 270
   Gly Val Leu Ser Ala Thr Ile Leu Tyr Glu Ile Leu Leu Gly Lys Ala
   275 280 285
   Thr Leu Tyr Ala Val Leu Val Ser Thr Leu Val Val Met Ala Met Val
   290 295 300
   Lys Arg Lys Asn Ser
   305
<210> 119
   <211> 305
   <212> PRT
   <213> Artificial
<220>
   <223> Synthetic
<220>
   <221> misc_feature
   <223> amino acid sequence of full length chain of F4-CGH-Mut62b/Cpb:
   305 aa
<400> 119
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Asn Lys Val Phe Pro Pro Glu Val Ala
       130                 135                 140
   Val Phe Glu Pro Ser Glu Ala Glu Ile Ser His Thr Gln Lys Ala Thr
   145                 150                 155                 160
   Leu Val Cys Leu Ala Thr Gly Phe Phe Pro Asp His Val Glu Leu Ser
                   165                 170                 175
   Trp Trp Val Asn Gly Lys Glu Val His Ser Gly Val Ser Thr Asp Pro
               180                 185                 190
   Gln Ala Tyr Lys Glu Ser Asn Tyr Ser Tyr Cys Leu Ser Ser Arg Leu
           195                 200                 205
   Arg Val Ser Ala Thr Phe Trp His Asn Pro Arg Asn His Phe Arg Cys
       210                 215                 220
   Gln Val Gln Phe His Gly Leu Ser Glu Glu Asp Lys Trp Pro Glu Gly
   225                 230                 235                 240
   Ser Pro Lys Pro Val Thr Gln Asn Ile Ser Ala Glu Ala Trp Gly Arg
                   245                 250                 255
   Ala Asp Cys Gly Phe Thr Ser Val Ser Tyr Gln Gln Gly Val Leu Ser
               260                 265                 270
   Ala Thr Ile Leu Tyr Glu Ile Leu Leu Gly Lys Ala Thr Leu Tyr Ala
           275                 280                 285
   Val Leu Val Ser Ala Leu Val Leu Met Ala Met Val Lys Arg Lys Asp
       290                 295                 300
   Phe
   305
<210> 120
   <211> 305
   <212> PRT
   <213> Artificial
<220>
   <223> Synthetic
<220>
   <221> misc_feature
   <223> amino acid sequence of full length chain of Cp-beta:
   305 aa
<400> 120
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65 70 75 80
   Asp Gly Tyr Ser Val Ser Arg Ser Lys Thr Glu Asp Phe Leu Leu Thr
   85 90 95
   Leu Glu Ser Ala Thr Ser Ser Gln Thr Ser Val Tyr Phe Cys Ala Ile
   100 105 110
   Ser Glu Val Gly Val Gly Gln Pro Gln His Phe Gly Asp Gly Thr Arg
   115 120 125
   Leu Ser Ile Leu Glu Asp Leu Arg Asn Val Thr Pro Pro Lys Val Ser
   130 135 140
   Leu Phe Glu Pro Ser Lys Ala Glu Ile Ala Asn Lys Gln Lys Ala Thr
   145 150 155 160
   Leu Val Cys Leu Ala Arg Gly Phe Phe Pro Asp His Val Glu Leu Ser
   165 170 175
   Trp Trp Val Asn Gly Lys Glu Val His Ser Gly Val Ser Thr Asp Pro
   180 185 190
   Gln Ala Tyr Lys Glu Ser Asn Tyr Ser Tyr Cys Leu Ser Ser Arg Leu
   195 200 205
   Arg Val Ser Ala Thr Phe Trp His Asn Pro Arg Asn His Phe Arg Cys
   210 215 220
   Gln Val Gln Phe His Gly Leu Ser Glu Glu Asp Lys Trp Pro Glu Gly
   225 230 235 240
   Ser Pro Lys Pro Val Thr Gln Asn Ile Ser Ala Glu Ala Trp Gly Arg
   245 250 255
   Ala Asp Cys Gly Phe Thr Ser Val Ser Tyr Gln Gln Gly Val Leu Ser
   260 265 270
   Ala Thr Ile Leu Tyr Glu Ile Leu Leu Gly Lys Ala Thr Leu Tyr Ala
   275 280 285
   Val Leu Val Ser Ala Leu Val Leu Met Ala Met Val Lys Arg Lys Asp
   290 295 300
   Phe
   305
<210> 121
   <211> 309
   <212> PRT
   <213> Artificial
<220>
   <223> Synthetic
<220>
   <221> misc_feature
   <223> amino acid sequence of full length chain of Mutant m40b:
   309 aa
<400> 121
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Arg Asn Val Thr Pro Pro Lys Val Ser
       130                 135                 140
   Leu Phe Glu Pro Ser Lys Ala Glu Ile Ala Asn Lys Gln Lys Ala Thr
   145                 150                 155                 160
   Leu Val Cys Leu Ala Arg Gly Phe Phe Pro Asp His Val Glu Leu Ser
   165 170 175
   Trp Trp Val Asn Gly Lys Glu Val His Ser Gly Val Ser Thr Asp Pro
   180 185 190
   Gln Pro Leu Lys Glu Gln Pro Ala Leu Asn Asp Ser Arg Tyr Cys Leu
   195 200 205
   Ser Ser Arg Leu Arg Val Ser Ala Thr Phe Trp Gln Asn Pro Arg Asn
   210 215 220
   His Phe Arg Cys Gln Val Gln Phe Tyr Gly Leu Ser Glu Asn Asp Glu
   225 230 235 240
   Trp Thr Gln Asp Arg Ala Lys Pro Val Thr Gln Ile Val Ser Ala Glu
   245 250 255
   Ala Trp Gly Arg Ala Asp Cys Gly Phe Thr Ser Val Ser Tyr Gln Gln
   260 265 270
   Gly Val Leu Ser Ala Thr Ile Leu Tyr Glu Ile Leu Leu Gly Lys Ala
   275 280 285
   Thr Leu Tyr Ala Val Leu Val Ser Ala Leu Val Leu Met Ala Met Val
   290 295 300
   Lys Arg Lys Asp Phe
   305
<210> 122
   <211> 264
   <212> PRT
   <213> Artificial
<220>
   <223> Synthetic
<220>
   <221> misc_feature
   <223> amino acid sequence of full length chain of Cys-F4 (H/M) alpha
   264 aa
<400> 122
   Met Leu Leu Glu His Leu Leu Ile Ile Leu Trp Met Gln Leu Thr Trp
   1 5 10 15
   Val Ser Gly Gln Gln Leu Asn Gln Ser Pro Gln Ser Met Phe Ile Gln
               20                  25                  30
   Glu Gly Glu Asp Val Ser Met Asn Cys Thr Ser Ser Ser Ile Phe Asn
           35                  40                  45
   Thr Trp Leu Trp Tyr Lys Gln Asp Pro Gly Glu Gly Pro Val Leu Leu
       50                  55                  60
   Ile Ala Leu Tyr Lys Ala Gly Glu Leu Thr Ser Asn Gly Arg Leu Thr
   65                  70                  75                  80
   Ala Gln Phe Gly Ile Thr Arg Lys Asp Ser Phe Leu Asn Ile Ser Ala
                   85                  90                  95
   Ser Ile Pro Ser Asp Val Gly Ile Tyr Phe Cys Ala Gly Gly Thr Gly
               100                 105                 110
   Asn Gln Phe Tyr Phe Gly Thr Gly Thr Ser Leu Thr Val Ile Pro Asn
           115                 120                 125
   Ile Gln Asn Pro Glu Pro Ala Val Tyr Gln Leu Lys Asp Pro Arg Ser
       130                 135                 140
   Gln Asp Ser Thr Leu Cys Leu Phe Thr Asp Phe Asp Ser Gln Ile Asn
   145                 150                 155                 160
   Val Pro Lys Thr Met Glu Ser Gly Thr Phe Ile Thr Asp Lys Cys Val
                   165                 170                 175
   Leu Asp Met Lys Ala Met Asp Ser Lys Ser Asn Gly Ala Ile Ala Trp
               180                 185                 190
   Ser Asn Gln Thr Ser Phe Thr Cys Gln Asp Ile Phe Lys Glu Thr Asn
           195                 200                 205
   Ala Thr Tyr Pro Ser Ser Asp Val Pro Cys Asp Ala Thr Leu Thr Glu
       210                 215                 220
   Lys Ser Phe Glu Thr Asp Met Asn Leu Asn Phe Gln Asn Leu Ser Val
   225                 230                 235                 240
   Met Gly Leu Arg Ile Leu Leu Leu Lys Val Ala Gly Phe Asn Leu Leu
                   245                 250                 255
   Met Thr Leu Arg Leu Trp Ser Ser
               260
<210> 123
   <211> 305
   <212> PRT
   <213> Artificial
<220>
   <223> Synthetic
<220>
   <221> misc_feature
   <223> amino acid sequence of full length chain of Cys-F4 (H/M) beta1
   305 aa
<400> 123
   Met Gly Thr Arg Leu Phe Phe Tyr Val Ala Leu Cys Leu Leu Trp Thr
   1               5                   10                  15
   Gly His Met Asp Ala Gly Ile Thr Gln Ser Pro Arg His Lys Val Thr
               20                  25                  30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Arg Asn Val Thr Pro Pro Lys Val Ser
       130                 135                 140
   Leu Phe Glu Pro Ser Lys Ala Glu Ile Ala Asn Lys Gln Lys Ala Thr
   145                 150                 155                 160
   Leu Val Cys Leu Ala Arg Gly Phe Phe Pro Asp His Val Glu Leu Ser
                   165                 170                 175
   Trp Trp Val Asn Gly Lys Glu Val His Ser Gly Val Cys Thr Asp Pro
   180 185 190
   Gln Ala Tyr Lys Glu Ser Asn Tyr Ser Tyr Cys Leu Ser Ser Arg Leu
   195 200 205
   Arg Val Ser Ala Thr Phe Trp His Asn Pro Arg Asn His Phe Arg Cys
   210 215 220
   Gln Val Gln Phe His Gly Leu Ser Glu Glu Asp Lys Trp Pro Glu Gly
   225 230 235 240
   Ser Pro Lys Pro Val Thr Gln Asn Ile Ser Ala Glu Ala Trp Gly Arg
   245 250 255
   Ala Asp Cys Gly Ile Thr Ser Ala Ser Tyr Gln Gln Gly Val Leu Ser
   260 265 270
   Ala Thr Ile Leu Tyr Glu Ile Leu Leu Gly Lys Ala Thr Leu Tyr Ala
   275 280 285
   Val Leu Val Ser Thr Leu Val Val Met Ala Met Val Lys Arg Lys Asn
   290 295 300
   Ser
   305
<210> 124
   <211> 305
   <212> PRT
   <213> Artificial
<220>
   <223> Synthetic
<220>
   <221> misc_feature
   <223> amino acid sequence of full length chain of Cys-F4 (H/M) beta2
   305 aa
<400> 124
   Met Gly Thr Arg Leu Phe Phe Tyr Val Ala Leu Cys Leu Leu Trp Thr
   1 5 10 15
   Gly His Met Asp Ala Gly Ile Thr Gln Ser Pro Arg His Lys Val Thr
   20 25 30
   Glu Thr Gly Thr Pro Val Thr Leu Arg Cys His Gln Thr Glu Asn His
           35                  40                  45
   Arg Tyr Met Tyr Trp Tyr Arg Gln Asp Pro Gly His Gly Leu Arg Leu
       50                  55                  60
   Ile His Tyr Ser Tyr Gly Val Lys Asp Thr Asp Lys Gly Glu Val Ser
   65                  70                  75                  80
   Asp Gly Tyr Ser Val Ser Arg Ser Lys Thr Glu Asp Phe Leu Leu Thr
                   85                  90                  95
   Leu Glu Ser Ala Thr Ser Ser Gln Thr Ser Val Tyr Phe Cys Ala Ile
               100                 105                 110
   Ser Glu Val Gly Val Gly Gln Pro Gln His Phe Gly Asp Gly Thr Arg
           115                 120                 125
   Leu Ser Ile Leu Glu Asp Leu Arg Asn Val Thr Pro Pro Lys Val Ser
       130                 135                 140
   Leu Phe Glu Pro Ser Lys Ala Glu Ile Ala Asn Lys Gln Lys Ala Thr
   145                 150                 155                 160
   Leu Val Cys Leu Ala Arg Gly Phe Phe Pro Asp His Val Glu Leu Ser
                   165                 170                 175
   Trp Trp Val Asn Gly Lys Glu Val His Ser Gly Val Cys Thr Asp Pro
               180                 185                 190
   Gln Ala Tyr Lys Glu Ser Asn Tyr Ser Tyr Cys Leu Ser Ser Arg Leu
           195                 200                 205
   Arg Val Ser Ala Thr Phe Trp His Asn Pro Arg Asn His Phe Arg Cys
       210                 215                 220
   Gln Val Gln Phe His Gly Leu Ser Glu Glu Asp Lys Trp Pro Glu Gly
   225                 230                 235                 240
   Ser Pro Lys Pro Val Thr Gln Asn Ile Ser Ala Glu Ala Trp Gly Arg
                   245                 250                 255
   Ala Asp Cys Gly Ile Thr Ser Ala Ser Tyr His Gln Gly Val Leu Ser
               260                 265                 270
   Ala Thr Ile Leu Tyr Glu Ile Leu Leu Gly Lys Ala Thr Leu Tyr Ala
   275 280 285
   Val Leu Val Ser Gly Leu Val Leu Met Ala Met Val Lys Lys Lys Asn
   290 295 300
   Ser
   305
<210> 125
   <211> 269
   <212> PRT
   <213> Artificial
<220>
   <223> Synthetic
<220>
   <221> misc_feature
   <223> amino acid sequence of full length chain of Cys-F5-(H/M) alpha
   269 aa
<400> 125
   Met Met Lys Ser Leu Arg Val Leu Leu Val Ile Leu Trp Leu Gln Leu
   1 5 10 15
   Ser Trp Val Trp Ser Gln Gln Lys Glu Val Glu Gln Asn Ser Gly Pro
   20 25 30
   Leu Ser Val Pro Glu Gly Ala Ile Ala Ser Leu Asn Cys Thr Tyr Ser
   35 40 45
   Asp Arg Gly Ser Gln Ser Phe Phe Trp Tyr Arg Gln Tyr Ser Gly Lys
   50 55 60
   Ser Pro Glu Leu Ile Met Phe Ile Tyr Ser Asn Gly Asp Lys Glu Asp
   65 70 75 80
   Gly Arg Phe Thr Ala Gln Leu Asn Lys Ala Ser Gln Tyr Val Ser Leu
   85 90 95
   Leu Ile Arg Asp Ser Gln Pro Ser Asp Ser Ala Thr Tyr Leu Cys Ala
   100 105 110
   Val Asn Phe Gly Gly Gly Lys Leu Ile Phe Gly Gln Gly Thr Glu Leu
   115 120 125
   Ser Val Lys Pro Asn Ile Gln Asn Pro Glu Pro Ala Val Tyr Gln Leu
   130 135 140
   Lys Asp Pro Arg Ser Gln Asp Ser Thr Leu Cys Leu Phe Thr Asp Phe
   145 150 155 160
   Asp Ser Gln Ile Asn Val Pro Lys Thr Met Glu Ser Gly Thr Phe Ile
   165 170 175
   Thr Asp Lys Cys Val Leu Asp Met Lys Ala Met Asp Ser Lys Ser Asn
   180 185 190
   Gly Ala Ile Ala Trp Ser Asn Gln Thr Ser Phe Thr Cys Gln Asp Ile
   195 200 205
   Phe Lys Glu Thr Asn Ala Thr Tyr Pro Ser Ser Asp Val Pro Cys Asp
   210 215 220
   Ala Thr Leu Thr Glu Lys Ser Phe Glu Thr Asp Met Asn Leu Asn Phe
   225 230 235 240
   Gln Asn Leu Ser Val Met Gly Leu Arg Ile Leu Leu Leu Lys Val Ala
   245 250 255
   Gly Phe Asn Leu Leu Met Thr Leu Arg Leu Trp Ser Ser
   260 265
<210> 126
   <211> 304
   <212> PRT
   <213> Artificial
<220>
   <223> Synthetic
<220>
   <221> misc_feature
   <223> amino acid sequence of full length chain of Cys-F5 (H/M) beta1
   304 aa
<400> 126
   Met Arg Ile Arg Leu Leu Cys Cys Val Ala Phe Ser Leu Leu Trp Ala
   1 5 10 15
   Gly Pro Val Ile Ala Gly Ile Thr Gln Ala Pro Thr Ser Gln Ile Leu
   20 25 30
   Ala Ala Gly Arg Arg Met Thr Leu Arg Cys Thr Gln Asp Met Arg His
   35 40 45
   Asn Ala Met Tyr Trp Tyr Arg Gln Asp Leu Gly Leu Gly Leu Arg Leu
   50 55 60
   Ile His Tyr Ser Asn Thr Ala Gly Thr Thr Gly Lys Gly Glu Val Pro
   65 70 75 80
   Asp Gly Tyr Ser Val Ser Arg Ala Asn Thr Asp Asp Phe Pro Leu Thr
   85 90 95
   Leu Ala Ser Ala Val Pro Ser Gln Thr Ser Val Tyr Phe Cys Ala Ser
   100 105 110
   Ser Leu Ser Phe Gly Thr Glu Ala Phe Phe Gly Gln Gly Thr Arg Leu
   115 120 125
   Thr Val Val Glu Asp Leu Arg Asn Val Thr Pro Pro Lys Val Ser Leu
   130 135 140
   Phe Glu Pro Ser Lys Ala Glu Ile Ala Asn Lys Gln Lys Ala Thr Leu
   145 150 155 160
   Val Cys Leu Ala Arg Gly Phe Phe Pro Asp His Val Glu Leu Ser Trp
   165 170 175
   Trp Val Asn Gly Lys Glu Val His Ser Gly Val Cys Thr Asp Pro Gln
   180 185 190
   Ala Tyr Lys Glu Ser Asn Tyr Ser Tyr Cys Leu Ser Ser Arg Leu Arg
   195 200 205
   Val Ser Ala Thr Phe Trp His Asn Pro Arg Asn His Phe Arg Cys Gln
   210 215 220
   Val Gln Phe His Gly Leu Ser Glu Glu Asp Lys Trp Pro Glu Gly Ser
   225 230 235 240
   Pro Lys Pro Val Thr Gln Asn Ile Ser Ala Glu Ala Trp Gly Arg Ala
   245 250 255
   Asp Cys Gly Ile Thr Ser Ala Ser Tyr Gln Gln Gly Val Leu Ser Ala
   260 265 270
   Thr Ile Leu Tyr Glu Ile Leu Leu Gly Lys Ala Thr Leu Tyr Ala Val
   275 280 285
   Leu Val Ser Thr Leu Val Val Met Ala Met Val Lys Arg Lys Asn Ser
   290 295 300
<210> 127
   <211> 304
   <212> PRT
   <213> Artificial
<220>
   <223> Synthetic
<220>
   <221> misc_feature
   <223> amino acid sequence of full length chain of Cys-F5 (H/M) beta2
   304 aa
<400> 127
   Met Arg Ile Arg Leu Leu Cys Cys Val Ala Phe Ser Leu Leu Trp Ala
   1 5 10 15
   Gly Pro Val Ile Ala Gly Ile Thr Gln Ala Pro Thr Ser Gln Ile Leu
   20 25 30
   Ala Ala Gly Arg Arg Met Thr Leu Arg Cys Thr Gln Asp Met Arg His
   35 40 45
   Asn Ala Met Tyr Trp Tyr Arg Gln Asp Leu Gly Leu Gly Leu Arg Leu
   50 55 60
   Ile His Tyr Ser Asn Thr Ala Gly Thr Thr Gly Lys Gly Glu Val Pro
   65 70 75 80
   Asp Gly Tyr Ser Val Ser Arg Ala Asn Thr Asp Asp Phe Pro Leu Thr
   85 90 95
   Leu Ala Ser Ala Val Pro Ser Gln Thr Ser Val Tyr Phe Cys Ala Ser
   100 105 110
   Ser Leu Ser Phe Gly Thr Glu Ala Phe Phe Gly Gln Gly Thr Arg Leu
   115 120 125
   Thr Val Val Glu Asp Leu Arg Asn Val Thr Pro Pro Lys Val Ser Leu
   130 135 140
   Phe Glu Pro Ser Lys Ala Glu Ile Ala Asn Lys Gln Lys Ala Thr Leu
   145 150 155 160
   Val Cys Leu Ala Arg Gly Phe Phe Pro Asp His Val Glu Leu Ser Trp
   165 170 175
   Trp Val Asn Gly Lys Glu Val His Ser Gly Val Cys Thr Asp Pro Gln
   180 185 190
   Ala Tyr Lys Glu Ser Asn Tyr Ser Tyr Cys Leu Ser Ser Arg Leu Arg
   195 200 205
   Val Ser Ala Thr Phe Trp His Asn Pro Arg Asn His Phe Arg Cys Gln
   210 215 220
   Val Gln Phe His Gly Leu Ser Glu Glu Asp Lys Trp Pro Glu Gly Ser
   225 230 235 240
   Pro Lys Pro Val Thr Gln Asn Ile Ser Ala Glu Ala Trp Gly Arg Ala
   245 250 255
   Asp Cys Gly Ile Thr Ser Ala Ser Tyr His Gln Gly Val Leu Ser Ala
   260 265 270
   Thr Ile Leu Tyr Glu Ile Leu Leu Gly Lys Ala Thr Leu Tyr Ala Val
   275 280 285
   Leu Val Ser Gly Leu Val Leu Met Ala Met Val Lys Lys Lys Asn Ser
   290 295 300
<210> 128
   <211> 269
   <212> PRT
   <213> Artificial
<220>
   <223> Synthetic
<220>
   <221> misc_feature
   <223> amino acid sequence of full length chain of Cys-NY-ESO-1-(H/M) alpha
   269 aa
<400> 128
   Met Asn Tyr Ser Pro Gly Leu Val Ser Leu Ile Leu Leu Leu Leu Gly
   1 5 10 15
   Arg Thr Arg Gly Asn Ser Val Thr Gln Met Glu Gly Pro Val Thr Leu
               20                  25                  30
   Ser Glu Glu Ala Phe Leu Thr Ile Asn Cys Thr Tyr Thr Ala Thr Gly
           35                  40                  45
   Tyr Pro Ser Leu Phe Trp Tyr Val Gln Tyr Pro Gly Glu Gly Leu Gln
       50                  55                  60
   Leu Leu Leu Lys Ala Thr Lys Ala Asp Asp Lys Gly Ser Asn Lys Gly
   65                  70                  75                  80
   Phe Glu Ala Thr Tyr Arg Lys Glu Thr Thr Ser Phe His Leu Glu Lys
                   85                  90                  95
   Gly Ser Val Gln Val Ser Asp Ser Ala Val Tyr Phe Cys Ala Leu Ser
               100                 105                 110
   Ala Asn Gln Ala Gly Thr Ala Leu Ile Phe Gly Lys Gly Thr Thr Leu
           115                 120                 125
   Ser Val Ser Ser Asn Ile Gln Asn Pro Glu Pro Ala Val Tyr Gln Leu
       130                 135                 140
   Lys Asp Pro Arg Ser Gln Asp Ser Thr Leu Cys Leu Phe Thr Asp Phe
   145                 150                 155                 160
   Asp Ser Gln Ile Asn Val Pro Lys Thr Met Glu Ser Gly Thr Phe Ile
                   165                 170                 175
   Thr Asp Lys Cys Val Leu Asp Met Lys Ala Met Asp Ser Lys Ser Asn
               180                 185                 190
   Gly Ala Ile Ala Trp Ser Asn Gln Thr Ser Phe Thr Cys Gln Asp Ile
           195                 200                 205
   Phe Lys Glu Thr Asn Ala Thr Tyr Pro Ser Ser Asp Val Pro Cys Asp
       210                 215                 220
   Ala Thr Leu Thr Glu Lys Ser Phe Glu Thr Asp Met Asn Leu Asn Phe
   225                 230                 235                 240
   Gln Asn Leu Ser Val Met Gly Leu Arg Ile Leu Leu Leu Lys Val Ala
                   245                 250                 255
   Gly Phe Asn Leu Leu Met Thr Leu Arg Leu Trp Ser Ser
                   260                 265
<210> 129
   <211> 301
   <212> PRT
   <213> Artificial
<220>
   <223> Synthetic
<220>
   <221> misc_feature
   <223> amino acid sequence of full length chain of Cys-NY-ESO-1 (H/M) beta1
   301 aa
<400> 129
   Met Leu Leu Leu Leu Leu Leu Leu Gly Pro Gly Ser Gly Leu Gly Ala
   1               5                   10                  15
   Val Val Ser Gln His Pro Ser Arg Val Ile Cys Lys Ser Gly Thr Ser
               20                  25                  30
   Val Lys Ile Glu Cys Arg Ser Leu Asp Phe Gln Ala Thr Thr Met Phe
           35                  40                  45
   Trp Tyr Arg Gln Phe Pro Lys Gln Ser Leu Met Leu Met Ala Thr Ser
       50                  55                  60
   Asn Glu Gly Ser Lys Ala Thr Tyr Glu Gln Gly Val Glu Lys Asp Lys
   65                  70                  75                  80
   Phe Leu Ile Asn His Ala Ser Leu Thr Leu Ser Thr Leu Thr Val Thr
                   85                  90                  95
   Ser Ala His Pro Glu Asp Ser Ser Phe Tyr Ile Cys Ser Ala Ser Val
               100                 105                 110
   Ala Thr Glu Thr Gln Tyr Phe Gly Pro Gly Thr Arg Leu Leu Val Leu
           115                 120                 125
   Glu Asp Leu Arg Asn Val Thr Pro Pro Lys Val Ser Leu Phe Glu Pro
       130                 135                 140
   Ser Lys Ala Glu Ile Ala Asn Lys Gln Lys Ala Thr Leu Val Cys Leu
   145                 150                 155                 160
   Ala Arg Gly Phe Phe Pro Asp His Val Glu Leu Ser Trp Trp Val Asn
   165 170 175
   Gly Lys Glu Val His Ser Gly Val Cys Thr Asp Pro Gln Ala Tyr Lys
   180 185 190
   Glu Ser Asn Tyr Ser Tyr Cys Leu Ser Ser Arg Leu Arg Val Ser Ala
   195 200 205
   Thr Phe Trp His Asn Pro Arg Asn His Phe Arg Cys Gln Val Gln Phe
   210 215 220
   His Gly Leu Ser Glu Glu Asp Lys Trp Pro Glu Gly Ser Pro Lys Pro
   225 230 235 240
   Val Thr Gln Asn Ile Ser Ala Glu Ala Trp Gly Arg Ala Asp Cys Gly
   245 250 255
   Ile Thr Ser Ala Ser Tyr Gln Gln Gly Val Leu Ser Ala Thr Ile Leu
   260 265 270
   Tyr Glu Ile Leu Leu Gly Lys Ala Thr Leu Tyr Ala Val Leu Val Ser
   275 280 285
   Thr Leu Val Val Met Ala Met Val Lys Arg Lys Asn Ser
   290 295 300
<210> 130
   <211> 301
   <212> PRT
   <213> Artificial
<220>
   <223> Synthetic
<220>
   <221> misc_feature
   <223> amino acid sequence of full length chain of Cys-NY-ESO-1 (H/M) beta2
   301 aa
<400> 130
   Met Leu Leu Leu Leu Leu Leu Leu Gly Pro Gly Ser Gly Leu Gly Ala
   1 5 10 15
   Val Val Ser Gln His Pro Ser Arg Val Ile Cys Lys Ser Gly Thr Ser
   20 25 30
   Val Lys Ile Glu Cys Arg Ser Leu Asp Phe Gln Ala Thr Thr Met Phe
           35                  40                  45
   Trp Tyr Arg Gln Phe Pro Lys Gln Ser Leu Met Leu Met Ala Thr Ser
       50                  55                  60
   Asn Glu Gly Ser Lys Ala Thr Tyr Glu Gln Gly Val Glu Lys Asp Lys
   65                  70                  75                  80
   Phe Leu Ile Asn His Ala Ser Leu Thr Leu Ser Thr Leu Thr Val Thr
                   85                  90                  95
   Ser Ala His Pro Glu Asp Ser Ser Phe Tyr Ile Cys Ser Ala Ser Val
               100                 105                 110
   Ala Thr Glu Thr Gln Tyr Phe Gly Pro Gly Thr Arg Leu Leu Val Leu
           115                 120                 125
   Glu Asp Leu Arg Asn Val Thr Pro Pro Lys Val Ser Leu Phe Glu Pro
       130                 135                 140
   Ser Lys Ala Glu Ile Ala Asn Lys Gln Lys Ala Thr Leu Val Cys Leu
   145                 150                 155                 160
   Ala Arg Gly Phe Phe Pro Asp His Val Glu Leu Ser Trp Trp Val Asn
                   165                 170                 175
   Gly Lys Glu Val His Ser Gly Val Cys Thr Asp Pro Gln Ala Tyr Lys
               180                 185                 190
   Glu Ser Asn Tyr Ser Tyr Cys Leu Ser Ser Arg Leu Arg Val Ser Ala
           195                 200                 205
   Thr Phe Trp His Asn Pro Arg Asn His Phe Arg Cys Gln Val Gln Phe
       210                 215                 220
   His Gly Leu Ser Glu Glu Asp Lys Trp Pro Glu Gly Ser Pro Lys Pro
   225                 230                 235                 240
   Val Thr Gln Asn Ile Ser Ala Glu Ala Trp Gly Arg Ala Asp Cys Gly
                   245                 250                 255
   Ile Thr Ser Ala Ser Tyr His Gln Gly Val Leu Ser Ala Thr Ile Leu
               260                 265                 270
   Tyr Glu Ile Leu Leu Gly Lys Ala Thr Leu Tyr Ala Val Leu Val Ser
           275                 280                 285
   Gly Leu Val Leu Met Ala Met Val Lys Lys Lys Asn Ser
       290                 295                 300
<210> 131
   <211> 269
   <212> PRT
   <213> Artificial
<220>
   <223> Synthetic
<220>
   <221> misc_feature
   <223> amino acid sequence of full length chain of Cys-gp100-(H/M) alpha
   269 aa
<400> 131
   Met Val Lys Ile Arg Gln Phe Leu Leu Ala Ile Leu Trp Leu Gln Leu
   1               5                   10                  15
   Ser Cys Val Ser Ala Ala Lys Asn Glu Val Glu Gln Ser Pro Gln Asn
               20                  25                  30
   Leu Thr Ala Gln Glu Gly Glu Phe Ile Thr Ile Asn Cys Ser Tyr Ser
           35                  40                  45
   Val Gly Ile Ser Ala Leu His Trp Leu Gln Gln His Pro Gly Gly Gly
       50                  55                  60
   Ile Val Ser Leu Phe Met Leu Ser Ser Gly Lys Lys Lys His Gly Arg
   65                  70                  75                  80
   Leu Ile Ala Thr Ile Asn Ile Gln Glu Lys His Ser Ser Leu His Ile
                   85                  90                  95
   Thr Ala Ser His Pro Arg Asp Ser Ala Val Tyr Ile Cys Ala Ala Ser
               100                 105                 110
   Leu Ile Gln Gly Ala Gln Lys Leu Val Phe Gly Gln Gly Thr Arg Leu
           115                 120                 125
   Thr Ile Asn Pro Asn Ile Gln Asn Pro Glu Pro Ala Val Tyr Gln Leu
       130                 135                 140
   Lys Asp Pro Arg Ser Gln Asp Ser Thr Leu Cys Leu Phe Thr Asp Phe
   145                 150                 155                 160
   Asp Ser Gln Ile Asn Val Pro Lys Thr Met Glu Ser Gly Thr Phe Ile
                   165                 170                 175
   Thr Asp Lys Cys Val Leu Asp Met Lys Ala Met Asp Ser Lys Ser Asn
               180                 185                 190
   Gly Ala Ile Ala Trp Ser Asn Gln Thr Ser Phe Thr Cys Gln Asp Ile
           195                 200                 205
   Phe Lys Glu Thr Asn Ala Thr Tyr Pro Ser Ser Asp Val Pro Cys Asp
       210                 215                 220
   Ala Thr Leu Thr Glu Lys Ser Phe Glu Thr Asp Met Asn Leu Asn Phe
   225                 230                 235                 240
   Gln Asn Leu Ser Val Met Gly Leu Arg Ile Leu Leu Leu Lys Val Ala
                   245                 250                 255
   Gly Phe Asn Leu Leu Met Thr Leu Arg Leu Trp Ser Ser
               260                 265
<210> 132
   <211> 304
   <212> PRT
   <213> Artificial
<220>
   <223> Synthetic
<220>
   <221> misc_feature
   <223> amino acid sequence of full length chain of Cys-gp100 (H/M) beta1
   304 aa
<400> 132
   Met Asp Ser Trp Thr Phe Cys Cys Val Ser Leu Cys Ile Leu Val Ala
   1               5                   10                  15
   Lys His Thr Asp Ala Gly Val Ile Gln Ser Pro Arg His Glu Val Thr
               20                  25                  30
   Glu Met Gly Gln Glu Val Thr Leu Arg Cys Lys Pro Ile Ser Gly His
   35 40 45
   Asn Ser Leu Phe Trp Tyr Arg Gln Thr Met Met Arg Gly Leu Glu Leu
   50 55 60
   Leu Ile Tyr Phe Asn Asn Asn Val Pro Ile Asp Asp Ser Gly Met Pro
   65 70 75 80
   Glu Asp Arg Phe Ser Ala Lys Met Pro Asn Ala Ser Phe Ser Thr Leu
   85 90 95
   Lys Ile Gln Pro Ser Glu Pro Arg Asp Ser Ala Val Tyr Phe Cys Ala
   100 105 110
   Ser Ser Pro Gly Gly Asn Glu Gln Phe Phe Gly Pro Gly Thr Arg Leu
   115 120 125
   Thr Val Leu Glu Asp Leu Arg Asn Val Thr Pro Pro Lys Val Ser Leu
   130 135 140
   Phe Glu Pro Ser Lys Ala Glu Ile Ala Asn Lys Gln Lys Ala Thr Leu
   145 150 155 160
   Val Cys Leu Ala Arg Gly Phe Phe Pro Asp His Val Glu Leu Ser Trp
   165 170 175
   Trp Val Asn Gly Lys Glu Val His Ser Gly Val Cys Thr Asp Pro Gln
   180 185 190
   Ala Tyr Lys Glu Ser Asn Tyr Ser Tyr Cys Leu Ser Ser Arg Leu Arg
   195 200 205
   Val Ser Ala Thr Phe Trp His Asn Pro Arg Asn His Phe Arg Cys Gln
   210 215 220
   Val Gln Phe His Gly Leu Ser Glu Glu Asp Lys Trp Pro Glu Gly Ser
   225 230 235 240
   Pro Lys Pro Val Thr Gln Asn Ile Ser Ala Glu Ala Trp Gly Arg Ala
   245 250 255
   Asp Cys Gly Ile Thr Ser Ala Ser Tyr Gln Gln Gly Val Leu Ser Ala
   260 265 270
   Thr Ile Leu Tyr Glu Ile Leu Leu Gly Lys Ala Thr Leu Tyr Ala Val
   275 280 285
   Leu Val Ser Thr Leu Val Val Met Ala Met Val Lys Arg Lys Asn Ser
       290                 295                 300
<210> 133
   <211> 304
   <212> PRT
   <213> Artificial
<220>
   <223> Synthetic
<220>
   <221> misc_feature
   <223> amino acid sequence of full length chain of Cys-gp100 (H/M) beta2
   304 aa
<400> 133
   Met Asp Ser Trp Thr Phe Cys Cys Val Ser Leu Cys Ile Leu Val Ala
   1               5                   10                  15
   Lys His Thr Asp Ala Gly Val Ile Gln Ser Pro Arg His Glu Val Thr
               20                  25                  30
   Glu Met Gly Gln Glu Val Thr Leu Arg Cys Lys Pro Ile Ser Gly His
           35                  40                  45
   Asn Ser Leu Phe Trp Tyr Arg Gln Thr Met Met Arg Gly Leu Glu Leu
       50                  55                  60
   Leu Ile Tyr Phe Asn Asn Asn Val Pro Ile Asp Asp Ser Gly Met Pro
   65                  70                  75                  80
   Glu Asp Arg Phe Ser Ala Lys Met Pro Asn Ala Ser Phe Ser Thr Leu
                   85                  90                  95
   Lys Ile Gln Pro Ser Glu Pro Arg Asp Ser Ala Val Tyr Phe Cys Ala
               100                 105                 110
   Ser Ser Pro Gly Gly Asn Glu Gln Phe Phe Gly Pro Gly Thr Arg Leu
           115                 120                 125
   Thr Val Leu Glu Asp Leu Arg Asn Val Thr Pro Pro Lys Val Ser Leu
       130                 135                 140
   Phe Glu Pro Ser Lys Ala Glu Ile Ala Asn Lys Gln Lys Ala Thr Leu
   145                 150                 155                 160
   Val Cys Leu Ala Arg Gly Phe Phe Pro Asp His Val Glu Leu Ser Trp
                   165                 170                 175
   Trp Val Asn Gly Lys Glu Val His Ser Gly Val Cys Thr Asp Pro Gln
               180                 185                 190
   Ala Tyr Lys Glu Ser Asn Tyr Ser Tyr Cys Leu Ser Ser Arg Leu Arg
           195                 200                 205
   Val Ser Ala Thr Phe Trp His Asn Pro Arg Asn His Phe Arg Cys Gln
       210                 215                 220
   Val Gln Phe His Gly Leu Ser Glu Glu Asp Lys Trp Pro Glu Gly Ser
   225                 230                 235                 240
   Pro Lys Pro Val Thr Gln Asn Ile Ser Ala Glu Ala Trp Gly Arg Ala
                   245                 250                 255
   Asp Cys Gly Ile Thr Ser Ala Ser Tyr His Gln Gly Val Leu Ser Ala
               260                 265                 270
   Thr Ile Leu Tyr Glu Ile Leu Leu Gly Lys Ala Thr Leu Tyr Ala Val
           275                 280                 285
   Leu Val Ser Gly Leu Val Leu Met Ala Met Val Lys Lys Lys Asn Ser
       290                 295                 300
<210> 134
   <211> 795
   <212> DNA
   <213> Artificial
<220>
   <223> Synthetic
<220>
   <221> misc_feature
   <223> nucleotide sequence of full length chain of F4-Mut31 alpha
<400> 134
   atgttgcttg aacatttatt aataatcttg tggatgcagc tgacatgggt cagtggtcaa      60
   cagctgaatc agagtcctca atctatgttt atccaggaag gagaagatgt ctccatgaac     120
   tgcacttctt caagcatatt taacacctgg ctatggtaca agcaggaccc tggggaaggt     180
   cctgtcctct tgatagcctt atataaggct ggtgaattga cctcaaatgg aagactgact     240
   gctcagtttg gtataaccag aaaggacagc ttcctgaata tctcagcatc catacctagt     300
   gatgtaggca tctacttctg tgctggtggg accggtaacc agttctattt tgggacaggg     360
   acaagtttga cggtcattcc aaatatccag aaccctgacc ctgccgtgta ccagctgaga     420
   gactctaaat ccagtgacaa gtctgtctgc ctattcaccg attttgactc ccaaatcaat     480
   gtgccgaaaa ccatggaatc tggaacgttc atcactgaca aaactgtgct ggacatgaaa     540
   gctatggatt ccaagagcaa tggggccatt gcctggagca accagacaag cttcacctgc     600
   caagatatct tcaaagagac caacgccacc taccccagtt cagacgttcc ctgtgatgcc     660
   acgttgactg agaaaagctt tgaaacagat atgaacctaa actttcaaaa cctgtcagtt     720
   atgggactcc gaatcctcct gctgaaagta gccggattta acctgctcat gacgctgagg     780
   ctgtggtcca gttga                                                      795
<210> 135
   <211> 795
   <212> DNA
   <213> Artificial
<220>
   <223> Synthetic
<220>
   <221> misc_feature
   <223> nucleotide sequence of full length chain of F4-Mut74 alpha
<400> 135
   atgttgcttg aacatttatt aataatcttg tggatgcagc tgacatgggt cagtggtcaa     60
   cagctgaatc agagtcctca atctatgttt atccaggaag gagaagatgt ctccatgaac    120
   tgcacttctt caagcatatt taacacctgg ctatggtaca agcaggaccc tggggaaggt    180
   cctgtcctct tgatagcctt atataaggct ggtgaattga cctcaaatgg aagactgact    240
   gctcagtttg gtataaccag aaaggacagc ttcctgaata tctcagcatc catacctagt    300
   gatgtaggca tctacttctg tgctggtggg accggtaacc agttctattt tgggacaggg    360
   acaagtttga cggtcattcc aaatatccag aaccctgacc ctgccgtgta ccagctgaga    420
   gactctaaat ccagtgacaa gtctgtctgc ctattcaccg attttgattc tcaaacaaat    480
   gtgtcacaaa gtaaggattc tgatgtgtat atcacagaca aaactgtgct agacatgagg    540
   tctatggact tcaagagcaa cagtgctgtg gcctggagca acaaatctga ctttgcatgc    600
   caagatatct tcaaagagac caacgccacc taccccagtt cagacgttcc ctgtgatgcc    660
   acgttgactg agaaaagctt tgaaacagat atgaacctaa actttcaaaa cctgtcagtt    720
   atgggactcc gaatcctcct gctgaaagta gccggattta acctgctcat gacgctgagg    780
   ctgtggtcca gttga                                                     795
<210> 136
   <211> 918
   <212> DNA
   <213> Artificial
<220>
   <223> Synthetic
<220>
   <221> misc_feature
   <223> nucleotide sequence of full length chain of F4-Mut62 beta
<400> 136
   atgggcacaa ggttgttctt ctatgtggcc ctttgtctcc tgtggacagg acacatggat     60
   gctggaatca cccagagccc aagacacaag gtcacagaga caggaacacc agtgactctg    120
   agatgtcacc agactgagaa ccaccgctat atgtactggt atcgacaaga cccggggcat    180
   gggctgaggc tgatccatta ctcatatggt gttaaagata ctgacaaagg agaagtctca    240
   gatggctata gtgtctctag atcaaagaca gaggatttcc tcctcactct ggagtccgct    300
   accagctccc agacatctgt gtacttctgt gccatcagtg aggtaggggt tgggcagccc    360
   cagcattttg gtgatgggac tcgactctcc atcctagagg acctgaacaa ggtgttccca    420
   cccgaggtcg ctgtgtttga gccatcagaa gcagagatct cccacaccca aaaggccaca    480
   ctggtgtgcc tggccacagg cttcttccct gaccacgtgg agctgagctg gtgggtgaat    540
   gggaaggagg tgcacagtgg ggtcagcacg gaccctcagg cctacaagga gagcaattat    600
   agctactgcc tgagcagccg cctgagggtc tctgctacct tctggcacaa tcctcgcaac    660
   cacttccgct gccaagtgca gttccatggg ctttcagagg aggacaagtg gccagagggc    720
   tcacccaaac ctgtcacaca gaacatcagt gcagaggcct ggggccgagc agactgtggg    780
   attacctcag catcctatca acaaggggtc ttgtctgcca ccatcctcta tgagatcctg    840
   ctagggaaag ccaccctgta tgctgtgctt gtcagtacac tggtggtgat ggctatggtc    900
   aaaagaaaga attcatga                                                  918
<210> 137
   <211> 930
   <212> DNA
   <213> Artificial
<220>
   <223> Synthetic
<220>
   <221> misc_feature
   <223> nucleotide sequence of full length chain of F4-Mut97 beta
<400> 137
   atgggcacaa ggttgttctt ctatgtggcc ctttgtctcc tgtggacagg acacatggat 60
   gctggaatca cccagagccc aagacacaag gtcacagaga caggaacacc agtgactctg 120
   agatgtcacc agactgagaa ccaccgctat atgtactggt atcgacaaga cccggggcat 180
   gggctgaggc tgatccatta ctcatatggt gttaaagata ctgacaaagg agaagtctca 240
   gatggctata gtgtctctag atcaaagaca gaggatttcc tcctcactct ggagtccgct 300
   accagctccc agacatctgt gtacttctgt gccatcagtg aggtaggggt tgggcagccc 360
   cagcattttg gtgatgggac tcgactctcc atcctagagg acctgaacaa ggtgttccca 420
   cccgaggtcg ctgtgtttga gccatcagaa gcagagatct cccacaccca aaaggccaca 480
   ctggtgtgcc tggccacagg cttcttccct gaccacgtgg agctgagctg gtgggtgaat 540
   gggaaggagg tgcacagtgg ggtcagcacg gacccgcagc ccctcaagga gcagcccgcc 600
   ctcaatgact ccagatactg cctgagcagc cgcctgaggg tctcggccac cttctggcag 660
   aacccccgca accacttccg ctgtcaagtc cagttccatg ggctttcaga ggaggacaag 720
   tggccagagg gctcacccaa acctgtcaca cagaacatca gtgcagaggc ctggggccga 780
   gcagactgtg ggattacctc agcatcctat caacaagggg tcttgtctgc caccatcctc 840
   tatgagatcc tgctagggaa agccaccctg tatgctgtgc ttgtcagtac actggtggtg 900
   atggctatgg tcaaaagaaa gaattcatga 930
<210> 138
   <211> 795
   <212> DNA
   <213> Artificial
<220>
   <223> Synthetic
<220>
   <221> misc_feature
   <223> nucleotide sequence of full length chain of F4-AEK-Mut31a/Cpa
<400> 138
   atgttgcttg aacatttatt aataatcttg tggatgcagc tgacatgggt cagtggtcaa 60
   cagctgaatc agagtcctca atctatgttt atccaggaag gagaagatgt ctccatgaac 120
   tgcacttctt caagcatatt taacacctgg ctatggtaca agcaggaccc tggggaaggt 180
   cctgtcctct tgatagcctt atataaggct ggtgaattga cctcaaatgg aagactgact 240
   gctcagtttg gtataaccag aaaggacagc ttcctgaata tctcagcatc catacctagt 300
   gatgtaggca tctacttctg tgctggtggg accggtaacc agttctattt tgggacaggg 360
   acaagtttga cggtcattcc aaatatccag aaccctgacc ctgccgtgta ccagctgaga    420
   gactctaaat ccagtgacaa gtctgtctgc ctattcaccg attttgactc ccaaatcaat    480
   gtgccgaaaa ccatggaatc tggaacgttc atcactgaca aaactgtgct ggacatgaaa    540
   gctatggatt ccaagagcaa tggggccatt gcctggagca accagacaag cttcacctgc    600
   caagatatct tcaaagagac caacgccacc taccccagcc cagaaagttc ctgtgatgtc    660
   aagctggtcg agaaaagctt tgaaacagat acgaacctaa actttcaaaa cctgtcagtg    720
   attgggttcc gaatcctcct cctgaaggtg gccgggttta atctgctcat gacgctgcgg    780
   ctgtggtcca gctga                                                     795
<210> 139
   <211> 795
   <212> DNA
   <213> Artificial
<220>
   <223> Synthetic
<220>
   <221> misc_feature
   <223> nucleotide sequence of full length chain of F4-BFK-Mut74a/Cpa
<400> 139
   atgttgcttg aacatttatt aataatcttg tggatgcagc tgacatgggt cagtggtcaa     60
   cagctgaatc agagtcctca atctatgttt atccaggaag gagaagatgt ctccatgaac    120
   tgcacttctt caagcatatt taacacctgg ctatggtaca agcaggaccc tggggaaggt    180
   cctgtcctct tgatagcctt atataaggct ggtgaattga cctcaaatgg aagactgact    240
   gctcagtttg gtataaccag aaaggacagc ttcctgaata tctcagcatc catacctagt    300
   gatgtaggca tctacttctg tgctggtggg accggtaacc agttctattt tgggacaggg    360
   acaagtttga cggtcattcc aaatatccag aaccctgacc ctgccgtgta ccagctgaga    420
   gactctaaat ccagtgacaa gtctgtctgc ctattcaccg attttgattc tcaaacaaat    480
   gtgtcacaaa gtaaggattc tgatgtgtat atcacagaca aaactgtgct agacatgagg    540
   tctatggact tcaagagcaa cagtgctgtg gcctggagca acaaatctga ctttgcatgc    600
   caagatatct tcaaagagac caacgccacc taccccagcc cagaaagttc ctgtgatgtc    660
   aagctggtcg agaaaagctt tgaaacagat acgaacctaa actttcaaaa cctgtcagtg    720
   attgggttcc gaatcctcct cctgaaggtg gccgggttta atctgctcat gacgctgcgg    780
   ctgtggtcca gctga                                                     795
<210> 140
   <211> 918
   <212> DNA
   <213> Artificial
<220>
   <223> Synthetic
<220>
   <221> misc_feature
   <223> nucleotide sequence of full length chain of F4-CGH-Mut62b/Cpb
<400> 140
   atgggcacaa ggttgttctt ctatgtggcc ctttgtctcc tgtggacagg acacatggat      60
   gctggaatca cccagagccc aagacacaag gtcacagaga caggaacacc agtgactctg     120
   agatgtcacc agactgagaa ccaccgctat atgtactggt atcgacaaga cccggggcat     180
   gggctgaggc tgatccatta ctcatatggt gttaaagata ctgacaaagg agaagtctca     240
   gatggctata gtgtctctag atcaaagaca gaggatttcc tcctcactct ggagtccgct     300
   accagctccc agacatctgt gtacttctgt gccatcagtg aggtaggggt tgggcagccc     360
   cagcattttg gtgatgggac tcgactctcc atcctagagg acctgaacaa ggtgttccca     420
   cccgaggtcg ctgtgtttga gccatcagaa gcagagatct cccacaccca aaaggccaca     480
   ctggtgtgcc tggccacagg cttcttccct gaccacgtgg agctgagctg gtgggtgaat     540
   gggaaggagg tgcacagtgg ggtcagcacg gaccctcagg cctacaagga gagcaattat     600
   agctactgcc tgagcagccg cctgagggtc tctgctacct tctggcacaa tcctcgcaac     660
   cacttccgct gccaagtgca gttccatggg ctttcagagg aggacaagtg gccagagggc     720
   tcacccaaac ctgtcacaca gaacatcagt gcagaggcct ggggccgagc agactgtggc     780
   tttacctcgg tgtcctacca gcaaggggtc ctgtctgcca ccatcctcta tgagatcctg     840
   ctagggaagg ccaccctgta tgctgtgctg gtcagcgccc ttgtgttgat ggccatggtc     900
   aagagaaagg atttctga                                                   918
<210> 141
   <211> 795
   <212> DNA
   <213> Artificial
<220>
   <223> Synthetic
<220>
   <221> misc_feature
   <223> nucleotide sequence of full length chain of Cp-alpha
<400> 141
   atgttgcttg aacatttatt aataatcttg tggatgcagc tgacatgggt cagtggtcaa     60
   cagctgaatc agagtcctca atctatgttt atccaggaag gagaagatgt ctccatgaac    120
   tgcacttctt caagcatatt taacacctgg ctatggtaca agcaggaccc tggggaaggt    180
   cctgtcctct tgatagcctt atataaggct ggtgaattga cctcaaatgg aagactgact    240
   gctcagtttg gtataaccag aaaggacagc ttcctgaata tctcagcatc catacctagt    300
   gatgtaggca tctacttctg tgctggtggg accggtaacc agttctattt tgggacaggg    360
   acaagtttga cggtcattcc aaatatccag aacccagaac ctgctgtgta ccagttaaaa    420
   gatcctcggt ctcaggacag caccctctgc ctgttcaccg actttgactc ccaaatcaat    480
   gtgccgaaaa ccatggaatc tggaacgttc atcactgaca aaactgtgct ggacatgaaa    540
   gctatggatt ccaagagcaa tggggccatt gcctggagca accagacaag cttcacctgc    600
   caagatatct tcaaagagac caacgccacc taccccagcc cagaaagttc ctgtgatgtc    660
   aagctggtcg agaaaagctt tgaaacagat acgaacctaa actttcaaaa cctgtcagtg    720
   attgggttcc gaatcctcct cctgaaggtg gccgggttta atctgctcat gacgctgcgg    780
   ctgtggtcca gctga                                                     795
<210> 142
   <211> 918
   <212> DNA
   <213> Artificial
<220>
   <223> Synthetic
<220>
   <221> misc_feature
   <223> nucleotide sequence of full length chain of Cp-beta
<400> 142
   atgggcacaa ggttgttctt ctatgtggcc ctttgtctcc tgtggacagg acacatggat     60
   gctggaatca cccagagccc aagacacaag gtcacagaga caggaacacc agtgactctg    120
   agatgtcacc agactgagaa ccaccgctat atgtactggt atcgacaaga cccggggcat    180
   gggctgaggc tgatccatta ctcatatggt gttaaagata ctgacaaagg agaagtctca    240
   gatggctata gtgtctctag atcaaagaca gaggatttcc tcctcactct ggagtccgct    300
   accagctccc agacatctgt gtacttctgt gccatcagtg aggtaggggt tgggcagccc    360
   cagcattttg gtgatgggac tcgactctcc atcctagagg atctgagaaa tgtgactcca    420
   cccaaggtct ccttgtttga gccatcaaaa gcagagattg caaacaaaca aaaggctacc    480
   ctcgtgtgct tggccagggg cttcttccct gaccacgtgg agctgagctg gtgggtgaat    540
   ggcaaggagg tccacagtgg ggtcagcacg gaccctcagg cctacaagga gagcaattat    600
   agctactgcc tgagcagccg cctgagggtc tctgctacct tctggcacaa tcctcgcaac    660
   cacttccgct gccaagtgca gttccatggg ctttcagagg aggacaagtg gccagagggc    720
   tcacccaaac ctgtcacaca gaacatcagt gcagaggcct ggggccgagc agactgtggc    780
   tttacctcgg tgtcctacca gcaaggggtc ctgtctgcca ccatcctcta tgagatcctg    840
   ctagggaagg ccaccctgta tgctgtgctg gtcagcgccc ttgtgttgat ggccatggtc    900
   aagagaaagg atttctga                                                  918
<210> 143
   <211> 930
   <212> DNA
   <213> Artificial
<220>
   <223> Synthetic
<220>
   <221> misc_feature
   <223> nucleotide sequence of full length chain of Mutant m40b
<400> 143
   atgggcacaa ggttgttctt ctatgtggcc ctttgtctcc tgtggacagg acacatggat     60
   gctggaatca cccagagccc aagacacaag gtcacagaga caggaacacc agtgactctg    120
   agatgtcacc agactgagaa ccaccgctat atgtactggt atcgacaaga cccggggcat    180
   gggctgaggc tgatccatta ctcatatggt gttaaagata ctgacaaagg agaagtctca    240
   gatggctata gtgtctctag atcaaagaca gaggatttcc tcctcactct ggagtccgct    300
   accagctccc agacatctgt gtacttctgt gccatcagtg aggtaggggt tgggcagccc    360
   cagcattttg gtgatgggac tcgactctcc atcctagagg atctgagaaa tgtgactcca    420
   cccaaggtct ccttgtttga gccatcaaaa gcagagattg caaacaaaca aaaggctacc    480
   ctcgtgtgct tggccagggg cttcttccct gaccacgtgg agctgagctg gtgggtgaat    540
   gggaaggagg tgcacagtgg ggtcagcacg gacccgcagc ccctcaagga gcagcccgcc    600
   ctcaatgact ccagatactg cctgagcagc cgcctgaggg tctcggccac cttctggcag    660
   aacccccgca accacttccg ctgtcaagtc cagttctacg ggctctcgga gaatgacgag    720
   tggacccagg atagggccaa acccgtcacc cagatcgtca gcgccgaggc ctggggtaga    780
   gcagactgtg gctttacctc ggtgtcctac cagcaagggg tcctgtctgc caccatcctc    840
   tatgagatcc tgctagggaa ggccaccctg tatgctgtgc tggtcagcgc ccttgtgttg    900
   atggccatgg tcaagagaaa ggatttctga                                     930
<210> 144
   <211> 795
   <212> DNA
   <213> Artificial
<220>
   <223> Synthetic
<220>
   <221> misc_feature
   <223> nucleotide sequence of full length chain of Cys-F4 (H/M) alpha
<400> 144
   atgttgcttg aacatttatt aataatcttg tggatgcagc tgacatgggt cagtggtcaa      60
   cagctgaatc agagtcctca atctatgttt atccaggaag gagaagatgt ctccatgaac     120
   tgcacttctt caagcatatt taacacctgg ctatggtaca agcaggaccc tggggaaggt     180
   cctgtcctct tgatagcctt atataaggct ggtgaattga cctcaaatgg aagactgact     240
   gctcagtttg gtataaccag aaaggacagc ttcctgaata tctcagcatc catacctagt     300
   gatgtaggca tctacttctg tgctggtggg accggtaacc agttctattt tgggacaggg     360
   acaagtttga cggtcattcc aaatatccag aacccagaac ctgctgtgta ccagttaaaa     420
   gatcctcggt ctcaggacag caccctctgc ctgttcaccg actttgactc ccaaatcaat     480
   gtgccgaaaa ccatggaatc tggaacgttc atcactgaca aatgcgtgct ggacatgaaa     540
   gctatggatt ccaagagcaa tggggccatt gcctggagca accagacaag cttcacctgc     600
   caagatatct tcaaagagac caacgccacc taccccagtt cagacgttcc ctgtgatgcc     660
   acgttgactg agaaaagctt tgaaacagat atgaacctaa actttcaaaa cctgtcagtt     720
   atgggactcc gaatcctcct gctgaaagta gccggattta acctgctcat gacgctgagg     780
   ctgtggtcca gttga                                                      795
<210> 145
   <211> 918
   <212> DNA
   <213> Artificial
<220>
   <223> Synthetic
<220>
   <221> misc_feature
   <223> nucleotide sequence of full length chain of Cys-F4 (H/M) beta
<400> 145
   atgggcacaa ggttgttctt ctatgtggcc ctttgtctcc tgtggacagg acacatggat      60
   gctggaatca cccagagccc aagacacaag gtcacagaga caggaacacc agtgactctg     120
   agatgtcacc agactgagaa ccaccgctat atgtactggt atcgacaaga cccggggcat    180
   gggctgaggc tgatccatta ctcatatggt gttaaagata ctgacaaagg agaagtctca    240
   gatggctata gtgtctctag atcaaagaca gaggatttcc tcctcactct ggagtccgct    300
   accagctccc agacatctgt gtacttctgt gccatcagtg aggtaggggt tgggcagccc    360
   cagcattttg gtgatgggac tcgactctcc atcctagagg atctgagaaa tgtgactcca    420
   cccaaggtct ccttgtttga gccatcaaaa gcagagattg caaacaaaca aaaggctacc    480
   ctcgtgtgct tggccagggg cttcttccct gaccacgtgg agctgagctg gtgggtgaat    540
   ggcaaggagg tccacagtgg ggtctgcacg gaccctcagg cctacaagga gagcaattat    600
   agctactgcc tgagcagccg cctgagggtc tctgctacct tctggcacaa tcctcgcaac    660
   cacttccgct gccaagtgca gttccatggg ctttcagagg aggacaagtg gccagagggc    720
   tcacccaaac ctgtcacaca gaacatcagt gcagaggcct ggggccgagc agactgtggg    780
   attacctcag catcctatca acaaggggtc ttgtctgcca ccatcctcta tgagatcctg    840
   ctagggaaag ccaccctgta tgctgtgctt gtcagtacac tggtggtgat ggctatggtc    900
   aaaagaaaga attcatga                                                  918
<210> 146
   <211> 810
   <212> DNA
   <213> Artificial
<220>
   <223> Synthetic
<220>
   <221> misc_feature
   <223> nucleotide sequence of full length chain of Cys-F5 (H/M) alpha
<400> 146
   atgatgaaat ccttgagagt tttactagtg atcctgtggc ttcagttgag ctgggtttgg     60
   agccaacaga aggaggtgga gcagaattct ggacccctca gtgttccaga gggagccatt    120
   gcctctctca actgcactta cagtgaccga ggttcccagt ccttcttctg gtacagacaa    180
   tattctggga aaagccctga gttgataatg ttcatatact ccaatggtga caaagaagat    240
   ggaaggttta cagcacagct caataaagcc agccagtatg tttctctgct catcagagac    300
   tcccagccca gtgattcagc cacctacctc tgtgccgtga acttcggagg aggaaagctt    360
   atcttcggac agggaacgga gttatctgtg aaacccaata tccagaaccc agaacctgct    420
   gtgtaccagt taaaagatcc tcggtctcag gacagcaccc tctgcctgtt caccgacttt    480
   gactcccaaa tcaatgtgcc gaaaaccatg gaatctggaa cgttcatcac tgacaaatgc    540
   gtgctggaca tgaaagctat ggattccaag agcaatgggg ccattgcctg gagcaaccag    600
   acaagcttca cctgccaaga tatcttcaaa gagaccaacg ccacctaccc cagttcagac    660
   gttccctgtg atgccacgtt gactgagaaa agctttgaaa cagatatgaa cctaaacttt    720
   caaaacctgt cagttatggg actccgaatc ctcctgctga aagtagccgg atttaacctg    780
   ctcatgacgc tgaggctgtg gtccagttga                                      810
<210> 147
   <211> 915
   <212> DNA
   <213> Artificial
<220>
   <223> Synthetic
<220>
   <221> misc_feature
   <223> nucleotide sequence of full length chain of Cys-F5 (H/M) beta
<400> 147
   atgagaatca ggctcctgtg ctgtgtggcc ttttctctcc tgtgggcagg tccagtgatt    60
   gctgggatca cccaggcacc aacatctcag atcctggcag caggacggcg catgacactg   120
   agatgtaccc aggatatgag acataatgcc atgtactggt atagacaaga tctaggactg   180
   gggctaaggc tcatccatta ttcaaatact gcaggtacca ctggcaaagg agaagtccct   240
   gatggttata gtgtctccag agcaaacaca gatgatttcc ccctcacgtt ggcgtctgct   300
   gtaccctctc agacatctgt gtacttctgt gccagcagcc taagtttcgg cactgaagct   360
   ttctttggac aaggcaccag actcacagtt gtagaggatc tgagaaatgt gactccaccc   420
   aaggtctcct tgtttgagcc atcaaaagca gagattgcaa acaaacaaaa ggctaccctc   480
   gtgtgcttgg ccaggggctt cttccctgac cacgtggagc tgagctggtg ggtgaatggc   540
   aaggaggtcc acagtggggt ctgcacggac cctcaggcct acaaggagag caattatagc   600
   tactgcctga gcagccgcct gagggtctct gctaccttct ggcacaatcc tcgcaaccac   660
   ttccgctgcc aagtgcagtt ccatgggctt tcagaggagg acaagtggcc agagggctca   720
   cccaaacctg tcacacagaa catcagtgca gaggcctggg gccgagcaga ctgtgggatt   780
   acctcagcat cctatcaaca aggggtcttg tctgccacca tcctctatga gatcctgcta   840
   gggaaagcca ccctgtatgc tgtgcttgtc agtacactgg tggtgatggc tatggtcaaa   900
   agaaagaatt catga                                                    915